(19) Europäisches Patentamt / European Patent Office / Office européen des brevets

(11) EP 1 923 472 B1

(12) EUROPEAN PATENT SPECIFICATION

(45) Date of publication and mention of the grant of the patent:
**11.04.2012 Bulletin 2012/15**

(51) Int Cl.:
***C12Q 1/68*** *(2006.01)*

(21) Application number: **07120734.4**

(22) Date of filing: **20.04.2000**

(54) **Detection of nucleic acid reactions on bead arrays**

Erkennung von Nukleinsäurereaktionen auf Bead-Arrays

Détection de réactions d'acide nucléique sur les réseaux à perle

(84) Designated Contracting States:
**AT BE CH CY DE DK ES FI FR GB GR IE IT LI LU MC NL PT SE**

(30) Priority:
**20.04.1999 US 130089 P**
**20.05.1999 US 135053 P**
**20.05.1999 US 135051 P**
**20.05.1999 US 135123 P**
**22.10.1999 US 161148 P**
**22.10.1999 US 160917 P**
**22.10.1999 US 160927 P**
**22.10.1999 US 425633 P**
**25.02.2000 US 513362**
**03.03.2000 US 517945**
**27.03.2000 US 535854**

(43) Date of publication of application:
**21.05.2008 Bulletin 2008/21**

(62) Document number(s) of the earlier application(s) in accordance with Art. 76 EPC:
**00926204.9 / 1 196 630**

(73) Proprietor: **Illumina, Inc.**
**San Diego, California 92121-1975 (US)**

(72) Inventors:
- **Gunderson, Kevin**
  **Encinitas, CA 92024 (US)**
- **Stuelpnagel, John, R.**
  **Encinitas, CA 92024 (US)**
- **Chee, Mark, S.**
  **Encinitas, CA 92024 (US)**

(74) Representative: **Hill, Justin John et al**
**Ipulse (IP) Ltd.**
**Carrington House**
**126-130 Regent Street**
**London W1B 5SE (GB)**

(56) References cited:
**EP-A- 0 478 319** **WO-A-98/50782**
**WO-A-99/18434**

- **RONAGHI ET AL: "A SEQUENCING METHOD BASED ON REAL-TIME PYROPHOSPHATE" SCIENCE, AMERICAN ASSOCIATION FOR THE ADVANCEMENT OF SCIENCE, US, vol. 281, 17 July 1998 (1998-07-17), pages 363-365, XP002135869 ISSN: 0036-8075**
- **CZARNIK A W: "ILLUMINATING THE SNP GENOMIC CODE" MODERN DRUG DISCOVERY, AMERICAN CHEMICAL SOCIETY,, US, vol. 1, no. 2, 1998, pages 49-55, XP000920564 ISSN: 1099-8209**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

The present disclosure is directed to methods and compositions for the use of microsphere arrays to detect and quantify a number of nucleic add reactions. The methods find use in genotyping, i.e. the determination of the sequence of nucleic acids, particularly alterations such as nucleotide substitutions (mismatches) and single nucleotide polymorphisms (SNPs). Similarly, the methods find use in the detection and quantification of a nucleic acid target using a variety of amplification techniques, including both signal amplification and target amplification. The methods and compositions can be used in nucleic acid sequencing reactions as well. All applications can include the use of adapter sequences to allow for universal arrays.

BACKGROUND OF THE INVENTION

The detection of specific nucleic acids is an important tool for diagnostic medicine and molecular biology research. Gene probe assays currently play roles in identifying infectious organisms such as bacteria and viruses, in probing the expression of normal and mutant genes and identifying mutant genes such as oncogenes, in typing tissue for compatibility preceding tissue transplantation, in matching tissue or blood samples for forensic medicine, and for exploring homology among genes from different species.

Ideally, a gene probe assay should be sensitive, specific and easily automatable (for a review, see Nickerson, Current Opinion in Biotechnology 4:48-51 (1993)). The requirement for sensitivity (i.e. low detection limits) has been greatly alleviated by the development of the polymerase chain reaction (PCR) and other amplification technologies which allow researchers to amplify exponentially a specific nucleic add sequence before analysis (for a review, see Abramson et al., Current Opinion In Biotechnology, 4:41-47 (1993)).

Sensitivity, i.e. detection limits, remain a significant obstacle in nucleic acid detection systems, and a variety of techniques have been developed to address this issue. Briefly, these techniques can be classified as either target amplification or signal amplification. Target amplification involves the amplification (i.e. replication) of the target sequence to be detected, resulting In a significant Increase in the number of target molecules. Target amplification strategies include the polymerase chain reaction (PCR), strand displacement amplification (SDA), and nucleic acid sequence based amplification (NASBA).

Alternatively, rather than amplify the target, alternate techniques use the target as a template to replicate a signalling probe, allowing a small number of target molecules to result In a large number of signalling probes, that then can be detected. Signal amplification strategies include the ligase chain reaction (LCR), cycling probe technology (CPT), invasive cleavage techniques such as Invader™ technology, Q-Beta replicase (QβR) technology, and the use of "amplification probes" such as "branched DNA" that result in multiple label probes binding to a single target sequence.

The polymerase chain reaction (PCR) Is widely used and described, and involves the use of primer extension combined with thermal cycling to amplify a target sequence; see U.S. Patent Nos. 4.683.195 and 4.683.202. and PCR Essential Data, J. W. Wdey & sons, Ed. C.R. Newton, 1995 in addition, there are a number of variations of PCR which also find use in the invention, including "quantitative competitive PCR" or "QC-PCR", "arbitrarily primed PCR" or "AP-PCR", "immuno-PCR", "Alu-PCR", "PCR single strand conformational polymorphism" or "PCR-SSCP", allelic PCR (see Newton et al. Nucl. Acid Res. 17:2503 91989); "reverse transcriptase PCR" or "RT-PCR", "biotin capture PCR", "vectorette PCR", "panhandle PCR", and "PCR select cDNA subtraction", among others.

Strand displacement amplification (SDA) is generally described in Walker et al., in Molecular Methods for Virus Detection, Academic Press, Inc., 1995, and U.S. Patent Nos. 5,455,166 and 5,130,238,

Nucleic acid sequence based amplification (NASBA) Is generally described in U.S. Patent No. 5,409,818 and "Profiting from Gene-based Diagnostics", CTB International Publishing Inc., N.J., 1996,

Cycling probe technology (CPT) is a nucleic acid detection system based on signal or probe amplification rather than target amplification, such as is done In polymerase chain reactions (PCR). Cycling probe technology relies on a molar excess of labeled probe which contains a scissile linkage of RNA. Upon hybridization of the probe to the target, the resulting hybrid contains a portion of RNA:DNA. This area of RNA:DNA duplex is recognized by RNAseH and the RNA is excised, resulting in cleavage of the probe. The probe now consists of two smaller sequences which may be released, thus leaving the target intact for repeated rounds of the reaction. The unreacted probe is removed and the label is then detected. CPT is generally described in U.S. Patent Nos. 5,011,769, 5,403,719, 5,660,988, and 4,878,187, and PCT published applications WO 95/05480, WO 95/1416, and WO 95/00667,

The oligonucleotide ligation assay (OLA; sometimes referred to as the ligation chain reaction (LCR)) involve the ligation of at least two smaller probes into a single long probe, using the target sequence as the template for the ligase. See generally U.S. Patent Nos. 5,185,243, 5,679,524 and 5,573,907; EP 0 320 308 B1: EP 0 336 731 B1: EP 0 439182 B1; WO 90/01069; WO 89/12696; and WO 89/09835

Invader™ technology is based on structure-specific polymerases that cleave nucleic acids in a site-specific manner. Two probes are used: an "invader" probe and a "signalling" probe, that adjacently hybridize to a target sequence with a non-complementary overlap. The enzyme cleaves at the overlap due to its recognition of the "tail", and releases the "tali" with a label. This can then be detected. The Invader™ technology is described in U.S. Patent Nos. 5,848,717; 5,614,402; 5,719,028; 5,541,311; and 5,843,669,

"Rolling circle amplification" is based on extension of a circular probe that has hybridized to a target sequence. A polymerase is added that extends the probe sequence. As the circular probe has no terminus, the polymerase repeatedly extends the circular probe resulting in concatamers of the circular probe. As such, the probe is amplified. Rolling-circle amplification is generally described in Baner et a/. (1998) Nuc. Acids Res. 26: 5073-5078; Barany, F. (1991) Proc. Natl. Acad. Sci. USA 88:189-193; and Lizardi et al. (1998) Nat. Genet. 19: 225-232,

"Branched DNA" signal amplification relies on the synthesis of branched nucleic acids, containing a multiplicity of nucleic acid "arms" that function to increase the amount of label that can be put onto one probe. This technology is generally described In U.S. Patent Nos. 5,681,702, 5,597,909, 5,545,730, 5,594,117, 5,591,584, 5,571,670, 5,580,731, 5,571,670, 5,591,584, 5,624,802, 5,635,352, 5,594,118, 5,359,100, 5,124,246 and 5,681,697.

Similarily, dendrimers of nucleic acids serve to vastly increase the amount of label that can be added to a single molecule, using a similar idea but different compositions. This technology is as described In U.S. Patent No. 5,175,270 and Nitsen et al., J. Theor. Biol. 187273 (1997),

Specificity, In contrast, remains a problem in many currently available gene probe assays. The extent of molecular complementarity between probe and target defines the specificity of the interaction. In a practical sense, the degree of similarity between the target and other sequences In the sample also has an impact on specificity. Variations in the concentrations of probes, of targets and of salts in the hybridization medium, in the reaction temperature, and in the length of the probe may alter or influence the specificity of the probe/target interaction.

It may be possible under some circumstances to distinguish targets with perfect complementarity from targets with mismatches; this is generally very difficult using traditional technology such as filter hybridization, in situ hybridization etc., since small variations in the reaction conditions will alter the hybridization, although this may not be a problem if appropriate mismatch controls are provided. New experimental techniques for mismatch detection with standard probes include DNA ligation assays where single point mismatches prevent ligation and probe digestion assays in which mismatches create sites for probe cleavage.

Recent focus has been on the analysis of the relationship between genetic variation and phenotype by making use of polymorphic DNA markers. Previous work utilized short tandem repeats (STRs) as polymorphic positional markers; however, recent focus is on the use of single nucleotide polymorphism (SNPs), which occur at an average frequency of more than 1 per kilobase in human genomic DNA. Some SNPs, particularly those in and around coding sequences, are likely to be the direct cause of therapeutically relevant phenotypic variants and/or disease predisposition. There are a number of well known polymorphisms that cause clinically important phenotypes; for example, the apoE2/3/4 variants are associated with different relative risk of Alzheimer's and other diseases (see Cordor et al., Science 261 (1993). Multiplex PCR amplification of SNP loci with subsequent hybridization to oligonucleotide arrays has been shown to be an accurate and reliable method of simultaneously genotyping at least hundreds of SNPs; see Wang et al., Science, 280:1077 (1998); see also Schafer et al., Nature Biotechnology 16:33-39 (1998). The compositions of the present invention may easily be substituted for the arrays of the prior art.

There are a variety of particular techniques that are used to detect sequence, including mutations and SNPs. These include, but are not limited to, ligation based assays, cleavage based assays (mismatch and invasive cleavage such as Invader™), single base extension methods (see WO 92/15712, EP 0 371 437 B1, EP 0317 074 B1; Pastinen et al., Genome Res. 7:606-614 (1997); Syvänen, Clinica Chimica Acta 226:225-236 (1994); and WO 91/13075), and competitive probe analysis (e.g. competitive sequencing by hybridization; see below).

In addition, DNA sequencing is a crucial technology In biology today, as the rapid sequencing of genomes, including the human genome, is both a significant goal and a significant hurdle. Thus there is a significant need for robust, high-throughput methods. Tradidonally, the most common method of DNA sequencing has been based on polyacrylamide gel fractionation to resolve a population of chain-terminated fragments (Sanger et al., Proc. Natl. Acad. Sci. USA 74:5463 (1977); Maxam & Gilbert). The population of fragments, terminated at each position In the DNA sequence, can be generated in a number of ways. Typically, DNA polymerase is used to Incorporate dideoxynucleotides that serve as chain terminators.

Several alternative methods have been developed to Increase the speed and ease of DNA sequencing. For example, sequencing by hybridizatton has been described (Drmanac et al., Genomics 4:114 (1989); Koster et al., Nature Biotechnology 14:1123 (1996); U.S. Patent Nos. 5,525,464; 5,202,231 and 5,695,940, among others). Similarly, sequencing by synthesis Is an alternative to gel-based sequencing. These methods add and read only one base (or at most a few bases, typically of the same type) prior to polymerization of the next base. This can be referred to as time resolved" sequencing, to contrast from "gel-resolved" sequencing. Sequencing by synthesis has been described in U. S. Patent No 4,971,803 and Hyman, Anal. Biochem. 174:423 (1888);

Rosenthal, International Patent Application Publication 761107 (1989); Metzker et al., Nucl. Acids Res. 22:4259 (1994); Jones, Biotechniques 22:938 (1997); Ronaghi et al., Anal. Blochem. 242:84 (1998), Nyren et al., Anal. Biochem. 151:504 (1985). Detection of ATP sulfurylase activity is described in Karamohamed and Nyren, Anal. Blochem. 271:81 (1999). Sequencing using reversible chain terminating nucleotides is described In U.S. Patent Nos. 5,902,723 and 5,547,839, and Canard and Arzumanov, Gene 11:1 (1994), and Dyatkina and Arzumanov, Nucleic Acids Symp Ser 18:117 (1987). Reversible chain termination with DNA ligase Is described in U.S. Patent 5,403,708. Time resolved sequencing Is described in Johnson et al., Anal. Biochem. 136:192 (1984): Single molecule analysis is described in U.S. Patent No. 5,795,782 and Elgen and Rigler, Proc. Natl Acad Sci USA 91(13):5740 (1994),

One promising sequencing by synthesis methd Is based on the detection of the pyrophosphate (PPi) released during the DNA polymerase reaction. As nucleotriphosphates are added to a growing nucleic acid chain, they release PPi. This release can be quantitatively measured by the conversion of PPi to ATP by the enzyme sulfurylase, and the subsequent production of visible light by firefly luciferase.

Several assay systems haves been described that capitalize on this mechanism. See for example WO93/23564, WO 98/28440 and WO98/13523,

A preferred method is described In Ronaghi et al., Science 281:363 (1998). In this method, the four deoxynucleotides (dATP, dGTP, dCTP and dTTP; collectively dNTPs) are added stepwise to a partial duplex comprising a sequencing primer hybridized to a single stranded DNA template and Incubated with DNA polymerase, ATP sulfurylase, luciferase, and optionally a nucleotide-degrading enzyme such as apyrase. A dNTP is only incorporated into the growing DNA strand if it is complementary to the base in the template strand. The synthesis of DNA is accompanied by the release of PPi equal in molarity to the incorporated dNTP. The PPi is converted to ATP and the light generated by the luciferase is directly proportional to the amount of ATP. In some cases the unincorporated dNTPs and the produced ATP are degraded between each cycle by the nucleotide degrading enzyme.

In some cases the DNA template is associated with a solid support. To this end, there are a wide variety of known methods of attaching DNAs to solid supports. Recent work has focused on the attachment of binding ligands, including nucleic acid probes, to microspheres that are randomly distributed on a surface, including a fiber optic bundle, to form high density arrays. See for example WO99/18434, WO99/67641, and WO98/48726; WO98/50782; US2001-0029044, US6327410, US6424027, US2002-0051971, US654432, US6429027, US2002-051971 and US6544732. WO99/18434 discloses a microsphere-based fibre optic system for a range of uses, whereas Czamik, A.W., Illuminating the SNP Genomic Code describes the use of a similar system for analyzing gene sequences.

An additional technique utilizes sequencing by hybridization. For example, sequencing by hybridization has been described (Drmanac et al., Genomics 4: 114 (1989); U. S. Patent Nos. 5,525,464; 5,202,231 and 5,695,940, among others.

In addition, sequencing using mass spectrometry techniques has been described; see Koster et al., Nature Biotechnology 14: 1123 (1996).

Finally, the use of adapter-type sequences that allow the use of universal arrays has been described in limited contexts; see for example Chee et al., Nucl. Acid Res. 19: 3301 (1991); Shoemaker et al., Nature Genetics 14: 450 (1998); Barany, F. (1991) Proc. Natl. Acad. Sci. USA 88: 189-193; EP 0 799 897 A1; WO 97/31256.

WO99/18434, WO99/67461 and WO98/40726; WO98/50782; and US 7,348,181, US 6,327,410, US 6,429,027, US 2002-0051971 and US 6,544,732 describe novel compositions utilizing substrates with microsphere arrays, which allow for novel detection methods of nucleic acid hybridization.

Accordingly, it is an object of the present disclosure to provide detection and quantification methods for a variety of nucleic acid reactions, including genotyping, amplification reactions and sequencing reactions, utilizing microsphere arrays.

## SUMMARY OF THE INVENTION

In accordance with the above objects, the present invention provides methods of determining the identity of a nucleotide at a detection position In a target sequence. The methods comprise providing a hybridization complex comprising the target sequence and a capture probe covalentty attached to a microsphere on a surface of a substrate. The methods comprise determining the nucleotide at the detection position. The hybridization complex can comprise the capture probe, a capture extender probe, and the target sequence. In addition, the target sequence may comprise exogeneous adapter sequences.

In a further aspect, the present invention provides methods of sequencing a plurality of target nucleic adds. The methods comprise providing a plurality of hybridization complexes each comprising a target sequence and a sequencing primer that hybridizes to the first domain of the target sequence, the hybridization complexes are attached to a surface of a substrate. The methods comprise extending each of the primers by the addition of a first nucleotide to the first detection position using an enzyme to form an extended primer. The methods comprise detecting the release of pyrophosphate (PPi) to determine the type of the first nucleotide added onto the primers. In one aspect the hybridization complexes are attached to microspheres distributed on the surface. In an additional aspect the sequencing primer are attached to the surface. The hybridization complexes comprise the target sequence, the sequencing primer and a capture probe covalently attached to the surface. The hybridization complexes also comprise an adapter probe.

In an additional aspect, the method comprises extending the extended primer by the addition of a second nucleotide to the second detection position using an enzyme and detecting the release of pyrophosphate to determine the type of second nucleotide added onto the primers. In an additional aspect, the pyrophosphate is detected by contacting the pyrophosphate with a second enzyme that converts pyrophosphate into ATP, and detecting the ATP using a third enzyme. In one aspect, the second enzyme is sulfurylase and/or the third enzyme is luciferase.

In an additional aspect, the invention provides methods of sequencing a target nucleic acid comprising a first domain and an adjacent second domain, the second domain comprising a plurality of target positions. The method comprises providing a hybridization complex comprising the target sequence and a capture probe covalently attached to microspheres on a surface of a substrate and determining the identity of a plurality of bases at the target positions. The hybridization complex comprises the capture probe, an adapter probe, and the target sequence. In one aspect the sequencing primer is the capture probe.

In an additional aspect of the invention, the determining comprises providing a sequencing primer hybridized to the second domain, extending the primer by the addition of first nucleotide to the first detection position using a first enzyme to form an extended primer, detecting the release of pyrophosphate to determine the type of the first nucleotide added onto the primer, extending the primer by the addition of a second nucleotide to the second detection position using the enzyme, and detecting the release of pyrophosphate to determine the type of the second nucleotide added onto the primer. In an additional aspect pyrophosphate is detected by contacting the pyrophosphate with the second enzyme that converts pyrophosphate into ATP, and detecting the ATP using a third enzyme. In one aspect the second enzyme is sulfurylase and/or the third enzyme is luciferase.

In an additional aspect of the method for sequencing, the determining comprises providing a sequencing primer hybridized to the second domain, extending the primer by the addition of a first protected nucleotide using a first enzyme to form an extended primer, determining the identification of the first protected nucleotide, removing the protection group, adding a second protected nucleotide using the enzyme, and determining the identification of the second protected nucleotide.

In an additional aspect the invention provides a kit for nucleic acid sequencing comprising a composition comprising a substrate with a surface comprising discrete sites and a population of microspheres distributed on the sites, wherein the microspheres comprise capture probes. The kit also comprises an extension enzyme and dNTPs. The kit also comprises a second enzyme for the conversion of pyrophosphate to ATP and a third enzyme for the detection of ATP. In one aspect the dNTPs are labeled. In addition each dNTP comprises a different label.

Further disclosed herein are methods of detecting a target nucleic acid sequence comprising attaching a first adapter nucleic acid to a first target nucleic add sequence to form a modified first target nucleic acid sequence, and contacting the modified first target nucleic acid sequence with an array as outlined herein. The presence of the modified first target nucleic acid sequence is then detected.

In an additional aspect, the methods further comprise attaching a second adapter nucleic acid to a second target nucleic add sequence to form a modified second target nucleic acid sequence and contacting the modified second target nucleic acid sequence with the array.

## BRIEF DESCRIPTION OF THE DRAWINGS

Figures 1A, 1B and 1C depict three different means for attaching a target sequence to an array. The solid support 5 has microsphere 10 with capture probe 20 linked via a linker 15. Figure 1A depicts direct attachment; the capture probe 20 hybridizes to a first portion of the target sequence 25. Figure 1B depicts the use of a capture extender probe 30 that has a first portion that hybridizes to the capture probe 20 and a second portion that hybridizes to a first domain of the target sequence 25. Figure 1C shows the use of an adapter sequence 35, that has been added to the target sequence, for example during an amplification reaction as outlined herein.

Figures 9A, 9B. 9C, 9D, 9E, 9F and 9G depict means for SBE genotyping. Figure 9A depicts a "sandwich" assay, in which substrate 5 has a discrete site with a microsphere 10 comprising a capture probe 20 attached via a linker 15. The target sequence 25 has a first domain that hybridizes to the capture probe 20 and a second domain comprising a detection position 30 that hybridizes to an extension primer 50. As will be appreciated by those in the art, Figure 9A depicts a single detection position; however, depending on the system, a plurality of different primers can hybridize to different target domains; hence n is an integer of 1 or greater. In addition, the first domain of the target sequence may be an adapter sequence. Figure 9B depicts the use of a capture probe 20 that also serves as an extension primer. Figure 9D depicts the use of a capture extender probe 100, that has a first domain that will hybridize to the capture probe 20 and a second domain that will hybridize to a first domain of the target sequence 25.

## DETAILED DESCRIPTION OF THE INVENTION

The present disclosure is directed to the detection and quantification of a variety of nucleic acid reactions, particularly using microsphere arrays. In particular, it relates to the detection of amplification, genotyping, and sequencing reactions. In addition, it can be utilized with adapter sequences to create universal arrays.

Accordingly, the present disclosure provides compositions and methods for detecting and/or quantifying the products of nucleic acid reactions, such as target nucleic acid sequences, in a sample. As will be appreciated by those in the art, the sample solution may comprise any number of things, including, but not limited to, bodily fluids (including, but not limited to, blood, urine, serum, lymph, saliva, anal and vaginal secretions, perspiration and semen, of virtually any organism, with mammalian samples being preferred and human samples being particularly preferred); environmental samples (including, but not limited to, air, agricultural, water and soil samples); biological warfare agent samples; research samples; purified samples, such as purified genomic DNA, RNA, proteins, etc.; raw samples (bacteria, virus, genomic DNA, etc.; As will be appreciated by those in the art, virtually any experimental manipulation may have been done on the sample.

The present disclosure provides compositions and methods for detecting the presence or absence of target nucleic acid sequences in a sample. By "nucleic acid" or "oligonucleotide" or grammatical equivalents herein means at least two nucleotides covalently linked together. A nucleic acid of the present disclosure will generally contain phosphodiester bonds, although in some cases, as outlined below, nucleic acid analogs are included that may have alternate backbones, comprising, for example, phosphoramide (Beaucage et al., Tetrahedron 49(10):1925 (1993) and references therein; Letsinger, J. Org. Chem. 35:3800 (1970); Sprinzl et al., Eur. J. Blochem. 81:579 (1977); Letsinger et al., Nucl. Acids Res. 14:3487 (1988); Sawal et al, Chem. Lett. 805 (1984), Letsinger et al., J. Am. Chem. Soc. 110:4470 (1988); and Pauwels et al., Chemica Scripta 26:141 91886)), phosphorothioate (Mag et al., Nucleic Acids Res. 19:1437 (1891); and U.S. Patent No. 5,844,048), phosphorodithioate (Briu et al., J. Am. Chem. Soc. 111:2321 (1989), O-methylphophoroamidite linkages (see Eckstein, Oligonucleotides and Analogues: A Radical Approach, Oxford University Press), and peptide nucleic add backbones and linkages (see Egholm, J. Am. Chem. Soc. 114:1895 (1992); Meier et al., Chem. Int. Ed. Engl. 31:1008 (1992); Nielsen, Nature, 365:566 (1993); Carlsson et al., Nature 380:207 (1996), all of which are incorporated by reference). Other analog nucleic adds include those with positive backbones (Denpcy et al., Proc. Natl. Acad. Sci. USA 92:6097 (1995); non-ionic backbones (U.S. Patent Nos. 5,386,023, 5,637,684, 5,602,240, 5,216,141 and 4,469,863; Kledrowshi et al., Angew. Chem. Intl. Ed. English 30:423 (1991); Letsinger et al., J. Am. Chem. Soc. 110:4470 (1988); Letsinger et al., Nucleoside & Nucleotide 13:1597 (1994); Chapters 2 and 3, ASC Symposium Series 580, "Carbohydrate Modifications in Antisense Research", Ed. Y.S. Sanghui and P. Dan Cook; Mesmaeker et al., Bioorganic & Medicinal Chem. Lett. 4:395 (1994); Jeffs et al., J. Biomolecular NMR 34:17 (1994); Tetrahedron Lett 37:743 (1996)) and non-ribose backbones, including those described in U.S. Patent Nos. 5,235,033 and 5,034,506, and Chapters 6 and 7, ASC Symposium Series 580, "Carbohydrate Modifications in Antisense Research", Ed. Y.S. Songhui and P. Dan Cook. Nucleic acids containing one or more carbocyclic sugars are also included within the definition of nucleic acids (see Jenkins et al., Chem. Soc. Rev. (1995) pb169-176). Several nucleic acid analogs are described in Rawis, C & E News June 2, 1997 page 35.

. These modifications of the ribose-phosphate backbone may be done to facilitate the addition of labels, or to increases the stability and half-life of such molecules in physiological environments.

As will be appreciated by those in the art, all of these nucleic add analogs may find use in the present invention, in addition, mixtures of naturally occurring nucleic acids and analogs can be made. Alternatively, mixtures of different nucleic acid analogs, and mixtures of naturally occuring nucleic adds and analogs may be made.

Particularly preferred are peptide nucleic acids (PNA) which includes peptide nucleic acid analogs. These backbones are substantial non-ionic under neutral conditions, in contrast to the highly charged phosphodiester backbone of naturally occurring nucleic acids. This results in two advantages. First the PNA backbone exhibits improved hybridization kinetics. PNAs have larger changes in the melting temperature (Tm) for mismatched versus perfectly matched basepairs. DNA and RNA typically exhibit a 2-4°C drop in Tm for an internal mismatch. With the non-ionic PNA backbone, the drop is closer to 7-9°C. This allows for better detection of mismatches. Similarly, due to their non-ionic nature, hybridization of the bases attached to these backbones is relatively insensitive to salt concentration.

The nucleic acids may be single stranded or double stranded, as specified, or contain portions of both double stranded or single stranded sequence. The nucleic acid may be DNA, both genomic and cDNA, RNA or a hybrid, where the nucleic acid contains any combination of deoxyribo- and ribonucleotide, and any combination of bases, including uracil, adenine, thymine, cytosine, guanine, inosine, xathanine hypoxathanine, isocytosine, isoguanine, etc. A preferred embodiment utilizes isocytosine and isoguanine in nucleic acids designed to be complementary to other probes, rather than target sequences, as this reduces non-specific hybridization, as is generally described in U.S. Patent No. 5,681,702. As used herein, the term "nucleoside" includes nucleotides as well as nucleoside and nucleotide analogs, and modified nucleosides such as amino modified nucleosides. In addition, "nucleoside" includes non-naturally occurring analog structures. Thus for example the individual units of a peptide nucleic acid, each containing a base, are referred to herein as a nucleoside.

The compositions and methods of the disclosure are directed to the detection of target sequences. The term "target sequence" or target nucleic acid" or grammatical equivalents herein means a nucleic add sequence on a single strand of nucleic acid. The target sequence may be a portion of a gene, a regulatory sequence, genomic DNA, cDNA, RNA inducing mRNA and rRNA, or others. As is outlined herein, the target sequence may be a target sequence from a sample, or a secondary target such as a product of a reaction such as a detection sequence from an invasive cleavage reaction, a ligated probe from an OLA reaction, an extended probe from a PCR or SBE reaction, etc. Thus, for example, a target sequence from a sample is amplified to produce a secondary target that is detected; alternatively, an amplification step is done using a signal probe that is amplified, again producing a secondary target that is detected. The target sequence may be any length, with the understanding that longer sequences are more specific. As will be appreciated by those in the art, the complementary target sequence may take many forms. For example, it may be contained within a larger nucleic acid sequence, i.e. all or part of a gene or mRNA, a restriction fragment of a plasmid or genomic DNA, among others. As is outline more fully below, probes are made to hybridize to target sequences to determine the presence, absence or quantity of a target sequence in a sample. Generally speaking, this term will be understood by those skilled in the art. The target sequence may also be comprised of different target domains; for example, in "sandwich" type assays as outlined below, a first target domain of the sample target sequence may hybridize to a capture probe or a portion of capture extender probe, a second target domain may hybridize to a portion of an amplifier probe, a label probe, or a different capture or capture extender probe, etc. In addition, the target domains may be adjacent (i.e. contiguous) or separated. The terms "first" and "second" are not meant to confer an orientation of the sequences with respect to the 5'-3' orientation of the target sequence. For example, assuming a 5'-3' orientation of the complementary target sequence, the first target domain may be located either 5' to the second domain, or 3' to the second domain. In addition, as will be appreciated by those in the art, the probes on the surface of the array (e. g. attached to the microspheres) may be attached in either orientation, either such that they have a free 3' end or a free 5' end; in some embodiments, the probes can be attached at one or more internal positions, or at both ends.

If required, the target sequence is prepared using known techniques. For example, the sample may be treated to lyse the cells, using known lysis buffers, sonication, electroporafion, etc., with purification and amplification as outlined below occurring as needed, as will be appreciated by those in the art. In addition, the reactions outlined herein may be accomplished in a variety of ways, as will be appreciated by those in the art. Components of the reaction may be added simultaneously, or sequentially, in any order, with preferred embodiments outlined below. In addition, the reaction may include a variety of other reagents which may be included in the assays. These include reagents like salts, buffers, neutral proteins, e. g. albumin, detergents, etc., which may be used to facilitate optimal hybridization and detection, and/or reduce non-specific or background interactions. Also reagents that otherwise improve the efficiency of the assay, such as protease inhibitors, nuclease inhibitors, antimicrobial agents, etc., may be used, depending on the sample preparation methods and purity of the target.

In addition, in most embodiments, double stranded target nucleic acids are denatured to render them single stranded so as to permit hybridization of the primers and other probes of the invention. A preferred embodiment utilizes a thermal step, generally by raising the temperature of the reaction to about 95°C, although pH changes and other techniques may also be used.

As outlined herein, the disclosure provides a number of different primers and probes. By "primer nucleic acid" herein is meant a probe nucleic acid that will hybridize to some portion, i.e. a domain, of the target sequence. Probes of the present disclosure are designed to be complementary to a target sequence (either the target sequence of the sample or to other probe sequences, as is described below), such that hybridization of the target sequence and the probes occurs. As outlined below, this complementarity need not be perfect; there may be any number of base pair mismatches which will interfere with hybridization between the target sequence and the single stranded nucleic acids of the present invention. However, if the number of mutations is so great that no hybridization can occur under even the least stringent of hybridization conditions, the sequence is not a complementary target sequence. Thus, by substantial complementary" herein is meant that the probes are sufficiently complementary to the target sequences to hybridize under normal reaction conditions.

A variety of hybridization conditions may be used in the present invention, including high, moderate and low stringency conditions; see for example Manlatis et al., Molecular Cloning: A Laboratory Manual, 2d Edition, 1989, and Short Protocols in Molecular Biology, ed. Ausubel, et al. Stringent conditions are sequence-dependent and will be different In different circumstances. Longer sequences hybridize specifically at higher temperatures. An extensive guide to the hybridization of nucleic adds Is found in Tijssen, Techniques in Biochemistry and Molecular Biology-Hybridization with Nucleic Acid Probes, "Overview of principles of hybridization and the strategy of nucleic add assays" (1993). Generally, stringent conditions are selected to be about 5-10°C lower than the thermal melting point (Tm) for the specific sequence at a defined ionic strength and pH. The Tm is the temperature (under defined ionic strength, pH and nucleic acid concentration) at which 50% of the probes complementary to the target hybridize to the target sequence at equilibrium (as the target sequences are present in excess, at Tm, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e.g. 10 to 50 nucleotides) and at least about 60°C for long probes (e.g. greater than 50 nucleotides). Stringent conditions may also be achieved with the addition of helix destabilizing agents such as formamide. The hybridization conditions may also vary when a non-ionic backbone, i.e. PNA is used, as is known in the art. In addition, cross-linking agents may be added after target binding to cross-link, i.e. covalently attach, the two strands of the hybridization complex.

Thus, the assays are generally run under stringency conditions which allows formation of the hybridization complex only in the presence of target Stringency can be controlled by altering a step parameter that is a thermodynamic variable, including, but not limited to, temperature, formamide concentration, salt concentration, chaotropic salt concentration, pH, organic solvent concentration, etc.

These parameters may also be used to control non-specific binding, as is generally outlined in U.S. Patent No. 5,681,697. Thus it may be desirable to perform certain steps at higher stringency conditions to reduce non-specific binding.

The size of the primer nucleic acid may vary, as will be appreciated by those in the art, in general varying from 5 to 500 nucleotides in length, with primers of between 10 and 100 being preferred, between 15 and 50 being particularly preferred, and from 10 to 35 being especially preferred, depending on the use and amplification technique.

In addition, the different amplification techniques may have further requirements of the primers, as is more fully described below.

In addition, as outlined herein, a variety of labeling techniques can be done.

Labeling techniques

In general, either direct or indirect detection of the target products can be done. "Direct" detection as used In this context, as for the other reactions outlined herein, requires the incorporation of a label, in this case a detectable label, preferably an optical label such as a fluorophore, into the target sequence, with detection proceeding as outlined below. In this embodiment, the label (s) may be incorporated In a variety of ways: (1) the primers comprise the label(s), for example attached to the base, a ribose, a phosphate, or to analogous structures in a nucleic acid analog; (2) modified nucleosides are used that are modified at either the base or the ribose (or to analogous structures in a nucleic acid analog) with the label(s); these label-modified nucleosides are then converted to the triphosphate form and are incorporated into a newly synthesized strand by a polymerase; (3) modified nucleotides are used that comprise a functional group that can be used to add a detectable label; (4) modified primers are used that comprise a functional group that can be used to add a detectable label or (5) a label probe that is directly labeled and hybridizes to a portion of the target sequence can be used. Any of these methods result in a newly synthesized strand or reaction product that comprises labels, that can be directly detected as outlined below.

Thus, the modified strands comprise a detection label. By "detection label" or "detectable label" herein is meant a moiety that allows detection. This may be a primary label or a secondary label. Accordingly, detection labels may be primary labels (i.e. directly detectable) or secondary labels (indirectly detectable).

In one aspect, the detection label is a primary label. A primary label is one that can be directly detected, such as a fluorophore. In general, labels fall into three classes: a) isotopic labels, which may be radioactive or heavy isotopes; b) magnetic, electrical, thermal labels; and c) colored or luminescent dyes. Labels can also include enzymes (horseradish peroxidase, etc.) and magnetic particles. Preferred labels include chromophores or phosphors but are preferably fluorescent dyes. Suitable dyes for use In the invention include, but are not limited to, fluorescent lanthanide complexes, including those of Europium and Terbium, fluorescein, rhodamine, tetramethylrhodamine, eosin, erythrosin, coumarin, methylcoumarins, quantum dots (also referred to as "nanocrystals": see U.S. 6544732), pyrene, Malacite green, stilbene, Lucifer Yellow, Cascade Blue™, Texas Red, Cy dyes (Cy3, Cy5, etc.), alexa dyes, phycoerythin, bodipy, and others described In the 6th Edition of the Molecular Probes Handbook by Richard P. Haugland

In another aspect, a secondary detectable label is used. A secondary label is one that Is indirectly detected; for example, a secondary label can bind or react with a primary label for detection, can act on an additional product to generate a primary label (e.g. enzymes), or may allow the separation of the compound comprising the secondary label from unlabeled materials, etc. Secondary labels find particular use in systems requiring separation of labeled and unlabeled probes, such as SBE, OLA, invasive cleavage reactions, etc; in addition, these techniques may be used with many of the other techniques described herein. Secondary labels include, but are not limited to, one of a binding partner pair, chemically modifiable moieties; nuclease inhibitors, enzymes such as horseradish peroxidase, alkaline phosphatases, luciferases, etc.

Preferably, the secondary label is a binding partner pair. For example, the label may be a hapten or antigen, which will bind its binding partner. In a preferred embodiments, the binding partner can be attached to a solid support to allow separation of extended and non-extended primers. For example, suitable binding partner pairs include, but are not limited to: antigens (such as proteins (including peptides)) and antibodies (including fragments thereof (FAbs, etc.)); proteins and small molecules, including biotin/streptavidin; enzymes and substrates or inhibitors; other protein-protein interacting pairs; receptor-ligands; and carbohydrates and their binding partners. Nucleic add - nucleic acid binding proteins pairs are also useful. In general, the smaller of the pair is attached to the NTP for incorporation into the primer. Preferred binding partner pairs include, but are not limited to, biotin (or imino-blotin) and streptavidin, digeoxinin and Abs, and Prolinx™ reagents (see www.prolinxinc.com/ie4/home.hmtl).

Preferably, the binding partner pair comprises blotin or imino-blotin and streptavidin. Imino-biotin is particularly preferred as imino-biotin disassociates from streptavidin in pH 4.0 buffer while biotin requires harsh denaturants (e.g. 6 M guanidinium HCl, pH 1.5 or 90% formamide at 95°C).

In one aspect, the binding partner pair comprises a primary detection label (for example, attached to the NTP and therefore to the extended primer) and an antibody that will specifically bind to the primary detection label. By "specifically bind" herein is meant that the partners bind with specificity sufficient to differentiate between the pair and other components or contaminants of the system. The binding should be sufficient to remain bound under the conditions of the assay, including wash steps to remove non-specific binding. In some embodiments, the dissociation constants of the pair will be less than about $10^{-4}$-$10^{-6}$ $M^{-1}$, with less than about $10^{-5}$ to $10^{-8}$ $M^{-1}$ being preferred and less than about $10^{-7}$-$10^{-8}$ $M^{-1}$ being particularly preferred.

In one aspect, the secondary label is a chemically modifiable moiety. In this embodiment, labels comprising reactive functional groups are incorporated Into the nucleic acid. The functional group can then be subsequently labeled with a primary label. Suitable functional groups include, but are not limited to, amino groups, carboxy groups, maleimide groups, oxo groups and thiol groups, with amino groups and thiol groups being particularly preferred. For example, primary labels containing amino groups can be attached to secondary labels comprising amino groups, for example using linkers as are known in the art; for example, homo-or hetero-bifunctional linkers as are well known (see 1994 Pierce Chemical Company catalog, technical section on cross-linkers, pages 155-200).

For removal of unextended primers, it is preferred that the other half of the binding pair is attached to a solid support. In this embodiment, the solid support may be any as described herein for substrates and microspheres, and the form is preferably microspheres as well; for example, a preferred embodiment utilizes magnetic beads that can be easily introduced to the sample and easily removed, although any affinity chromatography formats may be used as well. Standard methods are used to attach the binding partner to the solid support, and can include direct or indirect attachment methods. For example, biotin labeled antibodies to fluorophores can be attached to streptavidin coated magnetic beads.

Thus, in this aspect, the extended primers comprise a binding partner that is contacted with its binding partner under conditions wherein the extended or reacted primers are separated from the unextended or unreacted primers. These modified primers can then be added to the array comprising capture probes as described herein.

<u>Removal of unextended primers</u>

Iit is desirable to remove the unextended or unreacted primers from the assay mixture, and particularly from the array, as unextended primers will compete with the extended (labeled) primers in binding to capture probes, thereby diminishing the signal. The concentration of the unextended primers relative to the extended primer may be relative high, since a large excess of primer is usually required to generate efficient primer annealing. Accordingly, a number of different techniques may be used to facilitate the removal of unextended primers.

In one aspect, the one or more of the probes comprise a secondary detectable label that can be used to separate extended and non-extended primers. As outlined above, detection labels may be primary labels (i. e. directly detectable) or secondary labels (indirectly detectable). A secondary label is one that is indirectly detected; for example, a secondary label can bind or react with a primary label for detection, or may allow the separation of the compound comprising the secondary label from unlabeled materials, etc. Secondary labels find particular use in systems requiring separation of labeled and unlabeled probes, such as SBE, OLA, invasive cleavage, etc. reactions; in addition, these techniques may be used with many of the other techniques described herein. Secondary labels include, but are not limited to, one of a binding partner pair; chemically modifiable moieties; nuclease inhibitors, etc.

Optionally, the secondary label is a binding partner pair as outlined above, in a preferred embodiment, the binding partner pair comprises biotin or imino-biotin and streptavidin. Imino-biotin is particularly preferred when the methods require the later separation of the pair, as imino-biotin disassociates from streptavidin in pH 4.0 buffer while biotin requires harsh denaturants (e.g. 6 M guanidinium HCl, pH 1.5 or 90% formamide at 95°C).

In addition, the use of sreptavidin/blotin systems can be used to separate unreacted and reacted probes (for example in SBE, invasive cleavage, etc.). For example, the addition of streptavidin to a nucleic acid greatly increases its size, as well as changes its physical properties, to allow more efficient separation techniques. For example, the mixtures can be size fractionated by exclusion chromatography, affinity chromatography, filtration or differential precipitation. Alternatively, an 3' exonuclease may be added to a mixture of 3' labeled blotin/streptavidin; only the unreacted oligonucleotides will be degraded. Following exonuclease treatment, the exonuclease and the streptavidin can be degraded using a protease such as proteinase K. The surviving nucleic acids (i.e. those that were biotinylated) are then hybridized to the array.

Optionally, the binding partner pair comprises a primary detection label (attached to the NTP and therefore to the extended primer) and an antibody that will specifically bind to the primary detection label.

In this aspect, it is preferred that the other half of the binding pair is attached to a solid support. In this embodiment, the solid support may be any as described herein for substrates and microspheres, and the form is preferably microspheres as well; for example, a preferred embodiment utilizes magnetic beads that can be easily introduced to the sample and easily removed, although any affinity chromatography formats may be used as well. Standard methods are used to attach the binding partner to the solid support, and can include direct or indirect attachment methods. For example, biotin labeled antibodies to fluorophores can be attached to streptavidin coated magnetic beads.

Thus, in this aspect, the extended primers comprise a binding member that is contacted with its binding partner under conditions wherein the extended primers are separated from the unextended primers. These extended primers can then be added to the array comprising capture probes as described herein.

Optionally, the secondary label is a chemically modifiable moiety. In this embodiment, labels comprising reactive functional groups are incorporated into the nucleic acid.

Preferably, the secondary label is a nuclease inhibitor. In this embodiment, the chainterminating NTPs are chosen to render extended primers resistant to nucleases, such as 3'-exonucleases. Addition of an exonuclease will digest the non-extended primers leaving only the extended primers to bind to the capture probes on the array. This may also be done with OLA, wherein the ligated probe will be protected but the unprotected ligation probe will be digested.

In this aspect, suitable 3'-exonucleases include, but are not limited to, exo 1, exo III, exo Vil, etc.

The present disclosure provides a variety of amplification reactions that can be detected using the arrays of the disclosure.

## AMPLIFICATION REACTIONS

Suitable amplification methods include, but are not limited to, polymerase chain reaction (PCR), strand displacement assay (SDA), transcription mediated amplification (TMA), nucleic acid sequence based amplification (NASBA) and rolling circle amplification (RCA). All of these methods require a primer nucleic acid (including nucleic acid analogs) that is hybridized to a target sequence to form a hybridization complex, and an enzyme is added that in some way modifies the primer to form a modified primer. For example, PCR generally requires two primers, dNTPs and a DNA polymerase; etc. Thus, in general, a target nucleic acid is added to a reaction mixture that comprises the necessary amplification components, and a modified primer is formed.

In general, the modified primer comprises a detectable label, such as a fluorescent label, which is either incorporated by the enzyme or present on the original primer. As required, the unreacted primers are removed, in a variety of ways, as will be appreciated by those in the art and outlined herein. The hybridization complex is then disassociated, and the modified primer is detected and optionally quantitated by a microsphere array. In some cases, the newly modified primer serves as a target sequence for a secondary reaction, which then produces a number of amplified strands, which can be detected as outlined herein.

Accordingly, the reaction starts with the addition of a primer nucleic acid to the target sequence which forms a hybridization complex. Once the hybridization complex between the primer and the target sequence has been formed, an enzyme, sometimes termed an"amplification enzyme", is used to modify the primer. As for all the methods outlined herein, the enzymes may be added at any point during the assay, either prior to, during, or after the addition of the primers. The identity of the enzyme will depend on the amplification technique used, as is more fully outlined below. Similarly, the modification will depend on the amplification technique, as outlined below.

Once the enzyme has modified the primer to form a modified primer, the hybridization complex is dissaccociated. In one aspect, dissociation is by modification of the assy conditions. In another aspect, the modified primer no longer hybridizes to the target nucleic acid and dissociates. Either one or both of these aspects can be employed in signal and target amplification reactions as described below. Generally, the amplification steps are repeated for a period of time to allow a number of cycles, depending on the number of copies of the original target sequence and the sensitivity of detection, with cycles ranging from 1 to thousands, with from 10 to 100 cycles being preferred and from 20 to 50 cycles being especially preferred.

After a suiatable time of amplification, unreacted primers are removed, in a variety of ways, as will be appreciarted by those in the art and described below, and the hybridization complex is disassociated. In general, the modified primer comprises a detachable lable, such as a fluorescent label, which is either incorporated by the enzyme or present on the original primer, and the modified primer is added to a microsphere array such is generally described in US7115884, US 6210910, US 200100290049, US 6327410, US2002-009719 and US 6429027; and PCT applications WO99/67641, WO99/18434, WO99/45357 and WO98/40726. The microsphere array comprises subpopulations of microspheres that comprise capture probes that will hybridise to the modified primers. Detection proceeds via detection of the label as an indication of the presence, absence or amount of the target sequence, as is more fully outlined below.

**TARGET AMPLIFICATION**

Target amplification involves the amplification (replication) of the target sequence to be detected, such that the number of copies of the target sequence is increased. Suitable target amplification techniques include, but are not limited to, the pollynmerase chain reaction (PCR), strand displacement amplification (SDA), transcription mediated amplification (TMA) and nucleic acid sequence based amplification (NASBA).

**POLYMERASE CHAIN REACTION AMPLIFICATION**

Optionally, the target amplification technique is PCR. The polymerase chain reaction (PCR) is widely used and described, and involves the use of primer extension compbined with thermal cycling to amplify a target sequence,; see U.S. Patent Nos 4,683,195 and 4,683,202, and PCR Essential Data,

In addition, there are a number of variations of PCR which also find use in the disclosure, including "quantitative competitive PCR" or "QC-PCR". "arbitrarily primed PCR" or "AP-PCR", "immuno-PCR", "Alu-PCR", "PCR single strand conformational polymorphism" or "PCR-SSCP", "reverse transcriptase PCR" or "RT-PCR", "biotin capture PCR", "vectorette PCR", "panhandle PCR", and "PCR select cDNA subtraction", "allele-specific PCR", among others. In some embodiments, PCR is not preferred.

In general, PCR may be briefly described as follows. A double stranded target nucleic add is denatured, generally by raising the temperature, and then cooled in the presence of an excess of a PCR primer, which then hybridizes to the first target strand. A DNA polymerase then acts to extend the primer with dNTPs, resulting in the synthesis of a new strand forming a hybridization complex. The sample is then heated again, to disassociate the hybridization complex, and the process is repeated. By using a second PCR primer for the complementary target strand, rapid and exponential amplification occurs. Thus PCR steps are denaturation, annealing and extension. The particulars of PCR are well known, and include the use of a thermostable polymerase such as Taq I polymerase and thermal cycling.

Accordingly, the PCR reaction requires at least one PCR primer, a polymerase, and a set of dNTPs. As outlined herein, the primers may comprise the label, or one or more of the dNTPs may comprise a label.

In general, as is more fully outlined below, the capture probes on the beads of the array are designed to be substantially complementary to the extended part of the primer; that is, unextended primers will not bind to the capture probes. Alternatively, as further described below, unreacted probes may be removed prior to addition to the array.

STRAND DISPLACEMENT AMPLIFICATION (SDA)

Optionally, the target amplification technique is SODA. Strand displacement amplification (SDA) is generally described in Walker et al., in Molecular Methods for Virus Detection, Academic Press, Inc., 1995, and U.S. Patent Nos. 5,455,166 and 5,130,238,

In general, SDA may be described as follows. A single stranded target nucleic acid, usually a DNA target sequence, is contacted with an SDA primer. An "SDA primer" generally has a length of 25-100 nucleotides, with SDA primers of approximately 35 nucleotides being preferred. An SDA primer is substantially complementary to a region at the 3' end of the target sequence, and the primer has a sequence at its 5' end (outside of the region that is complementary to the target) that is a recognition sequence for a restriction endonuclease, sometimes referred to herein as a "nicking enzyme" or a "nicking ondonuclease", as outlined below. The SDA primer then hybridizes to the target sequence. The SDA reaction mixture also contains a polymerase (an "SDA polymerase", as outlined below) and a mixture of all four deoxynucleoside-triphosphates (also called deoxynucleotides or dNTPs, i.e. dATP, dTTP, dCTP and dGTP), at least one species of which is a substituted or modified dNTP; thus, the SDA primer Is modified, i.e. extended, to form a modified primer, sometimes referred to herein as a "newly synthesized strand". The substituted dNTP is modified such that it will inhibit cleavage in the strand containing the substituted dNTP but will not inhibit cleavage on the other strand. Examples of suitable substituted dNTPs include, but are not limited, 2'deoxyadenosine 5'-O-(1-thiotriphosphate), 5-methyldeoxycytidine 5'-triphosphate, 2'-deoxyuridine 5'-triphosphate, adn 7-deaza-2'-deoxyguanosine 5'-triphosphate. In addition, the substitution of the dNTP may occur after incorporation into a newly synthesized strand; for example, a methylase may be used to add methyl groups to the synthesized strand. In addition, if all the nucleotides are substituted, the polymerase may have 5'→3' exonuclease activity. However, if less than all the nucleotides are substituted, the polymerase preferably lacks 5'→3' exonuclease activity.

As will be appreciated by those in the art, the recognition site/endonuclease pair can be any of a wide variety of known combinations. The endonuclease is chosen to cleave a strand either at the recognition site, or either 3' or 5' to it, without cleaving the complementary sequence, either because the enzyme only cleaves one strand or because of the incorporation of the substituted nucleotides. Suitable recognition site/endonuclease pairs are well known in the art; suitable endonucleases include, but are not limited to, HincII, HindII, AvaI, Fnu4HI, TthIIII, NcII, BstXI, BamHI, etc. A chart depicting suitable enzymes, and their corresponding recognition sites and the modified dNTP to use is found in U.S. Patent No. 5,455,166.

Once nicked, a polymerase (an "SDA polymerase") is used to extend the newly nicked strand, 5'→3', thereby creating another newly synthesized strand. The polymerase chosen should be able to intiate 5'→3' polymerization at a nick site, should also displace the polymerized strand downstream from the nick, and should lack 5'→3' exonuclease activity (this may be additionally accomplished by the addition of a blocking agent). Thus, suitable polymerases in SDA include, but are not limited to, the Klenow fragment of DNA polymerase I, SEQUENASE 1.0 and SEQUENASE 2.0 (U.S. Biochemical), T5 DNA polymerase and Phi29 DNA polymerase.

Accordingly, the SDA reaction requires, in no particular order, an SDA primer, an SDA polymerase, a nicking endonuclease, and dNTPs, at least one species of which is modified. Again, as outlined above for PCR, preferred embodiments utilize capture probes complementary to the newly synthesized portion of the primer, rather than the primer region, to allow unextended primers to be removed.

In general, SDA does not require thermocycling. The temperature of the reaction is generally set to be high enough to prevent non-specific hybridization but low enough to allow specific hybridization; this is generally from about 37°C to about 42°C, depending on the enzymes.

Preferably, as for most of the amplification techniques described herein, a second amplification reaction can be done using the complementary target sequence, resulting in a substantial increase in amplification during a set period of time. That is, a second primer nucleic add is hybridized to a second target sequence, that is substantially complementary to the first target sequence, to form a second hybridization complex. The addition of the enzyme, followed by disassociation of the second hybridization complex, results in the generation of a number of newly synthesized second strands.

## NUCLEIC ACID SEQUENCE BASED AMPLIFICATION (NASBA) AND TRANSCRIPTION MEDIATED AMPLIFICATION (TMA)

Optionally , the target amplification technique is nucleic acid sequence based amplification (NASBA). NASBA Is generally described in U.S. Patent No. 5,409,818; Sooknanan et al., Nucleic Add Sequence-Based Amplification, Ch. 12 (pp. 261-285) of Molecular Methods for Virus Detection, Academic Press, 1995; and "Profiting from Gene-based Diagnostics", CTB International Publishing Inc., N.J., 1996, . NASBA Is very similar to both TMA and QBR. Transcription mediated amplification (TMA) is generally described in U.S. Patent Nos. 5,399,491, 5,888,779, 5,705,365, 5,710,029. The main difference between NASBA and TMA is that NASBA utilizes the addition of RNAse H to effect RNA degradation, and TMA relies on inherent RNAse H activity of the reverse transcriptase.

In general, these techniques may be described as follows. A single stranded target nucleic acid, usually an RNA target sequence (sometimes referred to herein as "the first target sequence" or the first template"), is contacted with a first primer, generally referred to herein as a "NASBA primer" (although "TMA primer" is also suitable). Starting with a DNA target sequence is described below. These primers generally have a length of 25-100 nucleotides, with NASBA primers of approximately 50-75 nucleotides being preferred. The first primer is preferably a DNA primer that has at its 3' end a sequence that is substantially complementary to the 3' end of the first template. The first primer also has an RNA polymerase promoter at its 5' end (or its complement (antisense), depending on the configuration of the system). The first primer is then hybridized to the first template to form a first hybridization complex. The reaction mixture also includes a reverse transcriptase enzyme (an "NASBA reverse transcriptase") and a mixture of the four dNTPs, such that the first NASBA primer is modified, i.e. extended, to form a modified first primer, comprising a hybridization complex of RNA (the first template) and DNA (the newly synthesized strand).

By "reverse transcriptase" or "RNA-directed DNA polymerase" herein is meant an enzyme capable of synthesizing DNA from a DNA primer and an RNA template. Suitable RNA-directed DNA polymerases include, but are not limited to, avian myloblastosis virus reverse transcriptase ("AMV RT") and the Moloney murine leukemia virus RT. When the amplification reaction is TMA, the reverse transcriptase enzyme further comprises a RNA degrading activity as outlined below.

In addition to the components listed above, the NASBA reaction also includes an RNA degrading enzyme, also sometimes referred to herein as a ribonuclease, that will hydrolyze RNA of an RNA:DNA hybrid without hydrolyzing single- or double-stranded RNA or DNA. Suitable ribonucleases include, but are not limited to, RNase H from *E. coli* and calf thymus.

The ribonuclease activity degrades the first RNA template in the hybridization complex, resulting in a disassociation of the hybridization complex leaving a first single stranded newly synthesized DNA strand, sometimes referred to herein as "the second template".

In addition, the NASBA reaction also includes a second NASBA primer, generally comprising DNA (although as for all the probes herein, including primers, nucleic acid analogs may also be used). This second NASBA primer has a sequence at its 3' end that is substantially complementary to the 3' end of the second template, and also contains an antisense sequence for a functional promoter and the antisense sequence of a transcription initiation site. Thus, this primer sequence, when used as a template for synthesis of the third DNA template, contains sufficient information to allow specific and efficient binding of an RNA polymerase and initiation of transcription at the desired site. Preferred embodiments utilizes the antisense promoter and transcription initiation site are that of the T7 RNA polymerase, although other RNA polymerase promoters and initiation sites can be used as well, as outlined below.

The second primer hybridizes to the second template, and a DNA polymerase, also termed a "DNA-directed DNA polymerase", also present in the reaction, synthesizes a third template (a second newly synthesized DNA strand), resulting in second hybridization complex comprising two newly synthesized DNA strands.

Finally, the inclusion of an RNA polymerase and the required four ribonucleoside triphosphates (ribonucleotides or NTPs) results in the synthesis of an RNA strand (a third newly synthesized strand that is essentially the same as the first template). The RNA polymerase, sometimes referred to herein as a "DNA-directed RNA polymerase", recognizes the promoter and specifically initiates RNA synthesis at the initiation site. In addition, the RNA polymerase preferably synthesizes several copies of RNA per DNA duplex. Preferred RNA polymerases include, but are not limited to, T7 RNA polymerase, and other bacteriophage RNA polymerases including those of phage T3, phage ϕII, Salmonella phage sp6, or Pseudomonase phage gh-1.

In some aspects, TMA and NASBA are used with starting DNA target sequences. In this embodiment, it is necessary to utilize the first primer comprising the RNA polymerase promoter and a DNA polymerase enzyme to generate a double stranded DNA hybrid with the newly synthesized strand comprising the promoter sequence. The hybrid is then denatured and the second primer added.

The second primer hybridizes to the second template, and a DNA polymerase, also termed a"DNA-directed DNA polymerase", also present in the reaction, synthesizes a third template (a second newly Accordingly, the NASBA reaction requires, in no particular order, a first NASBA primer, a second NASBA primer comprising an antisense sequence of an RNA polymerase promoter, an RNA polymerase that recognizes the promoter, a reverse transcriptase, a DNA polymerase, an RNA degrading enzyme, NTPs and dNTPs, in addition to the detection components outlined below.

These components result in a single starting RNA template generating a single DNA duplex; however, since this DNA duplex results in the creation of multiple RNA strands, which can then be used to initiate the reaction again, amplification proceeds rapidly.

Accordingly, the TMA reaction requires, in no particular order, a first TMA primer, a second TMA primer comprising an antisense sequence of an RNA polymerase promoter, an RNA polymerase that recognizes the promoter, a reverse transcriptase with RNA degrading activity, a DNA polymerase, NTPs and dNTPs, in addition to the detection components outlined below.

These components result in a single starting RNA template generating a single DNA duplex; however, since this DNA duplex results in the creation of multiple RNA strands, which can then be used to initiate the reaction again, amplification proceeds rapidly.

As outlined herein, the detection of the newly synthesized strands can proceed in several ways. Direct detection can be done when the newly synthesized strands comprise detectable labels, either by incorporation into the primers or by incorporation of modified labelled nucleotides into the growing strand. Alternatively, as is more fully outlined below, indirect detection of unlabelled strands (which now serve as "targets" in the detection mode) can occur using a variety of sandwich assay configurations. As will be appreciated by those in the art, any of the newly synthesized strands can serve as the "target" for form an assay complex on a surface with a capture probe. In NASBA and TMA, it is preferable to utilize the newly formed RNA strands as the target, as this is where significant amplification occurs.

In this way, a number of secondary target molecules are made. As is more fully outlined below, these reactions (that is, the products of these reactions) can be detected in a number of ways.

## SINGLE BASE EXTENSION (SBE)

Optionally, single base extension (SBE; sometimes referred to as "minisequencing") is used for amplification. It should also be noted that SBE finds use in genotyping, as is described below. Briefly, SBE is a technique that utilizes an extension primer that hybridizes to the target nucleic acid. A polymerase (generally a DNA polymerase) is used to extend the 3' end of the primer with a nucleotide analog labeled a detection label as described herein. Based on the fidelity of the enzyme, a nucleotide is only incorporated Into the extension primer if it is complementary to the adjacent base in the target strand. Generally, the nucleotide is derivatized such that no further extensions can occur, so only a single nucleotide is added. However, for amplification reactions, this may not be necessary. Once the labeled nucleotide is added, detection of the label proceeds as outlined herein. See generally Sylvanen et al., Genomics 8:684-692 (1990); U.S. Patent Nos. 5,846,710 and 5,888,819; Pastinen et al., Genomics Res. T(6):808-614 (1997); all of which are expressly incorporated herein by reference.

The reaction is initiated by introducing the assay complex comprising the target sequence (i.e, the array) to a solution comprising a first nucleotide, frequently an nucleotide analog. By "nucleotide analog" in this context herein is meant a deoxynucleoside-triphosphate (also called deoxynucleotides or dNTPs, i.e. dATP, DTTP, dCTP and dGTP), that is further derivatized to be chain terminating. As will be appreciated by those in the art, any number of nucleotide analogs may be used, as long as a polymerase enzyme will still incorporate the nucleotide at the interrogation position. Preferred embodiments utilize dideoxy-triphosphate nucleotides (ddNTPs). Generally, a set of nucleotides comprising ddATP, ddCTP, ddGTP and ddTTP is used, at least one of which includes a label, and preferably all four. For amplification rather than genotyping reactions, the labels may all be the same; alternatively, different labels may be used.

Optionally, the nucleotide analogs comprise a detectable label, which can be either a primary or secondary detectable label. Preferred primary labels are those outlined above. However, the enzymatic incorporation of nucleotides comprising fluorophores is poor under many conditions; accordingly, preferred embodiments utilize secondary detectable labels. In addition, as outlined below, the use of secondary labels may also facilitate the removal of unextended probes.

In addition to a first nucleotide, the solution also comprises an extension enzyme, generally a DNA polymerase. Suitable DNA polymerases include, but are not limited to, the Klenow fragment of DNA polymerase I, SEQUENASE 1.0 and SEQUENASE 2.0 (U.S. Biochemical), T5 DNA polymerase and Phi29 DNA polymerase. If the NTP is complementary to the base of the detection position of the target sequence, which is adjacent to the extension primer, the extension enzyme will add it to the extension primer. Thus, the extension primer is modified, i.e. extended, to form a modified primer, sometimes referred to herein as a "newly synthesized strand".

A limitation of this method is that unless the target nucleic acid is In sufficient concentration, the amount of unextended primer in the reaction greatly exceeds the resultant extended-labeled primer. The excess of unextended primer competes with the detection of the labeled primer in the assays described herein. Accordingly, when SBE is used, preferred embodiments utilize methods for the removal of unextended primers as outlined herein.

One method to overcome this limitation is thermocycling minisequencing in which repeated cycles of annealing, primer extension, and heat denaturation using a thermocycler and thermo-stable polymerase allows the amplification of the extension probe which results in the accumulation of extended primers. For example, if the original unextended primer to target nucleic acid concentration is 100: 1 and 100 thermocycles and extensions are performed, a majority of the primer will be extended.

As will be appreciated by those in the art, the configuration of the SBE system can take on several forms. As for the LCR reaction described below, the reaction may be done in solution, and then the newly synthesized strands, with the base-specific detectable labels, can be detected. For example, they can be directly hybridized to capture probes that are complementary to the extension primers, and the presence of the label is then detected.

Alternatively, the SBE reaction can occur on a surface. For example, a target nucleic acid may be captured using a first capture probe that hybridizes to a first target domain of the target, and the reaction can proceed at a second target domain. The extended labeled primers are then bound to a second capture probe and detected.

Thus, the SBE reaction requires, in no particular order, an extension primer, a polymerase and dNTPs, at least one of which is labeled.

## GENOTYPING

In this embodiment, the disclosure provides compositions and methods for the detection (and optionally quantification) of differences or variations of sequences (e. g. SNPs) using bead arrays for detection of the differences. That is, the bead array serves as a platform on which a variety of techniques may be used to elucidate the nucleotide at the position of interest ("the detection position"). In general, the methods described herein relate to the detection of nucleotide substitutions, although as will be appreciated by those in the art, deletions, insertions, inversions, etc. may also be detected.

As outlined herein, in this embodiment the target sequence comprises a position for which sequence information is desired, generally referred to herein as the "detection position" or "detecton locus". In a preferred embodiment, the detection position is a single nucleotde, although in some embodiments, it may comprise a plurality of nucleotdes, either contiguous with each other or separated by one or more nucleotides. By"plurality"as used herein is meant at least two. As used herein, the base which basepairs with a detection position base in a hybrid is termed a "readout position" or an "interrogation position".

In some embodiments, as is outlined herein, the target sequence may not be the sample target sequence but instead is a product of a reaction herein, sometimes referred to herein as a "secondary" or "derivative" target sequence. Thus, for example, in SBE, the extended primer may serve as the target sequence; similarly, in invasive cleavage variations, the cleaved detection sequence may serve as the target sequence.

As above, if required, the target sequence is prepared using known techniques. Once prepared, the target sequence can be used in a variety of reactions for a variety of reasons. For example, in a preferred embodiment, genotyping reactions are done. Similarly, these reactions can also be used to detect the presence or absence of a target sequence. In addition, in any reaction, quantitation of the amount of a target sequence may be done. While the discussion below focuses on genotyping reactions, the discussion applies equally to detecting the presence of target sequences and/or their quantification.

Furthermore, as outlined below for each reaction, each of these techniques may be used in solid phase assays, where the reaction occurs on the surface and is detected.

## EXTENSION GENOTYPING

In this embodiment, any number of techniques are used to add a nucl otide to the readout position of a probe hybridized to the target sequence adjacent to the detection position. By relying on enzymatic specificity, preferentially a perfectly complementary base is added. All of these methods rely on the enzymatic incorporation of nucleotides at the detection position. This may be done using chain terminating dNTPs, such that only a single base is incorporated (e. g. single base extension methods), or under conditions that only a single type of nucleotide is added followed by identification of the added nucleotide (extension and pyrosequencing techniques).

Single Base Extension

Optionally, single base extension (SBE; sometimes referred to as "minisequencing") is used to determine the identity of the base at the detection position. SBE is as described above, and utilizes an extension primer that hybridizes to the target nucleic acid immediately adjacent to the detection position. A polymerase (generally a DNA polymerase) is used to extend the 3' end of the primer with a nucleotide analog labeled a detection label as described herein. Based on the fidelity of the enzyme, a nucleotide is only incorporated into the readout position of the growing nucleic acid strand if it is perfectly complementary to the base in the target strand at the detection position. The nucleotide may be derivatized such that no further extensions can occur, so only a single nucleotide is added, Once the labeled nucl otide is added, detection of the label proceeds as outlined herein.

The reaction is initiated by introducing the assay complex comprising the target sequence (i.e. the array) to a solution comprising a first nucleotide. In general, the nucleotides comprise a detectable label, which may be either a primary or a secondary label. In addition, the nucleotdes may be nucleotide analogs, depending on the configuration of the system. For example, if the dNTPs are added in sequential reactions, such that only a single type of dNTP can be added, the nucleotdes need not be chain terminating. In addition, in this embodiment, the dNTPs may all comprise the same type of label.

Alternatively, if the reaction comprises more than one dNTP, the dNTPs should be chain terminating, that is, they have a blocking or protecting group at the 3'position such that no further dNTPs may be added by the enzyme. As will be appreciated by those in the art, any number of nucl otide analogs may be used, as long as a polymerase enzyme will still incorporate the nucleotide at the readout position. Preferred embodiments utilize dideoxy-triphosphate nucleotides (ddNTPs) and halogenated dNTPs, Generally, a set of nucleotides comprising ddATP, ddCTP, ddGTP and ddTTP is used, each with a different detectable label, although as outlined herein, this may not be required. Alternative preferred embodiments use acyclo nucleotides (NEN). These chain terminating nucleotide analogs are particularly good substrates for Deep vent (exo) and thermosequenase.

In addition, as will be appreciated by those in the art, the single base extension reactions of the present disclosure allow the precise incorporation of modified bases into a growing nucleic acid strand. Thus, any number of modified nucleotides may be incorporated for any number of reasons, including probing structure-function relationships (e.g. DNA:DNA or DNA:protein interactions), cleaving the nucleic acid, crosslinking the nucleic acid, incorporate mismatches, etc.

As will be appreciated by those in the art, the configuration of the genotyping SBE system can take on several forms.

Solid Phase assay

The reaction may be done on a surface by capturing the target sequence and then running the SBE reaction, in a sandwich type format schematically depicted in Figure 9A. In this aspect, the capture probe hybridizes to a first domain of the target sequence (which can be endogeneous or an exogeneous adapter sequence added during an amplification reaction), and the extension primer hybridizes to a second target domain immediately adjacent to the detection position. The addition of the enzyme and the required NTPs results in the addition of the interrogation base. In this aspect, each NTP must have a unique label. Alternatively, each NTP reaction may be done sequentially on a different array. As is known by one of skill in the art, ddNTP and dNTP are the preferred substrates when DNA polymerase is the added enzyme; NTP is the preferred substrate when RNA polymerase is the added enzyme.

Furthermore, as is more fully outlined below and depicted in Figure 9D, capture extender probes can be used to attach the target sequence to the bead. In this aspect, the hybridization complex comprises the capture probe, the target sequence and the adapter sequence.

Similarly, the capture probe itself can be used as the extension probe, with its terminus being directly adjacent to the detection position. This is schematically depicted in Figure 9B. Upon the addition of the target sequence and the SBE reagents, the modified primer is formed comprising a detectable label, and then detected. Again, as for the solution based reaction, each NTP must have a unique label, the reactions must proceed sequentially, or different arrays must be used. Again, as is known by one of skill in the art, ddNTP and dNTP are the preferred substrates when DNA polymerase is the added enzyme; NTP is the preferred substrate when RNA polymerase is the added enzyme.

In addition, as outlined herein, the target sequence may be directly attached to the array; the extension primer hybridizes to it and the reaction proceeds.

Variations on this are shown in Figures 9E and 9F, where the capture probe and the extension probe adjacently hybridize to the target sequence. Either before or after extension of the extension probe, a ligation step may be used to attach the capture and extension probes together for stability. These are further described below as combination assays.

Removal of unextended primers

Optionally, for both SBE as well as a number of other reactions outlined herein, it is desirable to remove the unextended or unreacted primers from the assay mixture, and particularly from the array, as unextended primers will compete with the extended (labeled) primers in binding to capture probes, thereby diminishing the signal. The concentration of the unextended primers relative to the extended primer may be relatively high, since a large excess of primer is usually required to generate efficient primer annealing. Accordingly, a number of different techniques may be used to facilitate the removal of unextended primers. As outlined above, these generally include methods based on removal of unreacted primers by binding to a solid support, protecting the reacted primers and degrading the unextended ones, and separating the unreacted and reacted primers.

Non-terminated extension

Methods of adding a single base are used that do not rely on chain termination. That is, similar to SBE, enzymatic reactions that utilize dNTPs and polymerases can be used; however, rather than use chain terminating dNTPs, regular dNTPs are used. This method relies on a time-resolved basis of detection; only one type of base is added during the reaction. Thus, for example, four different reactions each containing one of the dNTPs can be done; this is generally accomplished by using four different substrates, although as will be appreciated by those in the art, not all four reactions need occur to identify the nucleotide at a detection position. In this embodiment, the signals from single additions can be compared to those from multiple additions; that is, the addition of a single ATP can be distinguished on the basis of signal intensity from the addition of two or three ATPs. These reactions are accomplished as outlined above for SBE, using extension primers and polymerases; again, one label or four different labels can be used, although as outlined herein, the different NTPs must be added sequentially.

A method of extension in this embodiment is pyrosequencing.

Pyrosequencing

Pyrosequencing is an extension and sequencing method that can be used to add one or more nucleotides to the detection position(s); it is very similar to SBE except that chain terminating NTPs need not be used (although they may be). Pyrosequencing relies on the detection of a reaction product. PPi, produced during the addition of an NTP to a growing oligonucleotide chain, rather than on a label attached to the nucleotde. One molecule of PPi is produced per dNTP added to the extension primer. That is, by running sequential reactions with each of the nucleotides, and monitoring the reaction products, the identity of the added base is determined.

The release of pyrophosphate (PPi) during the DNA polymerase reaction can be quantitatively measured by many different methods and a number of enzymatic methods have been described; see Reeves et al., Anal. Biochem. 28: 282 (1969); Guillory et al., Anal. Biochem. 39: 170 (1971); Johnson et al., Anal. Biochem. 15: 273 (1968); Cook et al., Anal. Biochem. 91: 557 (1978); Drake et al., Anal. Biochem. 94:117 (1979); WO93/23564; WO 98/28440; WO98/13523; Nyren et al., Anal. Biochem. 151: 504 (1985). The latter method allows continuos monitoring of PPi and has been termed ELIDA (Enzymatic Luminometric Inorganic Pyrophosphate Detection Assay). A preferred embodiment utilizes any method which can result in the generation of an optical signal, with preferred embodiments utilizing the generation of a chemiluminescent or fluorescent signal.

A preferred method monitors the creation of PPi by the conversion of PPi to ATP by the enzyme sulfurylase, and the subsequent production of visible light by firefly luciferase (see Ronaghi et al., Science 281: 363 (1998)). In this method, the four deoxynucleotides (dATP, dGTP, dCTP and dTTP; collectively dNTPs) are added stepwise to a partial duplex comprising a sequencing primer hybridized to a single stranded DNA template and incubated with DNA polymerase, ATP sulfurylase, luciferase, and optionally a nucleotide-degrading enzyme such as apyrase. A dNTP is only incorporated into the growing DNA strand if it is complementary to the base in the template strand. The synthesis of DNA is accompanied by the release of PPi equal in molarity to the incorporated dNTP. The PPi is converted to ATP and the light generated by the luciferase is directly proportional to the amount of ATP. In some cases the unincorporated dNTPs and the produced ATP are degraded between each cycle by the nucleotide degrading enzyme.

Accordingly, a preferred embodiment of the methods of the invention is as follows. A substrate comprising microspheres containing the target sequences and extension primers, forming hybridization complexes, is dipped or contacted with a reaction volume (chamber or well) comprising a single type of dNTP, an extension enzyme, and the reagents and enzymes necessary to detect PPi. If the dNTP is complementary to the base of the target portion of the target sequence adjacent to the extension primer, the dNTP is added, releasing PPi and generating detectable light, which is detected as generally described in US 6,327,410 and WO98/50782. If the dNTP is not complementary, no detectable signal results.

The substrate is then contacted with a second reaction volume (chamber) comprising a different dNTP and the additional components of the assay. This process is repeated if the identity of a base at a second detection position is desirable.

In a preferred embodiment, washing steps, i.e. the use of washing chambers, may be done in between the dNTP reaction chambers, as required. These washing chambers may optionally comprise a nucleotide-degrading enzyme, to remove any unreacted dNTP and decreasing the background signal, as is described in WO 98/28440, incorporated herein by reference.

As will be appreciated by those in the art, the system can be configured in a variety of ways, including both a linear progression or a circular one; for example, four arrays may be used that each can dip into one of four reaction chambers arrayed in a circular pattern. Each cycle of sequencing and reading is followed by a 90 degree rotation, so that each substrate then dips into the next reaction well.

In a preferred embodiment, one or more internal control sequences are used. That is, at least one microsphere in the array comprises a known sequence that can be used to verify that the reactions are proceeding correctly. In a preferred embodiment, at least four control sequences are used, each of which has a different nucleotide at each position: the first control sequence will have an adenosine at position 1, the second will have a cytosine, the third a guanosine, and the fourth a thymidine, thus ensuring that at least one control sequence is "lighting up" at each step to serve as an internal control.

As for simple extension and SBE, the pyrosequencing systems may be configured in a variety of ways; for example, the target sequence may be attached to the bead in a variety of ways, including direct attachment of the target sequence; the use of a capture probe with a separate extension probe; the use of a capture extender probe, a capture probe and a separate extension probe; the use of adapter sequences in the target sequence with capture and extension probes; and the use of a capture probe that also serves as the extension probe.

One additional benefit of pyrosequencing for genotyping purposes is that since the reaction does not rely on the incorporation of labels into a growing chain, the unreacted extension primers need not be removed.

## COMBINATION TECHNIQUES

It is also possible to combine two or more of these techniques to do genotyping, quantification,

### Novel combination of competitive hybridization and extension

In a preferred embodiment, a combination of competitive hybridization and extension, particularly SBE, is used. This may be generally described as follows. In this embodiment, different extension primers comprising different bases at the readout position are used. These are hybridized to a target sequence under stringency conditions that favor perfect matches, and then an extension reaction is done. Basically, the readout probe that has the match at the readout position will be preferentially extended for two reasons; first, the readout probe will hybridize more efficiently to the target (e.g. has a slower off rate), and the extension enzyme will preferentially add a nucleotide to a "hybridized" base. The reactions can then be detected In a number of ways, as outlined herein.

The system can take on a number of configurations, depending on the number of labels used, the use of adapters, whether a solution-based or surface-based assay is done, etc. Several preferred embodiments are shown in Figure 14.

In a preferred embodiment, at least two different readout probes are used, each with a different base at the readout position and each with a unique detectable label that allows the identification of the base at the readout position. As described herein, these detectable labels may be either primary or secondary labels, with primary labels being preferred. As for all the competitive hybridization reactions, a competition for hybridization exists with the reaction conditions being set to favor match over mismatch. When the correct match occurs, the 3' end of the hybridization complex is now double stranded and thus serves as a template for an extension enzyme to add at least one base to the probe, at a position adjacent to the readout position. As will be appreciated by those in the art, for most SNP analysis, the nucleotide next to the detection position will be the same in all the reactions.

In one embodiment, chain terminating nucleotides may be used; alternatively, non-terminating nucleotides may be used and multiple nucleotides may be added, if desired. The latter may be particularly preferred as an amplification step of sorts; if the nucleotides are labelled, the addition of multiple labels can results in signal amplification.

In a preferred embodiment, the nucleotides are analogs that allow separation of reacted and unreacted primers as described herein; for example, this may be done by using a nuclease blocking moiety to protect extended primers and allow preferentially degradation of unextended primers or biotin (or iminobiotin) to preferentially remove the extended primers (this is done in a solution based assay, followed by elution and addition to the array).

### Combinabon of ligation and extensions

Optionally, OLA and SBE are combined, as is sometimes referred to as "Genetic Bit" analysis and described in Nikforov et al., Nucleic Acid Res. 22: 4167 (1994). In this aspect, the two ligation probes do not hybridize adjacent; rather, they are separated by one or more bases. The addition of dNTPs and a polymerase, in addition to the ligation probes and the ligase, results in an extended, ligated probe. As for SBE, the dNTPs may carry different labels, or separate reactions can be run, if the SBE portion of the reaction is used for genotyping. Alternatively, if the ligation portion of the reaction is used for genotyping, either no extension occurs due to mismatch of the 3' base (such that the polymerase will not extend it), or no ligation occurs due to mismatch of the 5' base. As will be appreciated by those in the art, the reaction products are assayed using microsphere arrays. Again, as outlined herein, the assays may be solution based assays, with the ligated, extended probes being added to a microsphere array, or solid-phase assays. In addition, the unextended, unligated primers may be removed prior to detection as needed, as is outlined herein. Furthermore, adapter sequences may also be used as outlined herein for OLA.

## SEQUENCING

The present invention is directed to the sequencing of nucleic acids, particularly DNA, by synthesizing nucleic adds using the target sequence (i.e. the nucleic acid for which the sequence is determined) as a template. These methods can be generally described as follows. A target sequence is attached to a solid support, either directly or indirectly, as outlined below. The target sequence comprises a first domain and an adjacent second domain comprising target positions for which sequence information is desired. A sequencing primer is hybridized to the first domain of the target sequence, and an extension enzyme is added, such as a polymerase or a ligase, as outlined below. After the addition of each base, the identity of each newly added base is determined prior to adding the next base. This can be done in a variety of ways, including controlling the reaction rate and using a fast detector, such that the newly added bases are identified in real time. Alternatively, the addition of nucleotides is controlled by reversible chain termination, for example through the use of photocleavable blocking groups. Alternatively, the addition of nucleotides is controlled, so that the reaction is limited to one or a few bases at a time. The reaction is restarted after each cycle of addition and reading. Alternatively, the addition of nucleotides is accomplished by carrying out a ligation reaction with oligonucleotides comprising chain terminating oligonucleotides. Preferred methods of sequencing-by-synthesis include, but are not limited to, pyrosequencing, reversible-chain termination sequencing, time-resolved sequencing, ligation sequencing, and single-molecule analysis, all of which are described below.

The advantages of these "sequencing-by-synthesis" reactions can be augmented through the use of array techniques that allow very high density arrays to be made rapidly and Inexpensively, thus allowing rapid and inexpensive nucleic acid sequencing. By "array techniques" is meant techniques that allow for analysis of a plurality of nucleic acids in an array format. The maximum number of nucleic acids is limited only by the number of discrete loci on a particular array platform. As is more fully outlined below, a number of different array formats can be used.

The methods of the invention find particular use in sequencing a target nucleic acid sequence, i.e. identifying the sequence of a target base or target bases in a target nucleic acid, which can ultimately be used to determine the sequence of long nucleic acids.

As is outlined herein, the target sequence comprises positions for which sequence information is desired, generally referred to herein as the "target positions". In one embodiment, a single target position is elucidated; in a preferred embodiment, a plurality of target positions are elucidated. In general, the plurality of nucleotides in the target positions are contiguous with each other, although in some circumstances they may be separated by one or more nucleotides. By "plurality" as used herein, is meant at least two. As used herein, the base which basepairs with the target position base in a hybrid is termed the "sequence position". That is, as more fully outlined below, the extension of a sequence primer results in nucleotides being added in the sequence positions, that are perfectly complementary to the nucleotides in the target positions. As will be appreciated by one of ordinary skill in the art, identification of a plurality of target positions in a target nucleotide sequence results in the determination of the nucleotide sequence of the target nucleotide sequence.

As will be appreciated by one of ordinary skill in the art, this system can take on a number of different configurations, depending on the sequencing method used, the method of attaching a target sequence to a surface, etc. In general, the methods of the invention rely on the attachment of different target sequences to a solid support (which, as outlined below, can be accomplished in a variety of ways) to form an array. The target sequences comprise at least two domains: a first domain, for which sequence information is not desired, and to which a sequencing primer can hybridize, and a second domain, adjacent to the first domain, comprising the target positions for sequencing. A sequencing primer is hybridized to the target sequence, forming a hybridization complex, and then the sequencing primer is enzymatically extended by the addition of a first nucleotide into the first sequences position of the primer. This first nucleotide is then identified, as is outlined below, and then the process is repeated, to add nucleotides to the second, third, fourth, etc. sequence positions. The exact methods depend on the sequencing technique utilized, as outlined below.

Once the target sequence is associated onto the array as outlined below, the target sequence can be used in a variety of sequencing by synthesis reactions. These reactions are generally classified Into several categories, outlined below.

## SEQUENCING BY SYNTHESIS

As outlined herein, a number of sequencing by synthesis reactions are used to elucidate the identity of a plurality of bases at target positions within the target sequence. All of these reactions rely on the use of a target sequence comprising at least two domains; a first domain to which a sequencing primer will hybridize, and an adjacent second domain, for which sequence information is desired. Upon formation of the assay complex, extension enzymes are used to add dNTPs to the sequencing primer, and each addition of dNTP is "read" to determine the identity of the added dNTP. This may proceed for many cycles.

Pyrosequencing

In the claimed methods, pyrosequencing methods are done to sequence the nucleic acids. As outlined above, pyrosequencing is an extension method that can be used to add one or more nucleotides to the target positions. Pyrosequencing relies on the detection of a reaction product, pyrophosphate (PPi), produced during the addition of an NTP to a growing oligonucleotide chain, rather than on a label attached to the nucleotide. One molecule of PPi is produced per dNTP added to the extension primer. The detection of the PPi produced during the reaction is monitored using secondary enzymes; for example, preferred embodiments utilize secondary enzymes that convert the PPi into ATP, which also may be detected in a variety of ways, for example through a chemiluminescent reaction using luciferase and luciferin, or by the detection of NADPH. Thus, by running sequential reactions with each of the nucleotides, and monitoring the reaction products, the identity of the added base is determined.

Accordingly, the present invention provides methods of pyrosequencing on arrays; the arrays are microsphere arrays. In this embodiment, the target sequence comprises a first domain that is substantially complementary to a sequencing primer, and an adjacent second domain that comprises a plurality of target positions. By "Sequencing primer" herein is meant a nucleic acid that is substantially complementary to the first target domain, with perfect complementarity being preferred. As will be appreciated by those in the art, the length of the sequencing primer will vary with the conditions used. In general, the sequencing primer ranges from about 6 to about 500 or more basepairs in length, with from about 8 to about 100 being preferred, and from about 10 to about 25 being especially preferred.

Once the sequencing primer is added and hybridized to the target sequence to form a first hybridization complex (also sometimes referred to herein as an "assay complex"), the system is ready to initiate sequencing-by-synthesis. The methods described below make reference to the use of fiber optic bundle substrates with associated microspheres.

The reaction is initiated by introducing the substrate comprising the hybridization complex comprising the target sequence (i.e. the array) to a solution comprising a first nucleotide, generally comprising deoxynucleoside-triphosphates (dNTPs). Generally, the dNTPs comprise dATP, dTTP, dCTP and dGTP. The nucleotides may be naturally occurring, such as deoxynueotides, or non-naturally occurring, such as chain terminating nucleotides including dideoxynucleotides, as long as the enzymes used in the sequencing/detection reactions are still capable of recognizing the analogs. In addition, as more fully outlined below, for example in other sequencing-by-synthesis reactions, the nucleotides may comprise labels. The different dNTPs are added either to separate aliquots of the hybridization complex or preferably sequentially to the hybridization complex, as is more fully outlined below. In some embodiments it is important that the hybridization complex be exposed to a single type of dNTP at a time.

In addition, as will be appreciated by those in the art, the extension reactions of the present invention allow the precise incorporation of modified bases into a growing nucleic acid strand. Thus, any number of modified nucleotides may be incorporated for any number of reasons, including probing structure-function relationships (e.g. DNA:DNA or DNA:protein interactions), cleaving the nucleic acid, crosslinking the nucleic acid, incorporate mismatches, etc.

In addition to a first nucleotide, the solution also comprises an extension enzyme, generally a DNA polymerase. Suitable DNA polymerases include,but are not limited to, the Klenow fragment of DNA polymerase I, SEQUENASE 1.0 and SEQUENASE 2.0 (U.S. Biochemical), T5 DNA polymerase and Phi29 DNA polymerase. If the dNTP is complementary to the base of the target sequence adjacent to the extension primer, the extension enzyme will add it to the extension primer, releasing pyrophosphate (PPi). Thus, the extension primer is modified, i.e. extended, to form a modified primer, sometimes referred to herein as a "newly synthesized strand" The incorporation of a dNTP into a newly synthesized nucleic acid strand releases PPi, one molecule of PPi per dNTP incorporated.

The release of pyrophosphate (PPi) during the DNA polymerase reaction can be quantitatively measured by many different methods and a number of enzymatic methods have been described; see Reeves et al., Anal. Biochem. 28:282 (1969); Guillory et al., Anal. Biochem. 39:170 (1971); Johnson et al., Anal. Biochem. 15: 273 (1968); Cook et al., Anal. Biochem. 91:557 (1978); Drake et al., Anal. Biochem. 94:117 (1979); Ronaghi et al., Science 281:363 (1998); Barshop et al., Anal. Biochem. 197(1):266-272 (1991) WO93/23564: WO 98/28440: WO98/13523; Nyren et al., Anal. Biochem. 151:504 (1985); .The latter method allows continuous monitoring of PPi and has been termed ELIDA (Enzymatic Luminometric Inorganic Pyrophosphate Detection Assay). In a preferred embodiment, the PPi Is detected utilizing UDP-glucose pyrophosphorylase, phosphoglucomutase and glucose 6-phosphate dehydrogenase. See Justesen, et al., Anal. Biochem. 207(1):90.93 (1992); Lust et al.. Clin. Chem. Acta 66(2):241 (1976): and Johnson et al., Anal. Biochem. 26:137 (1968); . This reaction produces NADPH which can be detected fluoremetrically. A preferred embodiment utilizes any method which can result in the generation of an optical signal, with preferred embodiments utilizing the generation of a chemiluminescent or fluorescent signal.

Generally, these methods rely on secondary enzymes to detect the PPI; these methods generally rely on enzymes that will convert PPi into ATP, which can then be detected. A preferred method monitors the creation of PPi by the conversion of PPi to ATP by the enzyme sulfurylase, and the subsequent production of visible light by firefly luciferase (see Ronaghi et al., supra, and Barshop, supra). in this method, the four deoxynucleotides (dATP, dGTP, dCTP and dTTP; collectively dNTPs) are added stepwise to a partial duplex comprising a sequencing primer hybridized to a single stranded DNA template and incubated with DNA polymerase, ATP sulfurylase (and its substrate, adenosine 5'-phosphosulphate (APS)) luciferase (and its substrate luciferin), and optionally a nucleotide-degrading enzyme such as apyrase. A dNTP is only incorporated into the growing DNA strand if it is complementary to the base in the template strand. The synthesis of DNA is accompanied by the release of PPi equal in molarity to the incorporated dNTP. The PPi is converted to ATP and the light generated by the luciferase is directly proportional to the amount of ATP. In some cases the unincorporated dNTPs and the produced ATP are degraded between each cycle by the nucleotide degrading enzyme.

As will be appreciated by those in the art, if the target sequence comprises two or more of the same nucleotide in a row, more than one dNTP will be incorporated; however, the amount of PPi generated is directly proportional to the number of dNTPs incorporated and thus these sequences can be detected.

In addition, in a preferred embodiment, the dATP that is added to the reaction mixture is an analog that can be incorporated by the DNA polymerase into the growing oligonucleotide strand, but will not serve as a substrate for the second enzyme; for example, certain thiol-containing dATP analogs find particular use.

Accordingly, a preferred embodiment of the methods of the I nvention is as follows. A substrate comprising microspheres containing the target sequences and extension primers, forming hybridization complexes, is dipped or contacted with a volume (reaction chamber or well) comprising a single type of dNTP, an extension enzyme, and the reagents and enzymes necessary to detect PPi. If the dNTP is complementary to the base of the target portion of the target sequence adjacent to the extension primer, the dNTP is added, releasing PPi and generating detectable light, which is detected as generally described in US 6,327,410 and WO98/50782. If the dNTP is not complementary, no detectable signal results. The substrate is then contacted with a second reaction chamber comprising a different dNTP and the additional components of the assay. This process is repeated to generate a readout of the sequence of the target sequence.

In a preferred embodiment, washing steps, i.e. the use of washing chambers, may be done in between the dNTP reaction chambers, as required. These washing chambers may optionally comprise a nucleotide-degrading enzyme, to remove any unreacted dNTP and decreasing the background signal, as is described in WO 98/28440. In a preferred embodiment a flow cell is used as a reaction chamber, following each reaction the unreacted dNTP is washed away and may be replaced with an additional dNTP to be examined.

As will be appreciated by those in the art, the system can be configured in a variety of ways, including both a linear progression or a circular one; for example, four substrates may be used that each can dip into one of four reaction chambers arrayed in a circular pattern. Each cycle of sequencing and reading is followed by a 90 degree rotation, so that each substrate then dips into the next reaction well. This allows a continuous series of sequencing reactions on multiple substrates in parallel.

In a preferred embodiment, one or more internal control sequences are used. That is, at least one microsphere in the array comprises a known sequence that can be used to verify that the reactions are proceeding correctly. In a preferred embodiment, at least four control sequences are used, each of which has a different nucleotide at each position: the first control sequence will have an adenosine at position 1, the second will have a cytosine, the third a guanosine, and the fourth a thymidine, thus ensuring that at least one control sequence is"lighting up"at each step to serve as an internal control.

In a preferred embodiment, the reaction is run for a number of cycles untel the signal-to-noise ratio becomes low, generally from 20 to 70 cycles or more, with from about 30 to 50 being standard. In some embodiments, this is sufficient for the purposes of the experiment; for example, for the detection of certain mutations, including single nucleotide polymorphisms (SNPs), the experiment is designed such that the initial round of sequencing gives the desired information. In other embodiments, it is desirable to sequence longer targets, for example in excess of hundreds of bases. In this application, additional rounds of sequencing can be done.

For example, after a certain number of cycles, it is possible to stop the reaction, remove the newly synthesized strand using either a thermal step or a chemical wash, and start the reaction over, using for example the sequence information that was previously generated to make a new extension primer that will hybridize to the first target portion of the target sequence. That is, the sequence information generated in the first round is transferred to an oligonucleotide synthesizer, and a second extension primer is made for a second round of sequencing. In this way, multiple overlapping rounds of sequencing are used to generate long sequences from template nucleic acid strands. Alternatively, when a single target sequence contains a number of mutational "hot spots", primers can be generated using the known sequences in between these hot spots.

Additionally, the methods of the invention find use in the decoding of random microsphere arrays. That is, nucleic acids can be used as bead identifiers. By using sequencing-by-synthesis to read out the sequence of the nucleic acids, the beads can be decoded in a highly parallel fashion.

In addition, the methods find use in simultaneous analysis of multiple target sequence positions on a single array. For example, four separate sequence analysis reactions are performed. In the first reaction, positions containing a particular nucleotide ("A", for example) in the target sequence are analyzed. In three other reactions, C, G, and T are analyzed. An advantage of analyzing one base per reaction is that the baseline or background is flattened for the three bases excluded from the reaction. Therefore, the signal is more easily detected and the sensitivity of the assay is increased. Alternatively, each of the four sequencing reactions (A, G, C and T) can be performed simultaneously with a nested set of primers providing a significant advantage in that primer synthesis can be made more efficient

In another preferred embodiment each probe is represented by multiple beads in the array (see US 2001-0029049). As a result, each experiment can be replicated many times in parallel. As outlined below, averaging the signal from each respective probe in an experiment also allows for improved signal to noise and increases the sensitivity of detecting subtle perturbations in signal intensity patterns. The use of redundancy and comparing the patterns obtained from two different samples (e.g. a reference and an unknown), results in highly paralleled and comparative sequence analysis that can be performed on complex nucleic acid samples.

As outlined herein, the pyrosequencing systems may be configured in a variety of ways; for example, the target sequence may be attached to the array (e.g. the beads) in a variety of ways, including the direct attachment of the target sequence to the array; the use of a capture probe with a separate extension probe; the use of a capture extender probe, a capture probe and a separate extension probe; the use of adapter sequences in the target sequence with capture and extension probes; and the use of a capture probe that also serves as the extension probe.

In addition, as will be appreciated by those in the art, the target sequence may comprise any number of sets of different first and second target domains; that is, depending on the number of target positions that may be elucidated at a time, there may be several "rounds" of sequencing occurring, each time using a different target domain.

One additional benefit of pyrosequencing for genotyping purposes is that since the reaction does not rely on the incorporation of labels into a growing chain, the unreacted extension primers need not be removed.

Thus, pyrosequencing kits and reactions require, in no particularly order, arrays comprising capture probes, sequencing primers, an extension enzyme, and secondary enzymes and reactants for the detection of PPi, generally comprising enzymes to convert PPi into ATP (or other NTPs), and enzymes and reactants to detect ATP.

<u>Attachment of enzymes to arrays</u>

In the present invention, with regard to the secondary enzymes (i.e. enzymes other than extension enzymes) used in the reaction, the enzyme(s) are attached, preferably through the use of flexible linkers, to the sites on the array, i.e. the beads. For example, when pyrosequencing is done, one embodiment utilizes detection based on the generation of a chemiluminescent signal in the"zone" around the bead. By attaching the secondary enzymes required to generate the signal, an increased concentration of the required enzymes is obtained in the immediate vicinity of the reaction, thus allowing for the use of less enzyme and faster reaction rates for detection. Thus, embodiments utilize the attachment, preferably covalently (although as will be appreciated by those in the art, other attachment mechanisms may be used), of the non-extension secondary enzymes used to generate the signal. In some embodiments, the extension enzyme (e.g. the polymerase) may be attached as well, although this is not generally preferred.

The attachment of enzymes to array sites, particularly beads, is outlined in U.S.S.N. 09/287,573, hereby incorporated by reference, and will be appreciated by those in the art. In general, the use of flexible linkers are preferred, as this allows the enzymes to interact with the substrates. However, for some types of attachment, linkers are not needed. Attachment proceeds on the basis of the composition of the array site (i.e. either the substrate or the bead, depending on which array system is used) and the composition of the enzyme. In a preferred embodiment, depending on the composition of the array site (e.g. the bead), it will contain chemical functional groups for subsequent attachment of other moieties. For example, beads comprising a variety of chemical functional groups such as amines are commercially available. Preferred functional groups for attachment are amino groups, carboxy groups, oxo groups and thiol groups, with amino groups being particularly preferred. Using these functional groups, the enzymes can be attached using functional groups on the enzymes. For example, enzymes containing amino groups can be attached to particles comprising amino groups, for example using linkers as are known in the art; for example, homo-or hetero-bifunctional linkers as are well known (see 1994 Pierce Chemical Company catalog, technical section on cross-linkers, pages 155-200).

<u>Reversible Chain Termination Methods</u>

In a preferred embodiment, the sequencing-by-synthesis method utilized is reversible chain termination, In this embodiment, the rate of addition of dNTPs is controlled by using nucleotide analogs that contain a removable protecting group at the 3' position of the dNTP. The presence ot the protecting group prevents further addition of dNTPs at the 3' end, thus allowing time for detection of the nucleotide added (for example, utilizing a labeled dNTP). After acquisition of the identity of the dNTP added, the protecting group is removed and the cycle repeated. In this way, dNTPs are added one at a time to the sequencing primer to allow elucidation of the nucleotides at the target positions. See U.S. Patent Nos. 5,902,723; 5,547,839; Metzker et al., Nucl. Add Res. 22 (20):4259 (1994); Canard et sal., Gene 148(1):1-6 (1994); Dyatkina et al., Nucleic Acid Symp. Ser. 18: 117-120 (1987) .

Accordingly, the present disclosure provides methods and compositions for reversible chain termination sequencing-by-synthesis. Similar to pyrosequencing, the reaction requires the hybridization of a substantially complementary sequencing primer to a first target domain of a target sequence to form an assay complex.

The reaction is initiated by introducing the assay complex comprising the target sequence (i.e. the array) to a solution comprising a first nucleotide analog. By "nucleotide analog" in this context herein is meant a deoxynucleoside-triphosphate (also called deoxynucleotides or dNTPs, i.e. dATP, dTTP, dCTP and dGTP), that is further derivatized to be reversibly chain terminating As will be appreciated by those in the art, any number of nucleotide analogs may be used, as long as a polymerase enzyme will still incorporate the nucleotide at the sequence position.

Time-resolved sequencing

Optionally, time-resolved sequencing is done. This relies on controlling the reaction rate of the extension reaction and/or using a fast imaging system. Basically, the method involves a simple extension reaction that is either "slowed down", or imaged using a fast system, or both. What is important is that the rate of polymerization (extension) is significantly slower than the rate of image capture.

To allow for real time sequencing, parameters such as the speed of the detector (millisecond speed is preferred), and rate of polymerization will be controlled such that the rate of polymerization is significantly slower than the rate of image capture. Polymerization rates on the order of kilobases per minute (e.g. -10 milliseconds/ nucleotide), which can be adjusted, should allow a sufficiently wide window to find conditions where the sequential addition of two nucleotides can be resolved. The DNA polymerization reaction, which has been studied intensively, can easily be reconstituted in vitro and controlled by varying a number of parameters including reaction temperature and the concentration of nucleotide triphosphates.

In addition, the polymerase can be applied to the primer template complex prior to initiating the reaction. This serves to synchronize the reaction. Numerous polymerases are available, Some examples include, but are not limited to polymerases with 3' to 5' exonuclease activity, other nuclease activities, polymerases with different processivity, affinities for modified and unmodified nucleotide triphosphates, temperature optima, stability, and the like.

Thus, I the reaction proceeds as outlined above. The target sequence, comprising a first domain that will hybridize to a sequencing primer and a second domain comprising a plurality of target positions, is attached to an array as outlined below. The sequencing primers are added, along with an extension enzyme, as outlined herein, and dNTPs are added. Again, as outlined above, either four differently labeled dNTPs may be used simultaneously or, four different sequential reactions with a single label are done. In general, the dNTPs comprise either a primary or a secondary label, as outlined above.

Optionally, the extension enzyme is one that is relatively "slow". This may be accomplished in several ways. In one possibility, polymerase variants are used that have a lower polymerization rate than wild-type enzymes. Alternatively, the reaction rate may be controlled by varying the temperature and the concentration of dNTPs.

Optionally, a fast (millisecond) high-sensitivity Imaging system is used.

Optionally, DNA polymerization (extension) is monitored using light scattering, as is outlined in Johnson et al., Anal. Biochem. 136(1):192 (1984),

ATTACHMENT OF TARGET SEQUENCES TO ARRAYS

As is generally described herein, there are a variety of methods that can be used to attach target sequences to the solid supports of the invention, particularly to the microspheres that are distributed on a surface of a substrate. Most of these methods generally rely on capture probes attached to the array. However, the attachment may be direct or indirect. Direct attachment includes those situations wherein an endogeneous portion of the target sequence hybridizes to the capture probe, or where the target sequence has been manipulated to contain exogeneous adapter sequences that are added to the target sequence, for example during an amplification reaction, indirect attachment utilizes one or more secondary probes, termed a "capture extender probe" as outlined herein.

In a preferred embodiment, direct attachment is done, as is generally depicted in Figure 1A. In this embodiment, the target sequence comprises a first target domain that hybridizes to all or part of the capture probe.

In a preferred embodiment, direct attachment is accomplished through the use of adapters. The adapter is a chemical moiety that allows one to address the products of a reaction to a solid surface. The type of reaction includes the amplification, genotyping and sequencing reactions disclosed herein. The adapter chemical moiety is independent of the reaction. Because the adapters are Independent of the reaction, sets of adapters can be reused to create a "universal" array that can detect a variety of products from a reaction by attaching the set of adapters that address to specific locations within the array to different reactants.

Typically, the adapter and the capture probe on an array are binding partners, as defined herein. Although the use of other binding partners are possible, preferred embodiments utilize nucleic add adapters that are non-complementary to any reactants or target sequences, but are substantially complementary to all or part of the capture probe on the array.

Thus, an "adapter sequence" is a nucleic acid that is generally not native to the target sequence, I.e. is exogeneous, but is added or attached to the target sequence. It should be noted that in this context, the "target sequence" can include the primary sample target sequence, or can be a derivative target such as a reactant or product of the reactions outlined herein; thus for example, the target sequence can be a PCR product, a first ligation probe or a ligated probe in an OLA reaction, etc.

As will be appreciated by those in the art, the attachment, or joining, of the adapter sequence to the target sequence can be done in a variety of ways. In a preferred embodiment, the adapter sequences are added to the primers of the reaction (extension primers, amplification primers, readout probes, sequencing primers, Rolling Circle primers, etc.) during the chemical synthesis of the primers. The adapter then gets added to the reaction product during the reaction; for example, the primer gets extended using a polymerase to form the new target sequence that now contains an adapter sequence. Alternatively, the adapter sequences can be added enzymatically. Furthermore, the adapter can be attached to the target after synthesis; this post-synthesis attachment could be either covalent or non-covalent.

In this embodiment, one or more of the amplification primers comprises a first portion comprising the adapter sequence and a second portion comprising the primer sequence. Extending the amplification primer as is well known in the art results in target sequences that comprise the adapter sequences. The adapter sequences are designed to be substantially complementary to capture probes.

In addition, as will be appreciated by those in the art, the adapter can be attached either on the 3' or 5' ends, or in an internal position. For example, the adapter may be the detection sequence of an invasive cleavage probe. In the case of Rolling Circle probes, the adapter can be contained within the section between the probe ends. Adapters can also be attached to aptamers. Aptamers are nucleic acids that can be made to bind to virtually any target analyte; see Bock et ai., Nature 355: 564 (1992): Femulok et al., Current Op. Chem. Biol. 2: 230 (1998); and U. S. Patents 5,270,163, 5,475,096, 5,567,588, 5,595,877, 5,637,459, 5,683,867, 5,705,337, and related patents. In addition, as outlined below, the adapter can be attached to non-nucleic acid target analytes as well.

In one embodiment, a set of probes is hybridized to a target sequence; each probe is complementary to a different region of a single target but each contains the same adapter. Using a poly-T bead, the mRNA target is pulled out of the sample with the probes attached. Dehybridizing the probes attached to the target sequence and rehybridizing them to an array containing the capture probes complementary to the adapter sequences results in binding to the array. All adapters that have bound to the same target mRNA will bind to the same location on the array.

In a preferred embodiment, indirect attachment of the target sequence to the array is done, through the use of capture extender probes. "Capture extender" probes are generally depicted in Figure 1C, and other figures, and have a first portion that will hybridize to all or part of the capture probe, and a second portion that will hybridize to a first portion of the target sequence. Two capture extender probes may also be used. This has generally been done to stabilize assay complexes for example when the target sequence is large, or when large amplifier probes (particularly branched or dendrimer amplifier probes) are used.

When only capture probes are utilized, it is necessary to have unique capture probes for each target sequence; that is, the surface must be customized to contain unique capture probes; e.g. each bead comprises a different capture probe. In general, only a single type of capture probe should be bound to a bead; however, different beads should contain different capture probes so that different target sequences bind to different beads.

Alternatively, the use of adapter sequences and capture extender probes allow the creation of more "universal" surfaces. In a preferred embodiment, an array of different and usually artificial capture probes are made; that is, the capture probes do not have complementarity to known target sequences. The adapter sequences can then be added to any target sequences, or soluble capture extender probes are made; this allows the manufacture of only one kind of array, with the user able to customize the array through the use of adapter sequences or capture extender probes. This then allows the generation of customized soluble probes, which as Will be appreciated by those in the art is generally simpler and less costly.

As will be appreciated by those in the art, the length of the adapter sequences will vary, depending on the desired "strength" of binding and the number of different adapters desired. In a preferred embodiment, adapter sequences range from about 6 to about 500 basepairs in length, with from about 8 to about 100 being preferred, and from about 10 to about 25 being particularly preferred.

In one embodiment, microsphere arrays containing a single type of capture probe are made; in this embodiment, the capture extender probes are added to the beads prior to loading on the array. The capture extender probes may be additionally fixed or crosslinked, as necessary.

In a preferred embodiment, as outlined in Figure 1B, the capture probe comprises the sequencing primer; that is, after hybridization to the target sequence, it is the capture probe itself that is extended during the synthesis reaction.

In one embodiment, capture probes are not used, and the target sequences are attached directly to the sites on the array. For example, libraries of clonal nucleic acids, including DNA and RNA, are used. In this embodiment, individual nucleic adds are prepared, generally using conventional methods (including, but not limited to, propagation in plasmid or phage vectors, amplification techniques including PCR, etc.). The nucleic acids are preferably arrayed in some format, such as a microtiter plate format, and either spotted or beads are added for attachment of the libraries.

Attachment of the clonal libraries (or any of the nucleic acids outlined herein) may be done in a variety of ways, as will be appreciated by those in the art, including, but not limited to, chemical or affinity capture (for example, including the incorporation of derivatized nucleotides such as AminoLink or biotinylated nucleotides that can then be used to attach the nucleic acid to a surface, as well as affinity capture by hybridization), cross-linking, and electrostatic attachment, etc.

In a preferred embodiment, affinity capture is used to attach the clonal nucleic acids to the surface. For example, cloned nucleic acids can be derivatized, for example with one member of a binding pair, and the beads derivatized with the other member of a binding pair. Suitable binding pairs are as described herein for secondary labels and IBL/DBL pairs. For example, the cloned nucleic acids may be biotinylated (for example using enzymatic incorporate of biotinylated nucleotides, for by photoactivated cross-linking of biotin). Biotinylated nucleic acids can then be captured on streptavidin-coated beads, as is known in the art. Similarly, other hapten-receptor combinations can be used, such as digoxigenin and anti-digoxigenin antibodies. Alternatively, chemical groups can be added in the form of derivatized nucleotides, that can them be used to add the nucleic add to the surface.

Preferred attachments are covalent, although even relatively weak interactions (i.e. non-covalent) can be sufficient to attach a nucleic acid to a surface, if there are multiple sites of attachment per each nucleic acid. Thus, for example, electrostatic interactions can be used for attachment, for example by having beads carrying the opposite charge to the bioactive agent.

Similarly, affinity capture utilizing hybridization can be used to attach cloned nucleic acids to beads. For example, as is known in the art, polyA+RNA is routinely captured by hybridization to oligo-dT beads; this may include oligo-dT capture followed by a cross-linking step, such as psoralen crosslinking). If the nucleic acids of interest do not contain a polyA tract, one can be attached by polymerization with terminal transferase, or via ligation of an oligoA linker, as is known in the art.

Alternatively, chemical crosslinking may be done, for example by photoactivated crosslinking of thymidine to reactive groups, as is known in the art.

In general, special methods are required to decode clonal arrays, as is more fully outlined below.

## ASSAY AND ARRAYS

All of the above compositions and methods are directed to the detection and/or quantification of the products of nucleic acid reactions. The detection systems of the present disclosure are based on the incorporation (or in some cases, of the deletion) of a detectable label into an assay complex on an array.

Accordingly, the present disclosure provides methods and compositions useful in the detection of nucleic acids. As will be appreciated by those in the art, the compositions of the invention can take on a wide variety of configurations, as is generally outlined in the Figures. As is more fully outlined below, preferred systems of the disclosure work as follows. A target nucleic acid sequence is attached (via hybridization) to an array site. This attachment can be either directly to a capture probe on the surface, through the use of adapters, or indirectly, using capture extender probes as outlined herein. In some embodiments, the target sequence itself comprises the labels. Alternatively, a label probe is then added, forming an assay complex. The attachment of the label probe may be direct (i.e. hybridization to a portion of the target sequence), or indirect (i.e. hybridization to an amplifier probe that hybridizes to the target sequence), with all the required nucleic acids forming an assay complex.

Accordingly, the present disclosure provides array compositions comprising at least a first substrate with a surface comprising individual sites. By "array" or "biochip" herein is meant a plurality of nucleic acids In an array format; the size of the array will depend on the composition and end use of the array. Nucleic acids arrays are known in the art, and can be classified in a number of ways; both ordered arrays (e.g. the ability to resolve chemistries at discrete sites), and random arrays are included. Ordered arrays include, but are not limited to, those made using photolithography techniques (Affymetrix Genechip™), spotting techniques (Synteni end others), printing techniques (Hewlett Packard and Rosetta), three dimensional "gel pad" arrays, etc. The present invention utilizes microspheres on fiber optic bundles, as are outlined in WO99/18434, WO99/67641 AND WO98/40726; WO98/50782; and US2001-0029049; US6327410, US6429027, US2002-0051971 and US654432.

Arrays containing from about 2 different bioactive agents (e.g. different beads, when beads are used) to many millions can be made, with very large arrays being possible. Generally, the array will comprise from two to as many as a billion or more, depending on the size of the beads and the substrate, as well as the end use of the array, thus very high density, high density, moderate density, low density and very low density arrays may be made. Preferred ranges for very high density arrays are from about 10,000,000 to about 2,000,000,000, with from about 100,000,000 to about 1,000,000,000 being preferred (all numbers being in square cm). High density arrays range about 100,000 to about 10,000,000, with from about 1,000,000 to about 5,000,000 being particularly preferred. Moderate density arrays range from about 10,000 to about 100,000 being particularly preferred, and from about 20,000 to about 50,000 being especially preferred. Low density arrays are generally less than 10,000, with from about 1,000 to about 5,000 being preferred. Very low density arrays are less than 1,000, with from about 10 to about 1000 being preferred, and from about 100 to about 500 being particularly preferred. The compositions may not be in array format; that is, compositions comprising a single bioactive agent may be made as well. In addition, in some arrays, multiple substrates may be used, either of different or identical compositions. Thus for example, large arrays may comprise a plurality of smaller substrates.

In addition, one advantage of the present compositions is that particularly through the use of fiber optic technology, extremely high density arrays can be made. Thus for example, because beads of 200 μm or less (with beads of 200 nm possible) can be used, and very small fibers are known, it is possible to have as many as 40,000 or more (in some instances, 1 million) different elements (e.g. fibers and beads) in a 1 $mm^2$ fiber optic bundle, with densities of greater than 25,000,000 individual beads and fibers (again, in some instances as many as 50-100 million) per 0.5 $cm^2$ obtainable (4 million per square cm for 5 μ center-to-center and 100 million per square cm for 1 μ center-to-center).

By "substrate" or "solid support" or other grammatical equivalents herein is meant any material that can be modified to contain discrete individual sites appropriate for the attachment or association of beads and is amenable to at least one detection method. As will be appreciated by those in the art, the number of possible substrates is very large. Possible substrates include, but are not limited to, glass and modified or functionalized glass, plastics (including acrylics, polystyrene and copolymers of styrene and other materials, polypropylene, polyethylene, polybutylene, polyurethanes, Teflon, etc.), polysaccharides, nylon or nitrocellulose, resins, silica or silica-based materials including silicon and modified silicon, carbon, metals, inorganic glasses, plastics, optical fiber bundles, and a variety of other polymers. In general, the substrates allow optical detection and do not themselves appreciably fluoresce.

Generally the substrate is flat (planar), although as will be appreciated by those in the art, other configurations of substrates may be used as well; for example, three dimensional configurations can be used, for example by embedding the beads in a porous block of plastic that allows sample access to the beads and using a confocal microscope for detection. Similarly, the beads may be placed on the inside surface of a tube, for flow-through sample analysis to minimize sample volume. Preferred substrates include optical fiber bundles as discussed below, and flat planar substrates such as glass, polystyrene and other plastics and acrylics.

In a preferred embodiment, the substrate is an optical fiber bundle or array, as is generally described in US7115884 and US6210910, WO98/40726 and WO98/50782.

Preferred embodiments utilize preformed unitary fiber optic arrays. By "preformed unitary fiber optic array" herein is meant an array of discrete individual fiber optic strands that are co-axially disposed and joined along their lengths. The fiber strands are generally individually clad. However, one thing that distinguished a preformed unitary array from other fiber optic formats is that the fibers are not individually physically manipulatable; that is, one strand generally cannot be physically separated at any point along its length from another fiber strand.

Generally, the array of array compositions of the invention can be configured in several ways; see for example US6858394. In a preferred embodiment, as is more fully outlined below, a "one component" system is used. That is, a first substrate comprising a plurality of assay locations (sometimes also referred to herein as "assay wells"), such as a microtiter plate, is configured such that each assay location contains an individual array. That is, the assay location and the array location are the same. For example, the plastic material of the microtiter plate can be formed to contain a plurality of "bead wells" in the bottom of each of the assay wells. Beads containing the capture probes of the invention can then be loaded into the bead wells in each assay location as is more fully described below.

Alternatively, a "two component" system can be used. In this embodiment, the individual arrays are formed on a second substrate, which then can be fitted or "dipped" into the first microtiter plate substrate. A preferred embodiment utilizes fiber optic bundles as the individual arrays, generally with "bead wells" etched into one surface of each Individual fiber, such that the beads containing the capture probes are loaded onto the end of the fiber optic bundle. The composite array thus comprises a number of individual arrays that are configured to fit within the wells of a microtiter plate. By "composite array" or "combination array" or grammatical equivalents herein is meant a plurality of individual arrays, as outlined above. Generally the number of individual arrays is set by the size of the microtiter plate used; thus, 96 well, 384 well and 1536 well microtiter plates utilize composite arrays comprising 96, 384 and 1536 individual arrays, although as will be appreciated by those in the art, not each microtiter well need contain an individual array. It should be noted that the composite arrays can comprise individual arrays that are identical, similar or different. That is, in some embodiments, it may be desirable to do the same 2,000 assays on 96 different samples; alternatively, doing 192,000 experiments on the same sample (i.e. the same sample in each of the 96 wells) may be desirable.

Alternatively, each row or column of the composite array could be the same, for redundancy/quality control. As will be appreciated by those in the art, there are a variety of ways to configure the system. In addition, the random nature of the arrays may mean that the same population of beads may be added to two different surfaces, resulting in substantially similar but perhaps not identical arrays.

At least one surface of the substrate is modified to contain discrete, individual sites for later association of microspheres. These sites may comprise physically altered sites, i.e. physical configurations such as wells or small depressions in the substrate that can retain the beads, such that a microsphere can rest in the well, or the use of other forces (magnetic or compressive), or chemically altered or active sites, such as chemically functionalized sites, electrostatically altered sites, hydrophobically/ hydrophilically functionalized sites, spots of adhesive, etc.

The sites may be a pattern, i.e. a regular design or configuration, or randomly distributed. A preferred embodiment utilizes a regular pattern of sites such that the sites may be addressed in the X-Y coordinate plane. "Pattern" in this sense includes a repeating unit cell, preferably one that allows a high density of beads on the substrate. However, it should be noted that these sites may not be discrete sites. That is, it is possible to use a uniform surface of adhesive or chemical functionalities, for example, that allows the attachment of beads at any position. That is, the surface of the substrate is modified to allow attachment of the microspheres at individual sites, whether or not those sites are contiguous or non-contiguous with other sites. Thus, the surface of the substrate may be modified such that discrete sites are formed that can only have a single associated bead, or alternatively, the surface of the substrate is modified and beads may go down anywhere, but they end up at discrete sites.

In a preferred embodiment, the surface of the substrate is modified to contain wells, i.e. depressions in the surface of the substrate. This may be done as is generally known in the art using a variety of techniques, including, but not limited to, photolithography, stamping techniques, molding techniques and microstching techniques. As will be appreciated by those in the art, the technique used will depend on the composition and shape of the substrate.

In a preferred embodiment, physical alterations are made in a surface of the substrate to produce the sites. The substrate is a fiber optic bundle and the surface of the substrate is a terminal end of the fiber bundle, as is generally described in US6023540 and US6327410.

In this embodiment, wells are made in a terminal or distal end of a fiber optic bundle comprising individual fibers. In this embodiment, the cores of the individual fibers are etched, with respect to the cladding, such that small wells or depressions are formed at one end of the fibers. The required depth of the wells will depend on the size of the beads to be added to the wells.

Generally in this embodiment, the microspheres are non-covalently associated In the wells, although the wells may additionally be chemically functionalized as is generally described below, cross-linking agents may be used, or a physical barrier may be used, i.e. a film or membrane over the beads.

In a preferred embodiment, the surface of the substrate is modified to contain chemically modified sites, that can be used to attach, either covalently or non-covelently, the microspheres of the invention to the discrete sites or locations on the substrate. "Chemically modified sites" in this context includes, but is not limited to, the addition of a pattern of chemical functional groups including amino groups, carboxy groups, oxo groups and thiol groups, that can be used to covalently attach microspheres, which generally also contain corresponding reactive functional groups; the addition of a pattern of adhesive that can be used to bind the microspheres (either by prior chemical functionalization for the addition of the adhesive or direct addition of the adhesive); the addition of a pattern of charged groups (similar to the chemical functionalities) for the electrostatic attachment of the microspheres, i.e. when the microspheres comprise charged groups opposite to the sites; the addition of a pattern of chemical functional groups that renders the sites differentially hydrophobic or hydrophillo, such that the addition of similarly hydrophobic or hydrophilic microspheres under suitable experimental conditions will result in association of the microspheres to the sites on the basis of hydroaffinity. For example, the use of hydrophobic sites with hydrophobic beads, in an aqueous system, drives the association of the beads preferentially onto the sites. As outlined above, "pattern" in this sense includes the use of a uniform treatment of the surface to allow attachment of the beads at discrete sites, as well as treatment of the surface resulting in discrete sites. As Will be appreciated by those in the art, this may be accomplished In a variety of ways.

In some embodiments, the beads are not associated with a substrate. That is, the beads are In solution or are not distributed on a patterned substrate.

In a preferred embodiment, the compositions of the invention further comprise a population of microspheres. By "population" herein is meant a plurality of beads as outlined above for arrays. Within the population are separate subpopulations, which can be a single microsphere or multiple identical microspheres. That is, in some embodiments, as is more fully outlined below, the array may contain only a single bead for each capture probe; preferred embodiments utilize a plurality of beads of each type.

By "microspheres" or "beads" or "particles" or grammatical equivalents herein is meant small discrete particles. The composition of the beads will vary, depending on the class of capture probe and the method of synthesis. Suitable bead compositions include those used in peptide, nucleic acid and organic moiety synthesis, including, but not limited to, plastics, ceramics, glass, polystyrene, methylstyrene, acrylic polymers, paramagnetic materials, thoria sol, carbon graphite, titanium dioxide, latex or cross-linked dextrans such as Sepharose, cellulose, nylon, cross-linked micelles and Teflon may all be used. *"Microsphere Detection Guide"* from Bangs Laboratories, Fishers IN is a helpful guide.

The beads need not be spherical; irregular particles may be used. In addition, the beads may be porous, thus Increasing the surface area of the bead available for either capture probe attachment or tag attachment. The bead sizes range from nanometers, i.e. 100 nm, to millimeters, i.e. 1 mm, with beads from about 0.2 micron to about 200 microns being preferred, and from about 0.5 to about 5 micron being particularly preferred, although in some embodiments smaller beads may be used.

It should be noted that a key component of the invention is the use of a substrate/bead pairing that allows the association or attachment of the beads at discrete sites on the surface of the substrate, such that the beads do not move during the course of the assay.

Each microsphere comprises a capture probe, although as will be appreciated by those in the art, there may be some microspheres which do not contain a capture probe, depending on the synthetic methods.

Attachment of the nucleic acids may be done in a variety of ways, as will be appreciated by those in the art, including, but not limited to, chemical or affinity capture (for example, including the incorporation of derivatized nucleotides such as AminoLink or biotinylated nucleotides that can then be used to attach the nucleic acid to a surface, as well as affinity capture by hybridization), cross-linking, and electrostatic attachment, etc. In a preferred embodiment, affinity capture is used to attach the nucleic acids to the beads. For example, nucleic acids can be derivatized, for example with one member of a binding pair, and the beads derivatized with the other member of a binding pair. Suitable binding pairs are as described herein for IBL/DBL pairs. For example, the nucleic acids may be biotinylated (for example using enzymatic incorporate of biotinylated nucleotides, for by photoactivated cross-linking of biotin). Biotinylated nucleic acids can then be captured on streptavidin-coated beads, as is known in the art. Similarly, other hapten-receptor combinations can be used, such as digoxigenin and anti-digoxigenin antibodies. Alternatively, chemical groups can be added in the form of derivatized nucleotides, that can them be used to add the nucleic acid to the surface.

Preferred attachments are covalent, although even relatively weak interactions (i.e. non-covalent) can be sufficient to attach a nucleic acid to a surface, if there are multiple sites of attachment per each nucleic acid. Thus, for example, electrostatic interactions can be used for attachment, for example by having beads carrying the opposite charge to the bioactive agent.

Similarly, affinity capture utilizing hybridization can be used to attach nucleic acids to beads. For example, as-is known in the art, polyA+RNA is routinely captured by hybridization to oligo-dT beads; this may include oligo-dT capture followed by a cross-linking step, such as psoralen crosslinking). If the nucleic acids of interest do not contain a polyA tract, one can be attached by polymerization with terminal transferase, or via ligation of an oligoA linker, as is known in the art.

Alternatively, chemical crosslinking may be done, for example by photoactivated crosslinking of thymidine to reactive groups, as is known in the art.

In general, probes of the present disclosure are designed to be complementary to a target sequence (either the target sequence of the sample or to other probe sequences, as is described herein), such that hybridization of the target and the probes of the present invention occurs. This complementarily need not be perfect; there may be any number of base pair mismatches that will interfere with hybridization between the target sequence and the single stranded nucleic acids of the present invention. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under the selected reaction conditions.

In a preferred embodiment, each bead comprises a single type of capture probe, although a plurality of individual capture probes are preferably attached to each bead. Similarly, preferred embodiments utilize more than one microsphere containing a unique capture probe; that is, there is redundancy built into the system by the use of subpopulations of microspheres, each microsphere in the subpopulation containing the same capture probe.

As will be appreciated by those in the art, the capture probes may either be synthesized directly on the beads, or they may be made and then attached after synthesis. In a preferred embodiment, linkers are used to attach the capture probes to the beads, to allow both good attachment, sufficient flexibility to allow good interaction with the target molecule, and to avoid undesirable binding reactions.

In a preferred embodiment, the capture probes are synthesized directly on the beads. As is known in the art, many classes of chemical compounds are currently synthesized on solid supports, such as peptides, organic moieties, and nucleic acids. It is a relatively straightforward matter to adjust the current synthetic techniques to use beads.

In a preferred embodiment, the capture probes are synthesized first, and then covalently attached to the beads. As will be appreciated by those in the art, this will be done depending on the composition of the capture probes and the beads. The functionalization of solid support surfaces such as certain polymers with chemically reactive groups such as thiols, amines, carboxyls, etc. Is generally known in the art. Accordingly, "blank" microspheres may be used that have surface chemistries that facilitate the attachment of the desired functionality by the user. Some examples of these surface chemistries for blank microspheres include, but are not limited to, amino groups including aliphatic and aromatic amines, carboxylic acids, aldehydes, amides, chloromethyl groups, hydrazide, hydroxyl groups, sulfonates and sulfates.

When random arrays are used, an encoding/decoding system must be used. For exampe, when microsphere arrays are used, the beads are generally put onto the substrate randomly; as such there are several ways to correlate the functionality on the bead with its location, including the incorporation of unique optical signatures, generally fluorescent dyes, that could be used to identify the chemical functionality on any particular bead. This allows the synthesis of the candidate agents (i.e. compounds such as nucleic acids and antibodies) to be divorced from their placement on an array, i.e. the candidate agents may be synthesized on the beads, and then the beads are randomly distributed on a patterned surface. Since the beads are first coded with an optical signature, this means that the array can later be "decoded", i.e. after the array is made, a correlation of the location of an individual site on the array with the bead or candidate agent at that particular site can be made. This means that the beads may be randomly distributed on the array, a fast and inexpensive process as compared to either the in situ synthesis or spotting techniques of the prior art.

However, the drawback to these methods is that for a large array, the system requires a large number of different optical signatures, which may be difficult or time-consuming to utilize. Accordingly, the present invention provides several improvements over these methods, generally directed to methods of coding and decoding the arrays. That is, as will be appreciated by those in the art, the placement of the capture probes is generally random, and thus a coding/decoding system is required to identify the probe at each location in the array. This may be done in a variety of ways, as is more fully outlined below, and generally includes: a) the use a decoding binding ligand (DBL), generally directly labeled, that binds to either the capture probe or to identifier binding ligands (IBLs) attached to the beads; b) positional decoding, for example by either targeting the placement of beads (for example by using photoactivatible or photocleavable moleties to allow the selective addition of beads to particular locations), or by using either sub-bundles or selective loading of the sites, as are more fully outlined below; c) selective decoding, wherein only those beads that bind to a target are decoded; or d) combinations of any of these. In some, cases, as is more fully outlined below, this decoding may occur for all the beads, or only for those that bind a particular target sequence. Similarly, this may occur either prior to or after addition of a target sequence. In addition, as outlined herein, the target sequences detected may be either a primary target sequence (e.g. a patient sample), or a reaction product from one of the methods described herein (e.g. an extended SBE probe, a ligated probe, a cleaved signal probe, etc.).

Once the identity (i.e. the actual agent) and location of each microsphere in the array has been fixed, the array is exposed to samples containing the target sequences, although as outlined below, this can be done prior to or during the analysis as well. The target sequences can hybridize (either directly or indirectly) to the capture probes as is more fully outlined below, and results in a change in the optical signal of a particular bead.

In the present disclosure, "decoding" does not rely on the use of optical signatures, but rather on the use of decoding binding ligands that are added during a decoding step. The decoding binding ligands will bind either to a distinct identifier binding ligand partner that is placed on the beads, or to the capture probe itself. The decoding binding ligands are either directly or indirectly labeled, and thus decoding occurs by detecting the presence of the label. By using pools of decoding binding ligands in a sequential fashion, it is possible to greatly minimize the number of required decoding steps.

In some embodiments, the microspheres may additionally comprise identifier binding ligands for use in certain decoding systems. By "identifier binding ligands" or "IBLs" herein is meant a compound that will specifically bind a corresponding decoder binding ligand (DBL) to facilitate the elucidation of the identity of the capture probe attached to the bead. That is, the IBL and the corresponding DBL form a binding partner pair. By "specifically bind" herein is meant that the IBL binds its DBL with specificity sufficient to differentiate between the corresponding DBL and other DBLs (that is, DBLs for other IBLs), or other components or contaminants of the system. The binding should be sufficient to remain bound under the conditions of the decoding step, including wash steps to remove non-specific binding. In some embodiments, for example when the IBLs and corresponding DBLs are proteins or nucleic acids, the dissociation constants of the IBL to its DBL will be less than about $40^{-4}$-$10^{-6}$ $M^{-1}$, with less than about $10^{-5}$ to $10^{-9}$ $M^{-1}$ being preferred and less than about $10^{-7}$ -$10^{-9}$ $M^{-1}$ being particularly preferred.

IBL-DBL binding pairs are known or can be readily found using known techniques. For example, when the IBL is a protein, the DBLs include proteins (particularly including antibodies or fragments thereof (FAbs, etc.)) or small molecules, or vice versa (the IBL is an antibody and the DBL is a protein). Metal ion- metal ion ligands or chelators pairs are also useful. Antigen-antibody pairs, enzymes and substrates or inhibitors, other protein-protein Interacting pairs, receptor-ligands, complementary nucleic acids, and carbohydrates and their binding partners are also suitable binding pairs. Nucleic acid - nucleic acid binding proteins pairs are also useful. Similarly, as is generally described in U.S. Patents 5,270,163, 5,475,096, 5,567,588, 5,595,877, 5,637,459, 5,683,867,5,705,337, and related patents, hereby incorporated by reference, nucleic add "aptamers" can be developed for binding to virtually any target; such an aptamer-target pair can be used as the IBL-DBL pair. Similarly, there is a wide body of literature relating to the development of binding pairs based on combinatorial chemistry methods.

In a preferred embodiment, the IBL is a molecule whose color or luminescence properties change in the presence of a selectively-binding DBL. For example, the IBL may be a fluorescent pH indicator whose emission intensity changes with pH. Similarly, the IBL may be a fluorescent ion indicator, whose emission properties change with ion concentration.

Alternatively, the IBL is a molecule whose color or luminescence properties change in the presence of various solvents. For example, the IBL may be a fluorescent molecule such as an ethidium salt whose fluorescence intensity increases in hydrophobic environments. Similarly, the IBL may be a derivative of fluorescein whose color changes between aqueous and nonpolar solvents.

In one embodiment, the DBL may be attached to a bead, i.e. a "decoder bead", that may carry a label such as a fluorophore.

In a preferred embodiment, the IBL-DBL pair comprise substantially complementary single-stranded nucleic acids. In this embodiment, the binding ligands can be referred to as "identifier probes" and "decoder probes". Generally, the identifier and decoder probes range from about 4 basepairs in length to about 1000, with from about 6 to about 100 being preferred, and from about 8 to about 40 being particularly preferred. What is important is that the probes are long enough to be specific, i.e. to distinguish between different IBL-DBL pairs, yet short enough to allow both a) dissociation, if necessary, under suitable experimental conditions, and b) efficient hybridization.

In a preferred embodiment, as is more fully outlined below, the IBLs do not bind to DBLs. Rather, the IBLs are used as identifier moieties ("IMs") that are identified directly, for example through the use of mass spectroscopy.

Alternatively, in a preferred embodiment, the IBL and the capture probe are the same moiety; thus, for example, as outlined herein, particularly when no optical signatures are used, the capture probe can serve as both the identifier and the agent. For example, in the case of nucleic acids, the bead-bound probe (which serves as the capture probe) can also bind decoder probes, to identify the sequence of the probe on the bead. Thus, in this embodiment, the DBLs bind to the capture probes.

In a preferred embodiment, the microspheres may contain an optical signature. That is, as outlined in US6023540 and US6327410, previous work had each subpopulation of microspheres comprising a unique optical signature or optical tag that is used to identify the unique capture probe of that subpopulation of microspheres; that is, decoding utilizes optical properties of the beads such that a bead comprising the unique optical signature may be distinguished from beads at other locations with different optical signatures. Thus the previous work assigned each capture probe a unique optical signature such that any microspheres comprising that capture probe are identifiable on the basis of the signature. These optical signatures comprised dyes, usually chromophores or fluorophores, that were entrapped or attached to the beads themselves. Diversity of optical signatures utilized different fluorochromes, different ratios of mixtures of fluorochromes, and different concentrations (Intensities) of fluorochromes.

In a preferred embodiment, the present invention does not rely solely on the use of optical properties to decode the arrays. However, as will be appreciated by those in the art, it is possible in some embodiments to utilized optical signatures as an additional coding method, in conjunction with the present system. Thus, for example, as is more fully outlined below, the size of the array may be effectively increased while using a single set of decoding moieties in several ways, one of which is the use of optical signatures one some beads. Thus, for example, using one "set" of decoding molecules, the use of two populations of beads, one with an optical signature and one without, allows the effective doubling of the array size. The use of multiple optical signatures similarly increases the possible size of the array.

In a preferred embodiment, each subpopulation of beads comprises a plurality of different iBLs. By using a plurality of different IBLs to encode each capture probe, the number of possible unique codes is substantially increased. That is, by using one unique IBL per capture probe, the size of the array will be the number of unique IBLs (assuming no "reuse" occurs, as outlined below). However, by using a plurality of different IBLs per bead, n, the size of the array can be increased to $2^n$. when the presence or absence of each IBL is used as the indicator. For example, the assignment of 10 IBLs per bead generates a 10 bit binary code, where each bit can be designated as "1" (IBL is present) or "0" (IBL is absent). A 10 bit binary code has $2^{10}$ possible variants However, as is more fully discussed below, the size of the array may be further increased if another parameter is included such as concentration or intensity; thus for example, if two different concentrations of the IBL are used, then the array size increases as 3°. Thus, in this embodiment, each individual capture probe in the array is assigned a combination of IBLs, which can be added to the beads prior to the addition of the capture probe, after, or during the synthesis of the capture probe, i.e. simultaneous addition of IBLs and capture probe components.

Alternatively, the combination of different IBLs can be used to elucidate the sequence of the nucleic acid. Thus, for example, using two different IBLs (IBL1 and IBL2), the first position of a nucleic acid can be elucidatead: for example, adenosine can be represented by the presence of both IBL1 and IBL2; thymidine can be represented by the presence of IBL1 but not IBL2, cytosine can be represented by the presence of IBL2 but not IBL1, and guanosine can be represented by the absence of both. The second position of the nucleic acid can be done In a similar manner using IBL3 and IBL4; thus, the presence of IBL1, IBL2, IBL3 and IBL4 gives a sequence of AA; IBL1, IBL2, and IBL3 shows the sequence AT; IBL1, IBL3 and IBL4 gives the sequence TA, etc. The third position utilizes IBL5 and IBL6, etc. In this way, the use of 20 different identifiers can yield a unique code for every possible 10-mer.

In this way, a sort of "bar code" for each sequence can be constructed; the presence or absence of each distinct IBL will allow the identification of each capture probe.

In addition, to use of different concentrations or densities of IBLs allows a "reuse" of sorts. If, for example, the bead comprising a first agent has a 1X concentration of IBL, and a second bead comprising a second agent has a 10X concentration of IBL, using saturating concentrations of the corresponding labelled DBL allows the user to distinguish between the two beads.

Once the microspheres comprising the capture probes are generated, they are added to the substrate to form an array. It should be noted that while most of the methods described herein add the beads to the substrate prior to the assay, the order of making, using and decoding the array can vary. For example, the array can be made, decoded, and then the assay done. Alternatively, the array can be made, used in an assay, and then decoded; this may find particular use when only a few beads need be decoded. Alternatively, the beads can be added to the assay mixture, i.e. the sample containing the target sequences, prior to the addition of the beads to the substrate; after addition and assay, the array may be decoded. This is particularly preferred when the sample comprising the beads is agitated or mixed; this can increase the amount of target sequence bound to the beads per unit time, and thus (in the case of nucleic acid assays) increase the hybridization kinetics. The may find particular use in cases where the concentration of target sequence in the sample is low; generally, for low concentrations, long binding times must be used.

In general, the methods of masking the arrays and of decoding the arrays is done to maximize the number of different candidate agents that can be uniquely encoded. The compositions of the disclosure may be made In a variety of ways. In general, the arrays are made by adding a solution or slurry comprising the beads to a surface containing the sites for attachment of the beads. This may be done in a variety of buffers, including aqueous and organic solvents, and mixtures. The solvent can evaporate, and excess beads are removed.

In a preferred embodiment, when non-covalent methods are used to associate the beads with the array, a novel method of loading the beads onto the array is used. This method comprises exposing the array to a solution of particles (including microspheres and cells) and then applying energy, e.g. agitating or vibrating the mixture. This results in an array comprising more tightly associated particles, as the agitation is done with sufficient energy to cause weakly-associated beads to fall off (or out, in the case of wells). These sites are then available to bind a different bead. in this way, beads that exhibit a high affinity for the sites are selected. Arrays made in this way have two main advantages as compared to a more static loading: first of all, a higher percentage of the sites can be filled easily, and secondly, the arrays thus loaded show a substantial decrease in bead loss during assays. Thus, in a preferred embodiment, these methods are used to generate arrays that have at least about 50% of the sites filled, with at least about 75% being preferred, and at least about 90% being particularly preferred. Similarly, arrays generated in this manner preferably lose less than about 20% of the beads during an assay, with less than about 10% being preferred and less than about 5% being particularly preferred.

In this embodiment, the substrate comprising the surface with the discrete sites is immersed into a solution comprising the particles (beads, cells, etc.). The surface may comprise wells, as is described herein, or other types of sites on a patterned surface such that there is a differential affinity for the sites. This differnetial affinity results in a competitive process, such that particles that will associate more tightly are selected. Preferably, the entire surface to be "loaded" with beads is in fluid contact with the solution. This solution is generally a slurry ranging from about 10,000:1 beads:solution (vol:vol) to 1:1. Generally, the solution can comprise any number of reagents, including aqueous buffers, organic solvents, salts, other reagent components, etc. In addition, the solution preferably comprises an excess of beads; that is, there are more beads than sites on the array. Preferred embodiments utilize two-fold to billion-fold excess of beads.

The immersion can mimic the assay conditions; for example, if the array is to be "dipped" from above Into a microtiter plate comprising samples, this configuration can be repeated for the loading, thus minimizing the beads that are likely to fall out due to gravity.

Once the surface has been immersed, the substrate, the solution, or both are subjected to a competitive process, whereby the particles with lower affinity can be disassociated from the substrate and replaced by particles exhibiting a higher affinity to the site. This competitive process is done by the introduction of energy, in the form of heat, sonication, stirring or mixing, vibrating or agitating the solution or substrate, or both.

A preferred embodiment utilizes agitation or vibration. In general, the amount of manipulation of the substrate is minimized to prevent damage to the array; thus, preferred embodiments utilize the agitation of the solution rather than the array, although either will work. As will be appreciated by those in the art, this agitation can take on any number of forms, with a preferred embodiment utilizing microtiter plates comprising bead solutions being agitated using microtiter plate shakers.

The agitation proceeds for a period of time sufficient to load the array to a desired fill. Depending on the size and concentration of the beads and the size of the array, this time may range from about 1 second to days, with from about 1 minute to about 24 hours being preferred.

It should be noted that not all sites of an array may comprise a bead; that is, there may be some sites on the substrate surface which are empty. In addition, there may be some sites that contain more than one bead, although this is not preferred.

In some embodiments, for example when chemical attachment is done, it is possible to attach the beads In a non-random or ordered way. For example, using photoactivatible attachment linkers or photoactivatible adhesives or masks, selected sites on the array may be sequentially rendered suitable for attachment, such that defined populations of beads are laid down.

The arrays of the present disclosure are constructed such that information about the identity of the capture probe is built Into the array, such that the random deposition of the beads in the fiber wells can be "decoded" to allow identification of the capture probe at all positions. This may be done In a variety of ways, and either before, during or after the use of the array to detect target molecules.

Thus, after the array is made, it is "decoded" in order to identify the location of one or more of the capture probes, i.e. each subpopulation of beads, on the substrate surface.

In the claimed methods, pyrosequencing techniques are used to decode the array,

In a preferred embodiment, a selective decoding system is used. In this case, only those microspheres exhibiting a change in the optical signal as a result of the binding of a target sequence are decoded. This is commonly done when the number of "hits", i.e. the number of sites to decode, is generally low. That is, the array is first scanned under experimental conditions in the absence of the target sequences. The sample containing the target sequences is added, and only those locations exhibiting a change In the optical signal are decoded. For example, the beads at either the positive or negative signal locations may be either selectively tagged or released from the array (for example through the use of photocleavable linkers), and subsequently sorted or enriched in a fluorescence-activated cell sorter (FACS). That is, either all the negative beads are released, and then the positive beads are either released or analyzed In situ, or alternatively all the positives are released and analyzed. Alternatively, the labels may comprise halogenated aromatic compounds, and detection of the label is done using for example gas chromatography, chemical tags, isotopic tags mass spectral tags.

As will be appreciated by those In the art, this may also be done in systems where the array is not decoded; I.e. there need not ever be a correlation of bead composition with location. In this embodiment, the beads are loaded on the array, and the assay is run. The "positives", i.e. those beads displaying a change in the optical signal as is more fully outlined below, are then "marked" to distinguish or separate them from the "negative" beads. This can be done in several ways, preferably using fiber optic arrays. In a preferred embodiment, each bead contains a fluorescent dye. After the assay and the identification of the "positive" or "active beads", light is shown down either only the positive fibers or only the negative fibers, generally in the presence of a light-activated reagent (typically dissolved oxygen). In the former case, all the active beads are photobleached. Thus, upon non-selective release of all the beads with subsequent sorting, for example using a fluorescence activated cell sorter (FACS) machine, the non-fluorescent active beads can be sorted from the fluorescent negative beads. Alternatively, when light is shown down the negative fibers, all the negatives are non-fluorescent and the the postives are fluorescent, and sorting can proceed. The characterization of the attached capture probe may be done directly, for example using mass spectroscopy.

Alternatively, the identification may occur through the use of identifier moieties ("IMs"), which are similar to IBLs but need not necessarily bind to DBLs. That is, rather than elucidate the structure of the capture probe directly, the composition of the IMs may serve as the identified. Thus, for example, a specific combination of IMs can serve to code the bead, and be used to identify the agent on the bead upon release from the bead followed by subsequent analysis, for example using a gas chromatograph or mass spectroscope.

Alternatively, rather than having each bead contain a fluorescent dye, each bead comprises a non-fluorescent precursor to a fluorescent dye. For example, using photocleavable protecting groups, such as certain ortho-nitrobenzyl groups, on a fluorescent molecule, photoactivation of the fluorochrome can be done. After the assay, light is shown down again either the "positive" or the "negative" fibers, to distinquish these populations. The illuminated precursors are then chemically converted to a fluorescent dye. All the beads are then released from the array, with sorting, to form populations of fluorescent and non-fluorescent beads (either the positives and the negatives or vice versa).

In an alternate preferred embodiment, the sites of attachment of the beads (for example the wells) include a photopolymerizable reagent, or the photopolymerizable agent is added to the assembled array. After the test assay is run, light is shown down again either the "positive" or the "negative" fibers, to distinquish these populations. As a result of the irradiation, either all the positives or all the negatives are polymerized and trapped or bound to the sites, while the other population of beads can be released from the array.

In a preferred embodiment, the location of every capture probe is determined using decoder binding ligands (DBLs). As outlined above, DBLs are binding ligands that will either bind to identifier binding ligands, if present, or to the capture probes themselves, preferably when the capture probe is a nucleic acid or protein.

In a preferred embodiment, as outlined above, the DBL binds to the IBL.

In a preferred embodiment, the capture probes are single-stranded nucleic acids and the DBL is a substantially complementary single-stranded nucleic acid that binds (hybridizes) to the capture probe, termed a decoder probe herein. A decoder probe that is substantial complementary to each candidate probe is made and used to decode the array. In this embodiment, the candidate probes and the decoder probes should be of sufficient length (and the decoding step run-under suitable conditions) to allow specificity; i.e. each candidate probe binds to its corresponding decoder probe with sufficient specificity to allow the distinction of each candidate probe.

In a preferred embodiment, the DBLs are either directly or indirectly labeled. In a preferred embodiment, the DBL is directly labeled, that is, the DBL comprises a label. In an alternate embodiment, the DBL is indirectly labeled; that is, a labeling binding ligand (LBL) that will bind to the DBL is used. In this embodiment, the labeling binding ligand-DBL pair can be as described above for IBL-DBL pairs.

Accordingly, the identification of the location of the individual beads (or subpopulations of beads) is done using one or more decoding steps comprising a binding between the labeled DBL and either the IBL or the capture probe (i.e. a hybridization between the candidate probe and the decoder probe , when the capture probe is a nucleic add). After decoding, the DBLs can be removed and the array can be used; however, in some circumstances, for example when the DBL binds to an IBL and not to the capture probe, the removal of the DBL is not required (although it may be desirable in some circumstances). In addition, as outlined herein, decoding may be done either before the array is used to in an assay, during the assay, or after the assay.

In one embodiment, a single decoding step is done. In this embodiment, each DBL is labeled with a unique label, such that the the number of unique tags is equal to or greater than the number of capture probes (although in some cases, "reuse" of the unique labels can be done, as described herein; similarly, minor variants of candidate probes can share the same decoder, if the variants are encoded in another dimension, i.e. in the bead size or label). For each capture probe or IBL, a DBL is made that will specifically bind to it and contains a unique tag, for example one or more fluorochromes. Thus, the identity of each DBL, both its composition (i.e. its sequence when it is a nucleic add) and its label is known. Then by adding the DBLs to the array containing the capture probes under conditions which allow the formation of complexes (termed hybridization complexes when the components are nucleic adds) between the DBLs and either the capture probes or the IBLs, the location of each DBL can be elucidated. This allows the identification of the location of each capture probe; the random array has been decoded. The DBLs can then be removed, if necessary, and the target sample applied.

In a preferred embodiment, the number of unique labels is less than the number of unique capture probes, and thus a sequential series of decoding steps are used. In this embodiment, decoder probes are divided into n sets for decoding. The number of sets corresponds to the number of unique tags. Each decoder probe is labeled in n separate reactions with n distinct tags. All the decoder probes share the same n tags. The decoder probes are pooled so that each pool contains only one of the n tag versions of each decoder, and no two decoder probes have the same sequence of tags across all the pools. The number of pools required for this to be trues determined by the number of decoder probes and the n. Hybridization of each pool to the array generates a signal at every address.' The sequential hybridization of each pool in turn will generate a unique, sequence-specific code for each candidate probe. This identifies the candidate probe at each address in the array. For example, if four tags are used, then 4 X n sequential hybridizations can ideally distinguish $4^n$ sequences, although in some cases more steps may be required. After the hybridization of each pool, the hybrids are denatured and the decoder probes removed, so that the probes are rendered single-stranded for the next hybridization (although it is also possible to hybridize limiting amounts of target so that the available probe is not saturated. Sequential hybridizations can be carried out and analyzed by subtracting pre-existing signal from the previous hybridization).

An example is illustrative. Assuming an array of 16 probe nucleic adds (numbers 1-16), and four unique tags (four different fluors, for example; labels A-D). Decoder probes 1-16 are made that correspond to the probes on the beads. The first step is to label decoder probes 1-4 with tag A, decoder probes 5-8 with tag B, decoder probes 9-12 with tag C, and decoder probes 13-16 with tag D. The probes are mixed and the pool is contacted with the array containing the beads with the attached candidate probes. The location of each tag (and thus each decoder and candidate probe pair) is then determined. The first set of decoder probes are then removed. A second set is added, but this time, decoder probes 1, 5, 9 and 13 are labeled with tag A, decoder probes 2, 6, 10 and 14 are labeled with tag B, decoder probes 3, 7, 11 and 15 are labeled with tag C, and decoder probes 4, 8, 12 and 16 are labeled with tag D. Thus, those beads that contained tag A in both decoding steps contain candidate probe 1; tag A In the first decoding step and tag B in the second decoding step contain candidate probe 2: tag A In the first decoding step and tag C in the second step contain candidate probe 3; etc. In one embodiment, the decoder probes are labeled in situ; that is, they need not be labeled prior to the decoding reaction. In this embodiment, the incoming decoder probe is shorter than the candidate probe, creating a 5' "overhang" on the decoding probe. The addition of labeled ddNTPs (each labeled with a unique tag) and a polymerase will allow the addition of the tags in a sequence specific manner, thus creating a sequence-specific pattern of signals. Similarly, other modifications can be done, Including ligation, etc.

In addition, since the size of the array will be set by the number of unique decoding binding ligands, it is possible to "reuse" a set of unique DBLs to allow for a greater number of test sites. This may be done in several ways; for example, by using some subpopulations that comprise optical signatures. Similarly, the use of a positional coding scheme within an array; different sub-bundles may reuse the set of DBLs. Similarly, one embodiment utilizes bead size as a coding modality, thus allowing the reuse of the set of unique DBLs for each bead size. Alternatively, sequential partial loading of arrays with beads can also allow the reuse of DBLs. Furthermore, "code sharing" can occur as well.

In a preferred embodiment, the DBLs may be reused by having some subpopulation of beads comprise optical signatures. In a preferred embodiment to optical signature is generally a mixture of reporter dyes, preferably fluorescent By varying both the composition of the mixture (i.e. the ratio of one dye to another) and the concentration of the dye (leading to differences in signal intensity), matrices of unique optical signatures may be generated. This may be done by covalently attaching the dyes to the surface of the beads, or alternatively, by entrapping the dye within the bead.

In a preferred embodiment, the encoding can be accomplished in a ratio of at least two dyes, although more encoding dimensions may be added in the size of the beads, for example. In addition, the labels are distinguishable from one another; thus two different labels may comprise different molecules (i.e. two different fluors) or, alternatively, one label at two different concentrations or intensity.

In a preferred embodiment, the dyes are covalently attached to the surface of the beads. This may be done as is generally outlined for the attachment of the capture probes, using functional groups on the surface of the beads. As will be appreciated by those in the art, these attachments are done to minimize the effect on the dye.

In a preferred embodiment, the dyes are non-covalently associated with the beads, generally by entrapping the dyes in the pores of the beads.

Additionally, encoding in the ratios of the two or more dyes, rather than single dye concentrations, is preferred since it provides insensitivity to the intensity of light used to Interrogate the reporter dye's signature and detector sensitivity.

In a preferred embodiment, a spatial or positional coding system is done. In this embodiment, there are sub-bundles or subarrays (i.e. portions of the total array) that are utilized. By analogy with the telephone system, each subarray is an "area code", that can have the same tags (i.e. telephone numbers) of other subarrays; that are separated by virtue of the location of the subarray. Thus, for example, the same unique tags can be reused from bundle to bundle. Thus, the use of 50 unique tags in combination with 100 different subarrays can form an array of 5000 different capture probes. In this embodiment, it becomes important to be able to identify one bundle from another, in general, this is done either manually or through the use of marker beads, i.e. beads containing unique tags for each subarray.

In alternative embodiments, additional encoding parameters can be added, such as microsphere size. For example, the use of different size beads may also allow the reuse of sets of DBLs; that is, it is possible to use microspheres of different sizes to expand the encoding dimensions of the microspheres. Optical fiber arrays can be fabricated containing pixels with different fiber diameters or cross-sections; alternatively, two or more fiber optic bundles, each with different cross-sections of the individual fibers, can be added together to form a larger bundle; or fiber optic bundles with fiber of the same size cross-sections can be used, but just with different sized beads. With different diameters, the largest wells can be filled with the largest microspheres and then moving onto progressively smaller microspheres in the smaller wells until all size wells are then filled. In this manner, the same dye ratio could be used to encode microspheres of different sizes thereby expanding the number of different oligonucleotide sequences or chemical functionalities present in the array. Although outlined for fiber optic substrates, this as well as the other methods outlined herein can be used with other substrates and with other attachment modalities as well.

In a preferred embodiment, the coding and decoding is accomplished by sequential loading of the microspheres into the array. As outlined above for spatial coding, in this embodiment, the optical signatures can be 'reused'. In this embodiment, the library of microspheres each comprising a different capture probe (or the subpopulations each comprise a different capture probe), is divided into a plurality of sublibraries; for example, depending on the size of the desired array and the number of unique tags, 10 sublibraries each comprising roughly 10% of the total library may be made, with each sublibrary comprising roughly the same unique tags. Then, the first sublibrary is added to the fiber optic bundle comprising the wells, and the location of each capture probe is determined, generally through the use of DBLs. The second sublibrary is then added, and the location of each capture probe is again determined. The signal in this case will comprise the signal from the first DBL and the "second" DBL; by comparing the two matrices the location of each bead in each sublibrary can be determined. Similarly, adding the third, fourth, etc. sublibraries sequentially will allow the array to be filed.

In a preferred embodiment, codes can be "shared" In several ways. In a first embodiment, a single code (i.e. IBUDBL pair) can be assigned to two or more agents if the target sequences different sufficiently in their binding strengths. For example, two nucleic acid probes used in an mRNA quantitation assay can share the same code if the ranges of their hybridization signal intensities do not overlap. This can occur for example, when one of the target sequences is always present at a much higher concentration than the other. Alternatively, the two target sequences might always be present at a similar concentration, but differ in hybridization efficiency.

Alternatively, a single code can be assigned to multiple agents if the agents are functionally equivalent. For example, if a set of oligonucleotide probes are designed with the common purpose of detecting the presence of a particular gene, then the probes are functionally equivalent, even though they may differ in sequence. Similarly, an array of this type could be used to detect homologs of known genes, In this embodiment each gene is represented by a heterologous set of probes, hybridizing to different regions of the gene (and therefore differing in sequence). The set of probes share a common code. If a homolog is present, it might hybridize to some but not all of the probes. The level of homology might be indicated by the fraction of probes hybridizing, as well as the average hybridization intensity. similarly, multiple antibodies to the some protein could all share the same code.

In a preferred embodiment, decoding of self-assembled random arrays is done on the bases of pH titration. In this embodiment, in addition to capture probes, the beads comprise optical signatures, wherein the optical signatures are generated by the use of pH-responsive dyes (sometimes referred to herein as "ph dves") such as fluorophores. This embodiment is similar to that outlined in WO98/40726 and US6327410. except:

> that the dyes used In the present ivention exhibit changes m fluorescence intensity (or other properties) when the solution pH is adjusted from below the pKa to above the pKa (or vice versa). In a preferred embodiment, a set of pH dyes are used, each with a different pKa, preferably separated by at least 0.5 pH units. Preferred embodiments utilize a pH dye set of pKa's of 2.0, 2.5, 3,0. 3.5, 4.0, 4.5, 5.0, 5.5, 8.0, 6.5, 7.0, 7.5, 8.0, 8.5. 8.0, 9.5.10.0,10.5, 11, and 11.5. Each bead can contain any subset of the pH dyes, and in this way a unique code for the capture probe is generated. Thus, the decoding of an array is achieved by titrating the array from pH 1 to pH 13. and measuring the fluorescence signal from each bead as a function of solution pH.

Thus, the present disclosure provides array compositions comprising a substrate with a surface comprising discrete sites. A population of microspheres is distributed on the sites, and the population comprises at least a first and a second subpopulation. Each subpopulation comprises a capture probe, and, in addiction, at least one optical dye with a given pKa. The pKas of the different optical dyes are different.

In a preferred embodiment, "random" decoding probes can be made. By sequential hybridizations or the use of multiple labels, as is outlined above, a unique hybridization pattern can be generated for each sensor element. This allows all the beads representing a given clone to be identified as belonging to the same group. In general, this is done by using random or partially degenerate decoding probes, that bind in a sequence-dependent but not highly sequence-specific manner. The process can be repeated a number of times, each time using a different labeling entity, to generate a different pattern of singals based on quasi-specific interactions. In this way, a unique optical signature is eventually built up for each sensor element By applying pattern recognition or clustering algorithms to the optical signatures, the beads can be grouped Into sets that share the same signature (i.e. carry the same probes).

In order to identify the actual sequence of the clone itself, additional procedures are required; for example, direct sequencing can be done, or an ordered array containing the clones, such as a spotted cDNA array, to generate a "key" that links a hybridizations pattern to a specific clone.

Alternatively, clone arrays can be decoded using binary decoding with vector tags. For example, partially randomized oligos are cloned into a nucleic acid vector (e.g. plasmid, phage, etc.). Each oligonucleotide sequence consists of a subset of a limited set of sequences. For example, if the limites set comprises 10 sequences, each oligonucleotide may have some subset (or all of the 10) sequences. Thus each of the 10 sequences can be present or absent in the oligonucleotide: Therefore, there are $2^{10}$ or 1,024 possible combinations. The sequences may overlap, and minor variants can also be represented (e.g. A, C, T and G substitutions) to increase the number of possible combinations. A nucleic acid library is cloned into a vector containing the random code sequences. Alternatively, other methods such as PCR can be used to add the tags. In this way it is possible to use a small number of oligo decoding probes to decode an array of clones.

As will be appreciated by those in the art, the systems of the disclosure may take on a large number of different configurations, as is generally depicted in the Figures. In general, there are three types of systems that can be used: (1) "non-sandwich' systems (also referred to herein as "direct" detection) in which the target sequence itself is labeled with detectable labels (again, either because the primers comprise labels or due to the incorporation of labels into the newly synthesized strand); (2) systems in which label probes directly bind to the target analytes; and (3) systems in which label probes are indirectly bound to the target sequences, for example through the use of amplifier probes.

Detection of the reactions, including the direct detection of products and indirect detection utilizing label probes (i.e, sandwich assays), is preferably done by detecting assay complexes comprising detectable labels, which can be attached to the assay complex in a variety of ways, as is more fully described below.

Once the target sequence has preferably been anchored to the array, an amplifier probe is hybridized to the target sequence, either directly, or through the use of one or more label extender probes, which serves to allow "generic" amplifier probes to be made. As for all the steps outlined herein, this may be done simultaneously with capturing, or sequentially. Preferably, the amplifier probe contains a multiplicity of amplification sequences, although in some embodiments, as described below, the amplifier probe may contain only a single amplification sequence, or at least two amplification sequences. The amplifier probe may take on a number of different forms; either a branched conformation, a dendrimer conformation, or a linear "string" of amplification sequences. Label probes comprising detectable labels (preferably but not required to be fluorophores) then hybridize to the amplification sequences (or in some cases the label probes hybridize directly to the target sequence), and the labels detected, as is more fully outlined below.

Accordingly, the present disclosure provides compositions comprising an amplifier probe. By amplifier probe or "nucleic acid multimer" or "amplication multimer" or grammatical equivalents herein is meant a nucleic acid probe that is used to facilitate signal amplification. Amplifier probes comprise at least a first single-stranded nucleic acid probe sequence, as defined below, and at least one single-stranded nucleic acid amplification sequence, with a multiplicity of amplification sequences being preferred.

Amplifier probes comprise a first probe sequence that is used, either directly or indirectly, to hybridize, to the target sequence. That is, the amplifier probe itself may have a first probe sequence that is substantially complementary to the target sequence, or it has a first probe sequence that it substantially complementary to a portion of an additional probe, in this case called a label extender probe, that has a first portion that is substantially complementary to the target sequence. In a preferred embodiment, the first probe sequence of the amplifier probe is substantial complementary to the target sequence.

In general, as for all the probes herein, the first probe sequence is of a length sufficient to give specificity and stability. Thus generally, the probe sequences of the invention that are designed to hybridize to another nucleic acid (i.e, probe sequences, amplification sequences, portions or domains of larger probes) are at least about 5 nucleosides long, with at least about 10 being preferred and at least about 15 being especially preferred.

In a preferred embodiment, several different amplifier probes are used, each with first probe sequences that will hybridize to a different portion of the target sequence. That is, there is more than one level of amplification; the amplifier probe provides an amplification of signal due to a multiplicity of labelling events, and several different amplifier probes, each with this multiplicity of labels, for each target sequence is used. Thus, preferred embodiments utilize at least two different pools of amplifier probes, each pool having a different probe sequence for hybridization to different portions of the target sequence; the only real limitation on the number of different amplifier probes will be the length of the original target sequence. In addition, it is also possible that the different amplifier probes contain different amplification sequences, although this is generally not preferred.

In a preferred embodiment, the amplifier probe does not hybridize to the sample target sequence directly, but instead hybridizes to a first portion of a label extended probe. This is particularly useful to allow the use of "generic" amplifier probes, that is, amplifier probes that can be used with a variety of different targets. This may be desirable since several of the amplifier probes require special synthesis techniques. Thus, the addition of a relatively short probe as a label extender probe is preferred. Thus, the first probe sequence of the amplifier probe is substantially complementary, to a first portion or domain of a first label extender single-stranded nucleic acid probe. The label extender probe also contains a second portion or domain that is substantially complementary to a portion of the target sequence. Both of these portions are preferably at least about 10 to about 50 nucleotides in length, with a range of about 15 to about 30 being preferred. The terms "first" and "second" are not meant to confer an orientation of the sequences with respect to the 5'-3' orientation of the target or probe sequences. For examples, assuming a 5'-3' orientation of the complementary target sequence, the first portion may be located either 5' to the second portion, or 3' to the second portion. For convenience herein, the order of probe sequences are generally shown from left to right.

In a preferred embodiment, more than one label extender probe-amplifier probe pair may be used, that Is, n is more than 1. That is, a plurality of label extender probes may be used, each with a portion that is substantiate complementary to a different portion of the target sequence; this can serve as another level of amplification. Thus, a preferred embodiment utilizes pools of at least two label extender probes, with the upper limit being set by the length of the target sequence.

in a preferred embodiment, more than one label extender probe is used with a single amplifier probe to reduce non-specific binding, as is generally outlined in U.S. Patent No. 5,881,887

i. In this embodiment, a first portion of the first label extender probe hybridizes to a first portion of the target sequence, and the second portion of the first label extender probe hybridizes to a first probe sequence of the amplifier probe. A first portion of the second label extender probe hybridizes to a second portion of the target sequence, and the second portion of the second label extender probe hybridizes to a second probe sequence of the amplifier probe. These form structures sometimes referred to as "cruciform" structures or configurations, and are generally done to confer stability when large branched or dendrimeric amplifier probes are used.

In addition, as will be appreciated by those in the art, the label extender probes may interact with a preamplifier probe, described below, rather than the amplifier probe directly.

Similarly, as outlined above, a preferred embodiment utilizes several different amplifier probes, each with first probe sequences that will hybridize to a different portion of the label extender probe. In addition, as outlined above, it is also possible that the different amplifier probes contain different amplification sequences, although this is generally not preferred.

In addition to the first probe sequence, the amplifier probe also comprises at least one amplification sequence. An "amplification sequence" or "amplification segment" or grammatical equivalents herein is meant a sequence that is used, either directly or indirectly, to bind to a first portion of a label probe as is more fully described below. Preferably, the amplifier probe comprises a multiplicity of amplification sequences, with from about 3 to about 1000 being preferred, from about 10 to about 100 being particularly preferred, and about 50 being especially preferred. In some cases, for example, when linear amplifier probes are used, from 1 to about 20 is preferred with from about 5 to about 10 being particularly preferred.

The amplification sequences may be linked to each other In a variety of ways, as will be appreciated by those in the art. They may be covalently linked directly to each other, or to intervening sequences or chemical moieties, through nucleic acid linkages such as phosphodiester bonds, PNA bonds, etc., or through interposed linking agents such amino acid, carbohydrate or polyol bridges, or through other cross-linking agents or binding partners. The site(s) of linkage may be at the ends of a segment, and/of at one or more internal nucleotides in the strand. In a preferred embodiment, the amplification sequences are attached via nucleic acid linkages.

In a preferred embodiment branched amplifier probes are used, as are generally described in U.S. Patent No. 5,124,246. Branched amplifier probes may take on "fork-like" or "comb-like" conformations. "Fork-like" branched amplifier probes generally have three or more oligonucleotide segments emanating from a point of origin to form a branched structure. The point of origin may be another nucleotide segment or a multifunctional molecule to whcih at least three segments can be covalently or tightly bound. "Comb-like" branched amplifier probes have a linear backbone with a multiplicity of side chain oligonucleotides extending from the backbone. In either, conformation, the pendant segments will normally depend from a modified nucleotide or other organic moiety having the appropriate functional groups for attachment of oligonucleotides. Furthermore, in either conformation, a large number of amplification sequences are available for binding, either directly or indirectly, to detection probes. In general, these structures are made as is known In the art, using modified multifunctional nucleotides, as is described in U.S. Patent Nos. 5,635,352 and 5,124,248, among others.

In a preferred embodiment, dendrimer amplifier probes are used, as are generally described in U.S. Patent No. 5,175,270, Dendrimeric amplifier probes have amplification sequences that are attached via hybridization, and thus have portions of double-stranded nucleic acid as a component of their structure. The outer surface of the dendrimer amplifier probe has a multiplicity of amplification sequences.

In a preferred embodiment, linear amplifier probes are used, that have individual amplification sequences linked end-to-end either directly or with short intervening sequences to form a polymer. As with the other amplifier configurations, there may be additional sequences or moieties between the amplification sequences. In one embodiment, the linear amplifier probe has a single amplification sequence.

In addition, the amplifier probe may be totally linear, totally branched, totally dendrimeric, or any combination thereof.

The amplification sequences of the amplifier probe are used, either directly or indirectly, to bind to a label probe to allow detection. In a preferred embodiment, the amplification sequences of the amplifier probe are substantially complementary to a first portion of a label probe. Alternatively; amplifier extender, probes are used, that have a first portion that binds to the amplification sequence and a second portion that binds to the first portion of the label probe.

In addition, the compositions may include "preamplifier" molecules, which serves a bridging moiety between the label extender molecules and the amplifier probes. In this way, more amplifier and thus more labels are ultimately bound to the detection probes. Preamplifier molecules may be either linear or branched, and typically contain in the range of about 30-3000 nucleotides

Thus, label probes are either substantially complementary to an amplification sequence or to a portion of the target sequence.

Detection of the nucleic add reactions, including the direct detection of genotyping products and indirect detection utilizing label probes (i.e. sandwich assays), is done by detecting assay complexes comprising labels.

In a preferred embodiment, several levels of redundancy are built into the arrays. Building redundancy into an array gives several significant advantages, including the ability to make quantitative estimates of confidence about the data and signficant increases in sensitivity. Thus, preferred embodiments utilize array redundancy. As will be appreciated by those in the art, there are at least two types of redundancy that can be built into an array: the use of multiple identical sensor elements (termed herein "sensor redundancy"), and the use of multiple sensor elements directed to the same target analyte, but comprising different chemical functionalities (termed herein "target redundancy"). For example, for the detection of nucleic acids, sensor redundancy utilizes of a plurality of sensor elements such as beads comprising identical binding ligands such as probes. Target redundancy utilizes sensor elements with different probes to the same target one probe may span the first 25 bases of the target, a second probe may span the second 25 bases of the target, etc. By building in either or both of these types of redundancy into an array, significant benefits are obtained. For example, a variety of statistical mathematical analyses may be done.

In addition, while this is generally described herein for bead arrays, as will be appreciated by those in the art, this techniques can be used for any type of arrays designed to detect target analytes. Furthermore, while these techniques are generally described for nucleic acid systems, these techniques are useful in the detection of other binding ligand/target analyte systems as well.

In a preferred embodiment, sensor redundancy is used. In this embodiment, a plurality of sensor elements, e.g. beads, comprising identical bioactive agents are used. That is, each subpopulation comprises a plurality of beads comprising identical bioactive agents (e.g. binding ligands). By using a number of identical sensor elements for a given array, the optical signal from each sensor element can be combined and any number of statistical analyses run, as outlined below. This can be done for a variety of reasons. For example, in time varying measurements, redundancy can significantly reduce the noise in the system. For non-time based measurements, redundancy can significantly increase the confidence of the data.

In a preferred embodiment, a plurality of identical sensor elements are used. As will be appreciated by those in the art, the number of identical sensor elements will vary with the application and use of the sensor array. In general, anywhere from 2 to thousands may be used, with from 2 to 100 being preferred, 2 to 50 being particularly preferred and from 5 to 20 being especially preferred. In general, preliminary results indicate that roughly 10 beads gives a sufficient advantage, although for some applications, more identical sensor elements can be used.

Once obtained, the optical response signals from a plurality of sensor beads within each bead subpopulation can be manipulated and analyzed in a wide variety of ways, including baseline adjustment, averaging, standard deviation analysis, distribution and cluster analysis, confidence interval analysis, mean testing, etc.

In a preferred embodiment, the first manipulation of the optical response signals is an optional baseline adjustment, In a typical procedure, the standardized optical responses are adjusted to start at a value of 0.0 by subtracting the integer 1.0 from all data points. Doing this allows the baseline-loop data to remain at zero even when summed together and the random response signal noise is canceled out. When the sample is a fluid, the fluid pulse-loop temporal region, however, frequently exhibits a characteristic change in response, either positive, negative or neutral, prior to the sample pulse and often requires a baseline adjustment to overcome noise associated with drift in the first few data points due to charge buildup in the CCD camera. If no drift is present, typically the baseline from the first data point for each bead sensor is subtracted from all the response data for the same bead. If drift is observed, the average baseline from the first ten data points for each bead sensor is substracted from the all the response data for the same bead. By applying this baseline adjustment, when multiple bead responses are added together they can be amplified while the baseline remains at zero. Since all beads respond at the same time to the sample (e.g. the sample pulse), they all see the pulse at the exact same time and there is no registering or adjusting needed for overlaying their responses, In addition, other types of baseline adjustment may be done, depending on the requirements and output of the system used.

Once the baseline has been adjusted, a number of possible analyses may be run to generate known statistical parameters. Analyses based on redundancy are known and generally described in texts such as Freund and Walpole, Mathematical Statistics, Prentice Hall, Inc. New Jersey, 1980,

In a preferred embodiment, signal summing is done by simply adding the intensity values of all responses at each time point, generating a new temporal response comprised of the sum of all bead responses. These values can be baseline-adjusted or raw. As for all the analyses described herein, signal summing can be performed in real time or during post-data- acquisition data reduction and analysis. In one embodiment, signal summing is performed with a commercial spreadsheet program (Excel, Microsoft, Redmond, WA) after optical response date is collected.

In a preferred embodiment, cummulative response data is generated by simply adding all data points in successive time intervals. This final column, comprised of the sum of all data points at a particular time interval, may then be compared or plotted with the individual bead responses to determine the extent of signal enhancement or improved signal to noise ratios.

In a preferred embodiment, the mean of the subpopulation (i.e. the plurality of identical beads) is determined, using the well known Equation 1:

**Equation 1**

$$\mu = \sum \frac{x_i}{n}$$

In some embodiments, the subpopulation may be redefined to exclude some beads if necessary (for example for obvious outliers, as discussed below).

In a preferred embodiment, the standard deviation of the subpopulation can be determined, generally using Equation (for the entire subpopulation) and Equation 3 (for less than the entire subpopulation);

**Equation 2**

$$\sigma = \sqrt{\frac{\sum (x_i - \mu)^2}{n}}$$

**Equation 3**

$$s = \sqrt{\frac{\sum (x_i - \bar{x})^2}{n\ 1}}$$

As for the mean, the subpopulation may be redefined to exclude some beads if necessary (for example for obvious outliers, as discussed below).

In a preferred embodiment, statistical analyses are done to evaluate whether a particular data point has statistical validity within a subpopulation by using techniques including, but not limited to, t distribution and cluster analysis. The may be done to statistical discard outliers that may otherwise skew the result and increase the signal-to-nose ratio of any particular experiment. This may be done using Equation 4:

**Equation 4**

$$t = \frac{\bar{x}\ \mu}{s/\sqrt{n}}$$

In a preferred embodiment, the quality of the data is evaluated using confidence intervals, as is known in the art. Confidence intervals can be used to facilitate more comprehensive data processing to measure the statistical validity of a result.

In a preferred embodiment, statistical parameters of a subpopulation of beads are used to do hypothesis testing. One application is tests concerning means, also called mean testing. In this application, statistical evaluation is done to determine whether two subpopulations are different. For example, one sample could be compared with another sample for each subpopulation within an array to determine if the variation is statistically significant.

In addition, mean testing can also be used to differentiate two different assays that share the same code. If the two assays give results that are statistically distinct from each other, then the subpopulations that share a common code can be distinguished from each other on the basis of the assay and the mean test, shown below in Equation 5:

**Equation 5**

$$z = \frac{\bar{x}_1 - \bar{x}_2}{\sqrt{\frac{\sigma_1^2}{n_1} + \frac{\sigma_2^2}{n_2}}}$$

Furthermore, analyzing the distribution of individual members of a subpopulation of sensor elements may be done. For example, a subpopulation distribution can be evaluated to determine whether the distribution is binomial, Poisson, hypergeometric, etc.

In addition to the sensor redundancy, a preferred embodiment utilizes a plurality of sensor elements that are directed to a single target analyte but yet are not identical. For example, a single target nucleic acid analyte may have two or more sensor elements each comprising a different probe. This adds a level of confidence as non-specific binding interactions can be statistically minimized. When nucleic acid target analytes are to be evaluated, the redundant nucleic acid probes may be overlapping, adjacent, or spatially separated. However, it is preferred that two probes do not compete for a single binding site, so adjacent or separated probes are preferred. Similarly, when proteinaceous target analytes are to be evaluated, preferred embodiments utilize bioactive agent binding agents that bind to different parts of the target. For example, when antibodies (or antibody fragments) are used as bioactive agents for the binding of target proteins, preferred embodiments utilize antibodies to different epitopes.

In this embodiment, a plurality of different sensor elements may be used, with from about 2 to about 20 being preferred, and from about 2 to about 10 being especially preferred, and from 2 to about 5 being particularly preferred, including 2, 3, 4 or 5. Howeve, as above, more may also be used, depending on the application.

As above, any number of statistical analyses may be run on the data from target redundant sensors.

One benefit of the sensor element summing (referred to herein as "bead summing" when beads are used), is the increase in sensitivity that can occur.

In addition, the present disclosure is directed to the use of adapter sequences to assemble arrays comprising target analytes, including non-nucleic acid target analytes. By "target analyte" or "analyte" or grammatical equivalents herein is meant any molecule, compound or particle to be detected. As outlined below, target analytes preferably bind to binding ligands, as is more fully described below. As will be appreciated by those in the art, a large number of analytes may be detected using the present methods; basically, any target analyte for which a binding ligand, described below, may be made may be detected using the methods of the disclosure.

Suitable analytes include organic and inorganic molecules, including biomolecules. The analyte may be an environmental pollutant (including pesticides, insecticides, toxins, etc.); a chemical (including solvents, polymers, organic materials, etc.); therapeutic molecules (including therapeutic and abused drugs, antibiotics, etc.); biomolecules (including hormones, cytokines, proteins, lipids, carbohydrates, cellular membrane antigens and receptors (neural, hormonal, nutrient, and cell surface receptors) or their ligands, etc); whole cells (including procaryotic (such as pathogenic bacteria) and eukaryotic cells, including mammalian tumor cells); viruses (including retroviruses, herpesviruses, adenoviruses, lentiviruses, etc.); and spores; etc. Disclosed analytes are environmental pollutants; nucleic acids; proteins (including enzymes, antibodies, antigens, growth factors, cytokines, etc); therapeutic and abused drugs; cells; and viruses.

The target analyte may be a protein. As will be appreciated by those in the art, there are a large number of possible proteinaceous target analytes that may be detected using the present invention. By "proteins' or grammatical equivalents herein is meant proteins, oligopeptides and peptides, derivatives and analogs, including proteins containing non-naturally occurring, amino acids and amino acid analogs, and peptidomimetic structures. The side chains may be in either the (R) or the (S) configuration, In a preferred embodiment, the amino acids are in the (S) or L-configuration. As discussed below, when the protein is used as a binding ligand, it may be desirable to utilize protein analogs to retard degradation by sample contaminants.

Suitable protein target analytes include, but are not limited to, (1) immunoglobulins, particularly IgEs, IgGs and IgMs, and particularly therapeutically or diagnostically relevant antibodies, including but not limited to, for example, antibodies to human albumin, apolipoproteins (including apolipoprotein E), human chorionic gonadotropin, cortisol, α-fetoprotein, thyroxin, thyroid stimulating hormone (TSH), antithrombin, antibodies to pharmaceuticals (including antieptileptic drugs (phenytoin, primidone, carbariezepin, ethosuximide. valproic acid, and phenobarbitol), cardioactive drugs (dioxin, lidocaine, procainamide, and disopyramide), bronchodilators (theophylline), antibiotics (chloramphenicol, sulfonamides), antidepressants, immunosuppresants, abused drugs (amphetamine, methamphetamine, cannabinoids, cocaine and opiates) and antibodies to any number of viruses (including orthomyxoviruses, (e.g. influenza virus), paramyxoviruses (e.g respiratory syncytial virus, mumps virus, measles virus), adenoviruses, rhinoviruses, coronaviruses, reoviruses, togaviruses (e.g. rubella virus), parvoviruses, poxviruses (e.g. variola virus, vaccinia virus), enteroviruses (e.g. poliovirus, coxsackievirus), hepatitis viruses (including A, B and C), herpesviruses (e.g. Herpes simplex virus, varicella-zoster virus, cytomegalovirus, Epstein-Barr virus), rotaviruses, Norwalk viruses, hantavirus, arenavirus, rhabdovirus (e.g. rabies virus), retroviruses (including HIV, HTLV-I and -II), papovaviruses (e.g. papillomavirus), polyomaviruses, and picornaviruses, and the like), and bacteria (including a wide variety of pathogenic and non-pathogenic prokaryotes of interest including Bacillus; Vibrio, e.g. *V. Cholerae;* Escherichia, e.g. Enterotoxigenic *E. coli*, Shigella, e.g. *S. dysenteriae;* Salmonella, e.g. *S. typhi;* Mycobacterium e.g. *M. tuberculosis, M. leprae;* Clostridium, e.g. *C. botulinum, C. tetani, C. difficile, C.perfringens;* Comyebacterium, e.g. *C. diphtheriae;* Streptococcus, *S. pyogenes, S. pneumoniae;* Staphylococcus, *e.g. S. aureus;* Haemophilus, e.g. *H. influenzae;* Neisseria. e.g. *N. meningitidis, N. gonorrhoeae;* Yersinia, e.g. *G. lambliaY. pestis,* Pseudomonas, e.g. *P. aeruginosa, P. putida;* Chlamydia, e.g. *C. trachomatis;* Bordetella, e.g. *B. pertussis;* Treponema, e.g. *T. palladium;* and the like); (2) enzymes (and other proteins), including but not limited to, enzymes used as indicators of or treatment for heart disease, including creatine kinase, lactate dehydrogenase, aspartate amino transferase, troponin T, myoglobin, fibrinogen, cholesterol, triglycerides, thrombin, tissue plasminogen activator (tPA); pancreatic disease indicators including amylase, lipase, chymotrypsin and trypsin; liver function enzymes and proteins including cholinesterase, bilirubin, and alkaline phosphotase; aldolase, prostatic acid phosphatase, terminal deoxynucleotidyl transferase, and bacterial and viral enzymes such as HIV protease; (3) hormones and cytokines (many of which serve as ligands for cellular receptors) such as erythropoietin (EPO), thrombopoietin (TPO), the interleukins (including IL-1 through IL-17), insulin, insulin-like growth factors (including IGF-1 and -2), epidermal growth factor (EGF), transforming growth factors (including TGF-α and TGF-β), human growth hormone, transferrin, epidermal growth factor (EGF), low density lipoprotein, high density lipoprotein, leptin, VEGF, PDGF, ciliary neurotrophic factor, prolactin, adrenocorticotropic hormone (ACTH), calcitonin, human chorionic gonadotropin, cotrisol, estradiol, follicle stimulating hormone (FSH), thyroid-stimulating hormone (TSH), leutinzing hormone (LH), progeterone, testosterone, ; and (4) other proteins (including α-fetoprotein, carcinoembryonic antigen CEA.

In addition any of the biomolecules for which antibodies may be detected may be detected directly as well; that is, detection of virus or bacterial cells, therapeutic and abused drugs, etc., may be done directly.

Suitable target analytes include carbohydrates, including but not limited to, markers for breast cancer (CA15-3, CA 549, CA 27.29). mucin-like carcinoma associated antigen (MCA), ovarian cancer (CA125), pancreatic cancer (DE-PAN-2), and colorectal and pancreatic cancer (CA 19, CA 50, CA242).

The adapter sequences may be chosen as outlined above. These adapter sequences can then be added to the target analytes using a variety of techniques. In general, as described above, non covalent attachment using binding partner pairs may be done, or covalent attachment using chemical moieties (including linkers).

Once the adapter sequences are associated with the target analyte, including target nucleic acids, the compositions are added to an array. In one embodiment a plurality of hybrid adapter sequence/target analytes are pooled prior to addition to an array. All of the methods and compositions herein are drawn to compositions and methods for detecting the presence of target analytes, particularly nucleic acids, using adapter arrays.

Advantages of using adapters include but are not limited to, for example, the ability to create universal arrays. That is, a single array is utilized with each capture probe designed to hybridize with a specific adapter. The adapters are joined to any number of target analytes, such as nucleic acids, as is described herein. Thus, the same array is used for vastly different target analyses. Furthermore, hybridization of adapters with capture probes results in non-covalent attachment of the target nucleic acid to the microsphere. As such, the target nucleic/adapter hybrid is easily removed, and the microsphere/capture probe can be re-used. In addition, the construction of kits is greatly facilitated by the use of adapters. For example, arrays or microspheres can be prepared that comprise the capture probe; the adapters can be packaged along with the microspheres for attachment to any target analyte of interest. Thus, one need only attach the adapter to the target analyte and disperse on the array for the construction of an array of target analytes.

Once made, the compositions find use in a number of applications. The compositions may be used to probe a sample solution for the presence or absence of a target sequence, including the quantification of the amount of target sequence present.

For SNP analysis, the ratio of different labels at a particular location on the array indicates the homozygosity or heterozygosity of the target sample, assuming the same concentration of each readout probe is used. Thus, for example, assuming a first readout probe comprising a first base at the readout position with a first detectable label and a second readout probe comprising a second base at the readout position with a second detectable label, equal signals (roughly 1:1 (taking into account the different signal intensities of the different labels, different hybridization efficiencies, and other reasons)) of the first and second labels indicates a heterozygote. The absence of a signal from the first label (or a ratio of approximately 0:1) indicates a homozygote of the second detection base; the absence of a signal from the second label (or a ratio of approximately 1:0) indicates a homozygote for the first detection base. As is appreciated by those in the art, the actual ratios for any particular system are generally determined empirically. The ratios also allow for SNP quantitation

The present disclosure also finds use as a methodology for the detection of mutations or mismatches in target nucleic acid sequences. For example, recent focus has been on the analysis of the relationship between genetic variation and phenotype by making use of polymorphic DNA markers. Previous work utilized short tandem repeats (STRs) as polymorphic positional markers; however, recent focus is on the use of single nucleotide polymorphisms (SNPs), which occur at an average frequency of more than 1 per kilobase in human genomic DNA. Some SNPs, particularly those in and around coding sequences, are likely to be the direct cause of therapeutically relevant phenotypic variants. There are a number of well known polymorphisms that cause clinically important phenotypes; for example, the apoE2/3/4 variants are associated with different relative risk of Alzheimer's and other diseases (see Cordor et al., Science 261 (1993). Multiplex PCR amplification of SNP loci with subsequent hybridization to oligonucleotide arrays has been shown to be an accurate and reliable method of simultaneously genotyping at least hundreds of SNPs; see Wang et al., Science, 280:1077 (1998); see also Schafer et al., Nature Biotechnology 16:33-39 (1998). The compositions of the present invention may easily be substituted for the arrays of the prior art.

Generally, a sample containing a target analyte (whether for detection of the target analyte or screening for binding partners of the target analyte) is added to the array, under conditions suitable for binding of the target analyze to at least one of the capture probes, i.e. generally physiological conditions. The presence or absence of the target analyte is then detected. As will be appreciated by those In the art, this may be done in a variety of ways, generally through the use of a change in an optical signal. This change can occur via many different mechanisms. A few examples include the binding of a dye-tagged analyte to the bead, the production of a dye species on or near the beads, the destruction of an existing dye species, a change in the optical signature upon analyte interaction with dye on bead, or any other optical interrogatable event

Preferably, the change in optical signal occurs as a result of the binding of a target analyte that is labeled, either directly or indirectly, with a detectable label, preferably an optical label such as a fluorochrome. Thus, for example, when a proteinaceous target analyte is used, it may be either directly labeled with a fluor, or indirectly, for example through the use of a labeled antibody. Similarly, nucleic acids are easily labeled with fluorochromes, for example during PCR amplification Similarly known in the art. Alternatively, upon binding of the target sequence, a hydridization indicator may be used as the label. Hybridization indicators preferentially-associate with-double stranded nucleic acid, usually reversibly. Hybridization indicators include intercalators and minor and/or major groove binding moieties. In a preferred embodiment, intercalators may be used; since intercalation generally only occurs In the presence of double stranded nucleic acid, only in the presence of target. hybridization will the label light up. Thus, upon binding of the target analyte to a capture probe, there is a new optical signal generated at that site, which then may be detected.

Alternatively, in some cases, as discussed above, the target analyte such as an enzyme generates a species that is either directly or indirectly optical detectable.

Furthermore, in some embodiments, a change in the optical signature may be the basis of the optical signal. For example, the interaction of some chemical target analytes with some fluorescent dyes on the beads may alter the optical signature, thus generating a different optical signal.

As will be appreciated by those in the art, in some embodiments, the presence or absence of the target analyte may be done using changes in other optical or non-optical signals, including, but not limited to, surface enhanced. Raman spectroscopy. surface plasmon resonance, radioactivity, etc. ,

The assays may be run under a variety of experimental conditions, as will be appreciated by those in the art. A variety of other reagents may be included in the screening assays. These include reagents like salts, neutral proteins, e.g. albumin, detergents, etc which may be used to facilitate optimal protein-protein binding and/or reduce non-specific or background interactions. Also reagents that otherwise improve the efficiency of the assay, such as protease inhibitors, nuclease inhibitors, anti-microbial agents, etc., may be used. The mixture of components may be added in any order that provides for the requisite binding. Various blocking and washing steps may be utilized as is known in the art.

In addition, the present invention provides kits for the reactions of the inventions, comprising components of the assays as disclosed in the appended claims. In addition, a variety of other reagents may be included In the assays or the kits. These include reagents like salts, neutral proteins, e.g. albumin, detergents, etc which may be used to facilitate optimal protein-protein binding and/or reduce non-specific or background interactions. Also reagents that otherwise improve the efficiency of the assay, such as protease inhibitors, nuclease inhibitors, anti-microbial agents, etc., may be used. The mixture of components may be added in any order that provides for the requisite activity.

## Claims

1. A method of sequencing a plurality of target nucleic acids each comprising a first domain and an adjacent second domain, said second domain comprising a plurality of detection positions, said method comprising:

    a) providing a plurality of hybridization complexes each comprising a target sequence and a sequencing primer that hybridizes to the first domain of said target sequence, said hybridization complexes being attached to microspheres, said microspheres being associated with discrete individual sites on the surface of a substrate, wherein said substrate is an optical fiber bundle;
    b) extending each of said primers by the addition of a first nucleotide to the first detection position using a first enzyme to form an extended primer; and
    c) detecting the release of pyrophosphate (PPi) to determine the type of said first nucleotide added onto said primers;

    wherein said method comprises the use of secondary enzymes to generate a chemiluminescent signal, wherein said secondary enzymes are attached to said microspheres.

2. A method according to claim 1, wherein said sequencing primers are attached to said surface.

3. A method according to claim 1, wherein each of said hybridization complexes comprise a target sequence, said sequencing primer and a capture probe for hybridizing to said target sequence covalently attached to said surface.

4. A method according to claim 1, wherein each of said hybridization complexes comprises a target sequence, said sequencing primer, an adaptor probe and a capture probe for hybridizing to said target sequence covalently attached to said surface.

5. A method according to claim 3 or claim 4, wherein said microspheres comprise subpopulations of microspheres, wherein each microsphere in a subpopulation contains the same capture probe.

6. A method according to claim 3, claim 4 or claim 5, wherein said capture probe also serves as said sequencing primer.

7. A method according to claim 1 further comprising

    d) extending said extended primer by the addition of a second nucleotide to the second detection position using said enzyme; and
    e) detecting the release of pyrophosphate (PPi) to determine the type of said second nucleotide added onto said primers.

8. The method according to claim 1, wherein step (c) comprises:

i) contacting said PPi with a second enzyme that converts said PPi into ATP; and
ii) detecting said ATP using a third enzyme.

**9.** A method according to claim 8, wherein said second enzyme is sulfurylase

**10.** A method according to claim 8, wherein said third enzyme is luciferase.

**11.** A method according to any of claims 1 to 10, wherein said microspheres are randomly distributed on said discrete sites.

**12.** A method of sequencing a target nucleic acid comprising a first domain and an adjacent second domain, said second domain comprising a plurality of target positions, said method comprising:

a) providing a hybridization complex comprising said target sequence, an adaptor probe and a capture probe, wherein said adaptor probe is attached to said target sequence and is a binding partner of said capture probe, and wherein said capture probe is covalently attached to a microsphere on a surface of a substrate, wherein said substrate is an optical fiber bundle; and
b) determining the identity of a plurality of said target positions by pyrosequencing to detect release of pyrophosphate (PPi),

wherein said method comprises the use of secondary enzymes to detect PPi by generating a chemiluminescent signal, and wherein said secondary enzymes are attached to beads, said beads being associated with the surface of said optical fiber bundle.

**13.** A method according to claim 12, wherein said beads are microspheres.

**14.** A method according to claim 12 or claim 13, wherein said capture probe is a sequencing primer.

**15.** A method according to claim 12 or claim 13, wherein said determining comprises:

a) providing a sequencing primer hybridized to said second domain;
b) extending said primer by the addition of a first nucleotide to the first detection position using a first enzyme to form an extended primer;
c) detecting the release of pyrophosphate (PPi) to determine the type of said first nucleotide added onto said primer;
d) extending said primer by the addition of a second nucleotide to a second detection position using said enzyme; and
e) detecting the release of a pyrophosphate (PPi) to determine the type of said second nucleotide added onto said primer.

**16.** The method according to claim 12 or claim 13, wherein said PPi is detected by a method comprising:

a) contacting said PPi with a second enzyme that converts said PPi into ATP; and
b) detecting said ATP using a third enzyme.

**17.** A method according to claim 16 wherein said second enzyme is sulfurylase.

**18.** A method according to claim 16, wherein said third enzyme is luciferase.

**19.** A method according to claim 12 or claim 13, wherein said determining comprises:

a) providing a sequence primer hybridized to said second domain;
b) extending said primer by the addition of a first protected nucleotide using a first enzyme to form an extended primer;
c) determining the identification of first said protected nucleotide;
d) removing the protection group;
e) adding a second protected nucleotide using said first enzyme; and
f) determining the identification of said second protected nucleotide.

**20.** A method according to any of claims 12 to 19, comprising subpopulations of microspheres, each of said microspheres in said subpopulation comprising the same capture probe.

**21.** A method according to any of claims 12 to 20, wherein said capture probe also serves as said sequencing primer.

**22.** A method according to any of claims 12 to 21, wherein said substrate comprises discrete sites and said microspheres are randomly distributed on said sites.

**23.** A method according to claim 22, wherein said discrete sites are wells, and said microspheres are randomly distributed in said wells.

**24.** A kit for nucleic acid sequencing comprising:

a) a composition comprising:

i) a substrate with a surface comprising discrete sites, wherein said substrate is an optical fiber bundle; and
ii) a population of microspheres distributed on said sites; wherein said microspheres comprise capture probes for hybridizing to target nucleic acid sequences;

b) a extension enzyme for enzymatically extending an oligonucleotide chain;
c) dNTPs;
d) a second enzyme for the conversion of pyrophosphate (PPi) to ATP; and
e) a third enzyme for the detection of ATP

wherein said second and third enzymes are attached to beads distributed on said sites of said optical fiber bundle.

**25.** A kit according to claim 24, wherein said beads are said microspheres.

**26.** A kit according to claim 24 or 25, wherein said discrete sites are wells, and said microspheres are randomly distributed in said wells.

## Patentansprüche

**1.** Verfahren zum Sequenzieren einer Vielzahl von Target-Nukleinsäuren, wobei jede eine erste Domäne und eine benachbarte zweite Domäne umfasst, wobei die zweite Domäne eine Vielzahl von Nachweispositionen umfasst, wobei das Verfahren Folgendes umfasst:

a) die Bereitstellung einer Vielzahl von Hybridisierungskomplexen, wobei jeder Folgendes umfasst: eine Target-Sequenz und einen Sequenzierungsprimer, der die erste Domäne der Target-Sequenz hybridisiert, wobei die Hybridisierungskomplexe an Mikrosphären angebunden sind, wobei die Mikrosphären mit diskreten individuellem Stellen auf der Oberfläche eines Substrats assoziiert sind, wobei das Substrat ein optisches Faserbündel ist;
b) die Verlängerung von jedem der Primer durch das Zufügen eines ersten Nukleotids zur ersten Nachweisposition unter Verwendung eines ersten Enzyms zur Bildung eines verlängerten Primers;
und
c) den Nachweis der Freisetzung von Pyrophosphat (PPi) zur Bestimmung des den Primern zugefügten Typs des ersten Nukleotids;

wobei das Verfahren die Verwendung von sekundären Enzymen zur Herbeiführung eines Chemilumineszenzsignals umfasst, wobei die sekundären Enzyme an die Mikrosphären angebunden sind.

**2.** Verfahren nach Anspruch 1, wobei die Sequenzierungsprimer an die Oberfläche angebunden sind.

**3.** Verfahren nach Anspruch 1, wobei jeder der Hybridisierungskomplexe eine Target-Sequenz umfasst, wobei der Sequenzierungsprimer und eine Capture-Sonde zum Hybridisieren an die Target-Sequenz kovalent an die Oberfläche angebunden sind.

**4.** Verfahren nach Anspruch 1, wobei jeder der Hybridisierungskomplexe eine Target-Sequenz umfasst, wobei der Sequenzierungsprimer, eine Adapter-Sonde und eine Capture-Sonde zum Hybridisieren an die Target-Sequenz kovalent an die Oberfläche angebunden sind.

**5.** Verfahren nach Anspruch 3 oder 4, wobei die Mikrosphären Subpopulationen von Mikrosphären umfassen, wobei jede Mikrosphäre in einer Subpopulation die gleiche Capture-Sonde enthält.

**6.** Verfahren nach Anspruch 3, 4 oder 5, wobei die Capture-Sonde auch als der Sequenzierungsprimer dient.

**7.** Verfahren nach Anspruch 1, ferner umfassend:

d) die Verlängerung des verlängerten Primers durch das Zufügen eines zweiten Nukleotids zur zweiten Nachweisposition unter Verwendung des Enzyms; und
e) den Nachweis der Freisetzung von Pyrophosphat (PPi) zur Bestimmung des den Primern zugefügten Typs des zweiten Nukleotids.

**8.** Verfahren nach Anspruch 1, wobei Schritt (c) Folgendes umfasst:

i) das Inkontaktbringen des PPi mit einem zweiten Enzym, welches das PPi in ATP umwandelt; und
ii) den Nachweis des ATPs unter Verwendung eines dritten Enzyms.

**9.** Verfahren nach Anspruch 8, wobei das zweite Enzym Sulfurylase ist.

**10.** Verfahren nach Anspruch 8, wobei das dritte Enzym Luciferase ist.

**11.** Verfahren nach einem der Ansprüche 1 bis 10, wobei die Mikrosphären auf den diskreten Stellen zufällig verteilt sind.

**12.** Verfahren zum Sequenzieren einer Target-Nukleinsäure, umfassend eine erste Domäne und eine benachbarte zweite Domäne, wobei die zweite Domäne eine Vielzahl von Target-Positionen umfasst, wobei das Verfahren Folgendes umfasst:

a) die Bereitstellung eines Hybridisierungskomplexes, umfassend die Target-Sequenz, eine Adapter-Sonde und eine Capture-Sonde, wobei die Adapter-Sonde an die Target-Sequenz angebunden ist und ein Bindungspartner der Capture-Sonde ist, und wobei die Capture-Sonde kovalent an eine Mikrosphäre auf einer Oberfläche eines Substrats angebunden ist, wobei das Substrat ein optisches Faserbündel ist; und
b) die Bestimmung der Identität einer Vielzahl der Target-Positionen durch Pyrosequenzierung zum Nachweis der Freisetzung von Pyrophosphat (PPi),

wobei das Verfahren die Verwendung von sekundären Enzymen zum Nachweis von PPi durch Herbeiführung eines Chemilumineszenzsignals umfasst, und wobei die sekundären Enzyme an Beads angebunden sind, wobei die Beads mit der Oberfläche des optischen Faserbündels assoziiert sind.

**13.** Verfahren nach Anspruch 12, wobei die Beads Mikrosphären sind.

**14.** Verfahren nach Anspruch 12 oder 13, wobei die Capture-Sonde ein Sequenzierungsprimer ist.

**15.** Verfahren nach Anspruch 12 oder 13, wobei die Bestimmung Folgendes umfasst:

a) die Bereitstellung eines an die zweite Domäne hybridisierten Sequenzierungsprimers;
b) die Verlängerung des Primers durch das Zufügen eines ersten Nukleotids zur ersten Nachweisposition unter Verwendung eines ersten Enzyms zur Bildung eines verlängerten Primers;
c) den Nachweis der Freisetzung von Pyrophosphat (PPi) zur Bestimmung des dem Primer zugefügten Typs des ersten Nukleotids;
d) die Verlängerung des Primers durch das Zufügen eines zweiten Nukleotids zu einer zweiten Nachweisposition unter Verwendung des Enzyms; und
e) den Nachweis der Freisetzung eines Pyrophosphats (PPi) zur Bestimmung des dem Primer zugefügten Typs des zweiten Nukleotids.

**16.** Verfahren nach Anspruch 12 oder 13, wobei das PPi durch ein Verfahren nachgewiesen wird, umfassend:

a) das Inkontaktbringen des PPi mit einem zweiten Enzym, welches das PPi in ATP umwandelt; und
b) den Nachweis des ATPs unter Verwendung eines dritten Enzyms.

**17.** Verfahren nach Anspruch 16, wobei das zweite Enzym Sulfurylase ist.

**18.** Verfahren nach Anspruch 16, wobei das dritte Enzym Luciferase ist.

**19.** Verfahren nach Anspruch 12 oder 13, wobei die Bestimmung Folgendes umfasst:

a) die Bereitstellung eines an die zweite Domäne hybridisierten Sequenz-Primers;
b) die Verlängerung des Primers durch das Zufügen eines ersten geschützten Nukleotids unter Verwendung eines ersten Enzyms zur Bildung eines verlängerten Primers;
c) die Bestimmung der Identifikation des ersten geschützten Nukleotids;
d) die Entfernung der Schutzgruppe;
e) das Zufügen eines zweiten geschützten Nukleotids unter Verwendung des ersten Enzyms; und
f) die Bestimmung der Identifikation des zweiten geschützten Nukleotids.

**20.** Verfahren nach einem der Ansprüche 12 bis 19, umfassend Subpopulationen von Mikrosphären, wobei jede der Mikrosphären in der Subpopulation die gleiche Capture-Sonde umfasst.

**21.** Verfahren nach einem der Ansprüche 12 bis 20, wobei die Capture-Sonde auch als der Sequenzierungsprimer dient.

**22.** Verfahren nach einem der Ansprüche 12 bis 21, wobei das Substrat diskrete Stellen umfasst und die Mikrosphären auf den Stellen zufällig verteilt sind.

**23.** Verfahren nach Anspruch 22, wobei die diskreten Stellen Wells sind und die Mikrosphären in den Wells zufällig verteilt sind.

**24.** Kit zur Nukleinsäure-Sequenzierung, umfassend:

a) eine Zusammensetzung, umfassend:

i) ein Substrat mit einer Oberfläche, die diskrete Stellen umfasst, wobei das Substrat ein optisches Faserbündel ist; und
ii) eine Population von auf den Stellen verteilten Mikrosphären; wobei die Mikrosphären Capture-Sonden zum Hybridisieren an Target-Nukleinsäuresequenzen umfassen;

b) ein Verlängerungsenzym zur enzymatischen Verlängerung einer Oligonukleotidkette;
c) dNTPs;
d) ein zweites Enzym zur Umwandlung von Pyrophosphat (PPi) in ATP; und

e) ein drittes Enzym zum Nachweis von ATP,

wobei die zweiten und dritten Enzyme an Beads angebunden sind, die auf den Stellen des optischen Faserbündels verteilt sind.

**25.** Kit nach Anspruch 24, wobei die Beads Mikrosphären sind.

**26.** Kit nach Anspruch 24 oder 25, wobei die diskreten Stellen Wells sind, und die Mikrosphären in den Wells zufällig verteilt sind.

**Revendications**

**1.** Procédé de séquençage d'une pluralité d'acides nucléiques cibles, comprenant chacun un premier domaine et un deuxième domaine adjacent, ledit deuxième domaine comprenant une pluralité de positions de détection, ledit procédé comprenant les étapes suivantes :

a) mise à disposition d'une pluralité de complexes d'hybridation comprenant chacun une séquence cible et une amorce de séquençage qui s'hybride au premier domaine de ladite séquence cible, lesdits complexes d'hybridation se fixant à des microsphères, lesdites microsphères étant associées à des sites individuels distincts sur la surface du substrat, dans lequel ledit substrat est un faisceau de fibres optiques ;
b) élongation de chacune desdites amorces en ajoutant un premier nucléotide en première position de détection, à l'aide d'une première enzyme, afin de former une amorce allongée ; et
c) détection du départ de pyrophosphate (PPi) afin de déterminer le type dudit premier nucléotide ajouté sur lesdites amorces ;

dans lequel ledit procédé comprend l'utilisation d'enzymes secondaires pour générer un signal chimiluminescent, dans lequel lesdites enzymes secondaires se fixent auxdites microsphères.

**2.** Procédé selon la revendication 1, dans lequel lesdites amorces de séquençage se fixent à ladite surface.

**3.** Procédé selon la revendication 1, dans lequel chacun desdits complexes d'hybridation comprennent une séquence cible, ladite amorce de séquençage et une sonde de capture permettant l'hybridation à ladite séquence cible fixée par liaison covalente à ladite surface.

**4.** Procédé selon la revendication 1, dans lequel chacun desdits complexes d'hybridation comprennent une séquence cible, ladite amorce de séquençage, une sonde adaptatrice et une sonde de capture permettant l'hybridation à ladite séquence cible fixée par liaison covalente à ladite surface.

**5.** Procédé selon la revendication 3 ou 4, dans lequel lesdites microsphères comprennent des sous-populations de microsphères, dans lequel chaque microsphère dans une sous-population contient la même sonde de capture

**6.** Procédé selon la revendication 3, 4 ou 5, dans lequel ladite sonde de capture sert également de dite amorce de séquençage.

**7.** Procédé selon la revendication 1, comprenant en outre les étapes suivantes :

d) élongation de ladite amorce allongée en ajoutant un deuxième nucléotide en deuxième position de détection, à l'aide de ladite enzyme ; et
e) détection du départ de pyrophosphate (PPi) afin de déterminer le type dudit deuxième nucléotide ajouté sur lesdites amorces.

**8.** Procédé selon la revendication 1, dans lequel l'étape c) comprend les étapes suivantes :

i) mise en contact dudit PPi avec une deuxième enzyme convertissant ledit PPi en ATP ; et
ii) détection dudit ATP à l'aide d'une troisième enzyme.

**9.** Procédé selon la revendication 8, dans lequel ladite deuxième enzyme est la sulfurylase.

**10.** Procédé selon la revendication 8, dans lequel ladite troisième enzyme est la luciférase.

**11.** Procédé selon l'une quelconque des revendications 1 à 10, dans lequel lesdites microsphères sont réparties de manière aléatoire sur lesdits sites distincts.

**12.** Procédé de séquençage d'un acide nucléique cible, comprenant un premier domaine et un deuxième domaine adjacent, ledit deuxième domaine comprenant une pluralité de positions cibles, ledit procédé comprenant les étapes suivantes :

a) mise à disposition d'un complexe d'hybridation comprenant ladite séquence cible, une sonde adaptatrice et une sonde de capture, dans lequel ladite sonde adaptatrice se fixe à ladite séquence cible et est un partenaire de liaison de ladite sonde de capture, et dans lequel ladite sonde de capture se fixe par liaison covalente à une microsphère sur une surface d'un substrat, dans lequel ledit substrat est un faisceau de fibres optiques ; et
b) détermination de l'identité d'une pluralité desdites positions cibles par pyroséquençage afin de détecter le départ de pyrophosphate (PPi),

dans lequel ledit procédé comprend l'utilisation d'enzymes secondaires pour détecter le PPi en générant un signal chimiluminescent, et dans lequel lesdites enzymes secondaires se fixent à des billes, lesdites billes étant associées à la surface dudit faisceau de fibres optiques.

**13.** Procédé selon la revendication 12, dans lequel lesdites billes sont des microsphères.

**14.** Procédé selon la revendication 12 ou 13, dans lequel ladite sonde de capture est une amorce de séquençage.

**15.** Procédé selon la revendication 12 ou 13, dans lequel ladite étape de détermination comprend les étapes suivantes :

a) mise à disposition d'une amorce de séquençage hybridée audit deuxième domaine ;
b) élongation de ladite amorce en ajoutant un premier nucléotide en première position de détection, à l'aide d'une première enzyme, afin de former une amorce allongée ;
c) détection du départ de pyrophosphate (PPi) afin de déterminer le type dudit premier nucléotide ajouté sur ladite amorce ;
d) élongation de ladite amorce en ajoutant un deuxième nucléotide en deuxième position de détection, à l'aide de ladite enzyme ; et
e) détection du départ de pyrophosphate (PPi) afin de déterminer le type dudit deuxième nucléotide ajouté sur ladite amorce.

**16.** Procédé selon la revendication 12 ou 13, dans lequel ledit PPi est détecté par un procédé comprenant les étapes suivantes :

a) mise en contact dudit PPi avec une deuxième enzyme convertissant ledit PPi en
ATP ; et
b) détection dudit ATP à l'aide d'une troisième enzyme.

**17.** Procédé selon la revendication 16, dans lequel ladite deuxième enzyme est la sulfurylase.

**18.** Procédé selon la revendication 16, dans lequel ladite troisième enzyme est la luciférase.

**19.** Procédé selon la revendication 12 ou 13, dans lequel ladite étape de détermination comprend les étapes suivantes :

a) mise à disposition d'une amorce de séquençage hybridée audit deuxième domaine ;
b) élongation de ladite amorce en ajoutant un premier nucléotide protégé, à l'aide d'une première enzyme, afin de former une amorce allongée ;
c) détermination de l'identification dudit premier nucléotide protégé ;
d) suppression du groupe protecteur ;
e) ajout d'un deuxième nucléotide protégé à l'aide de ladite première enzyme ; et
f) détermination de l'identification dudit deuxième nucléotide protégé.

**20.** Procédé selon l'une quelconque des revendications 12 à 19, comprenant des sous-populations de microsphères, chacune desdites microsphères dans ladite sous-population comprenant la même sonde de capture.

**21.** Procédé selon l'une quelconque des revendications 12 à 20, dans lequel ladite sonde de capture sert également de dite amorce de séquençage.

**22.** Procédé selon l'une quelconque des revendications 12 à 21, dans lequel ledit substrat comprend des sites distincts et lesdites microsphères sont réparties de manière aléatoire sur lesdits sites.

**23.** Procédé selon la revendication 22, dans lequel lesdits sites distincts sont des puits et lesdites microsphères sont réparties de manière aléatoire dans lesdits puits.

**24.** Kit de séquençage d'acide nucléique, comprenant :

a) une composition comprenant :

i) un substrat avec une surface composée de sites distincts, dans lequel ledit substrat est un faisceau de fibres optiques ; et
ii) une population de microsphères réparties sur lesdits sites ; dans lequel lesdites microsphères comprennent des sondes de capture permettant l'hybridation à des séquences d'acide nucléiques cibles ;

b) une enzyme d'élongation permettant l'élongation d'une chaîne oligonucléotidique ;
c) des dNTP ;
d) une deuxième enzyme permettant la conversion du pyrophosphate (PPi) en ATP ; et
e) une troisième enzyme permettant la détection d'ATP,

dans lequel lesdites deuxième et troisième enzymes se fixent à des billes réparties sur lesdits sites dudit faisceau de fibres optiques.

**25.** Kit selon la revendication 24, dans lequel lesdites billes sont lesdites microsphères.

**26.** Kit selon la revendication 24 ou 25, dans lequel lesdits sites distincts sont des puits et lesdites microsphères sont réparties de manière aléatoire dans lesdits puits.

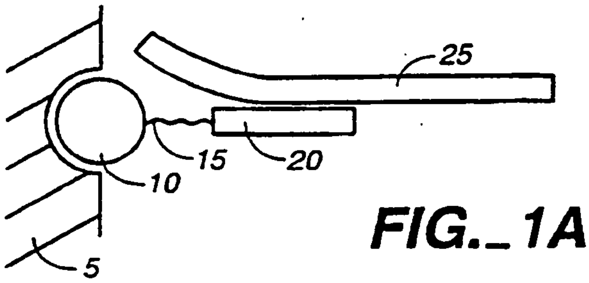


FIG._1A

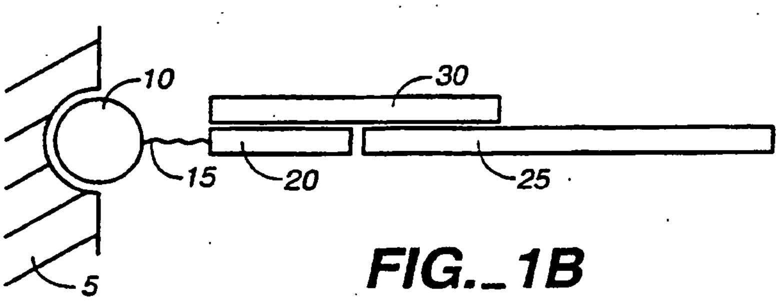


FIG._1B

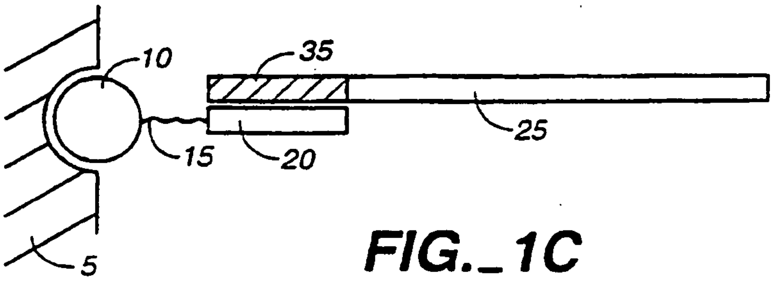


FIG._1C

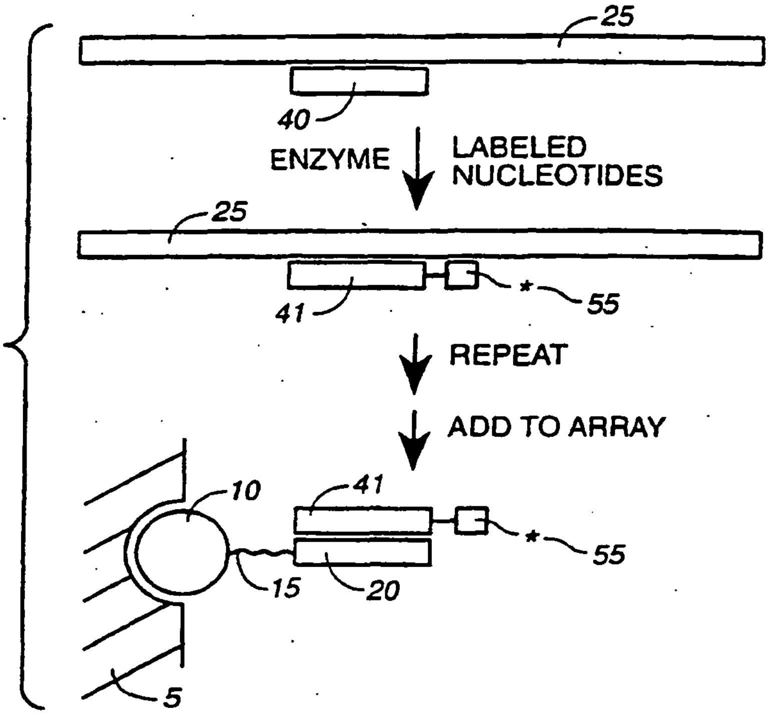
25
40
ENZYME
LABELED NUCLEOTIDES
25
41
55
REPEAT
ADD TO ARRAY
10
41
55
15
20
5

FIG._2A

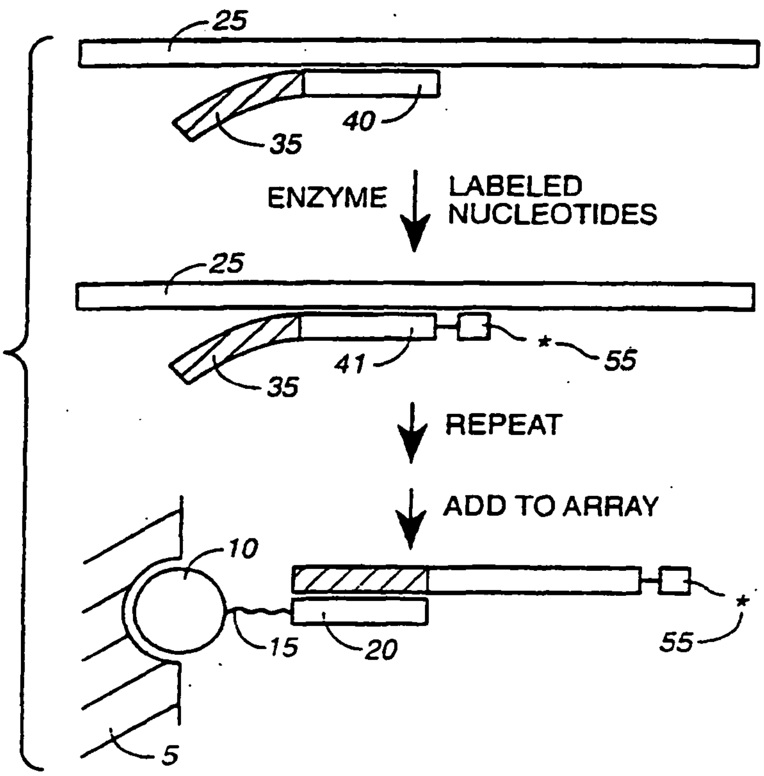
25
35
40
ENZYME
LABELED NUCLEOTIDES
25
41
35
55
REPEAT
ADD TO ARRAY
10
15
20
55
5

FIG._2B

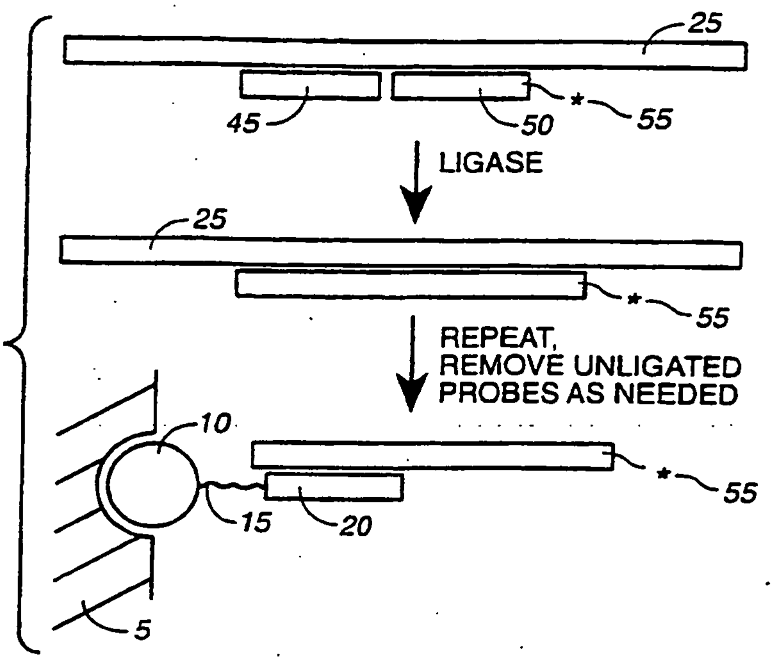
25
45
50
55
LIGASE
25
55
REPEAT,
REMOVE UNLIGATED
PROBES AS NEEDED
10
55
15
20
5

FIG._3A

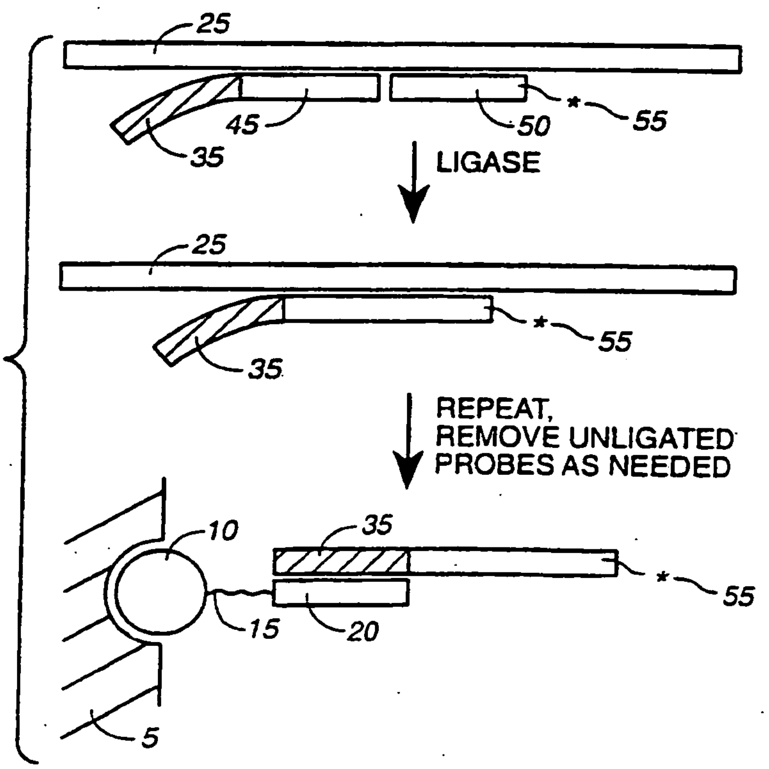
25
45
50
55
35
LIGASE
25
55
35
REPEAT,
REMOVE UNLIGATED
PROBES AS NEEDED
10
35
55
15
20
5

FIG._3B

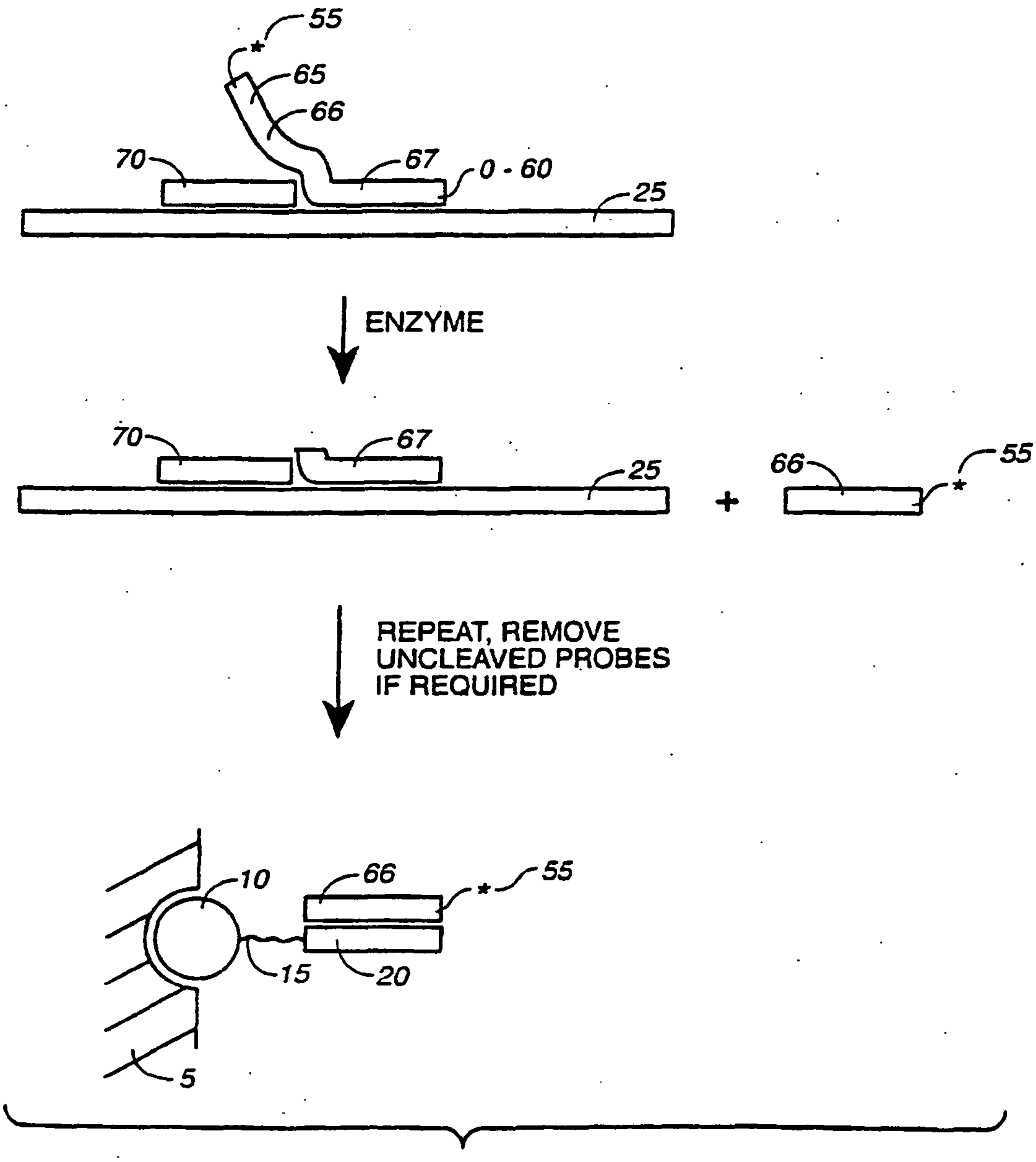
55
65
66
70
67
0 - 60
25
ENZYME
70
67
25
66
55
REPEAT, REMOVE
UNCLEAVED PROBES
IF REQUIRED
10
66
55
15
20
5

FIG._4

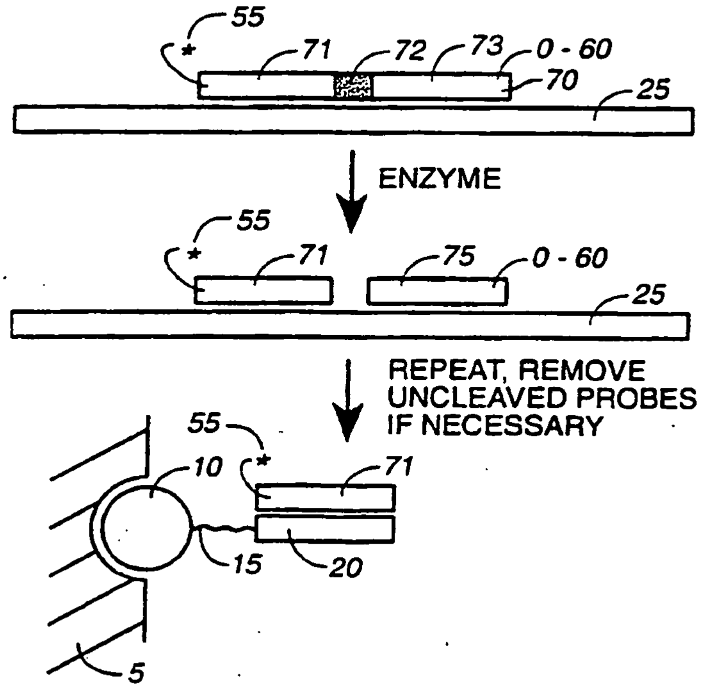
55
71
72
73
0 - 60
70
25
ENZYME
55
71
75
0 - 60
25
REPEAT, REMOVE
UNCLEAVED PROBES
IF NECESSARY
55
10
71
15
20
5

FIG._5A

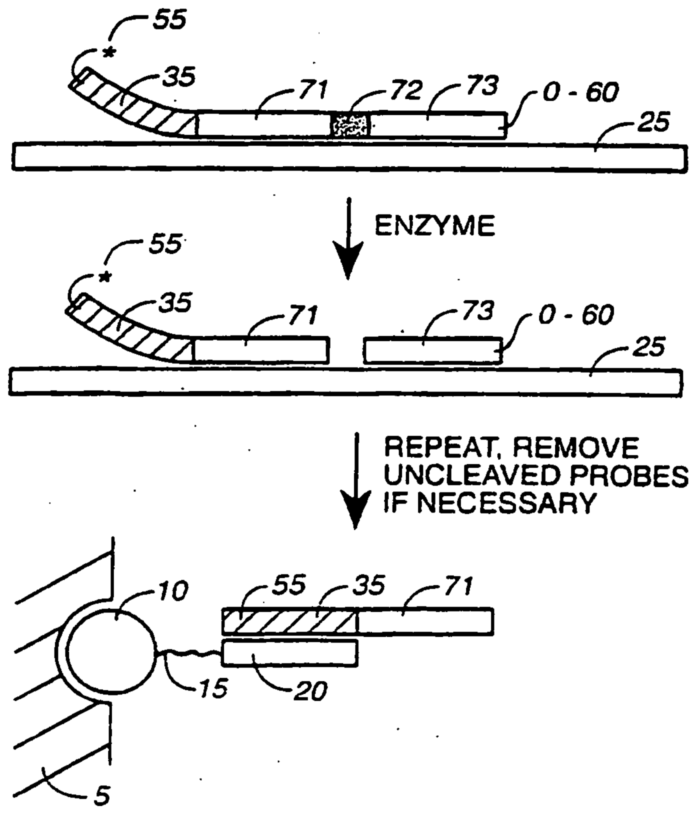
55
35
71
72
73
0 - 60
25
ENZYME
55
35
71
73
0 - 60
25
REPEAT, REMOVE
UNCLEAVED PROBES
IF NECESSARY
10
55
35
71
15
20
5

FIG._5B

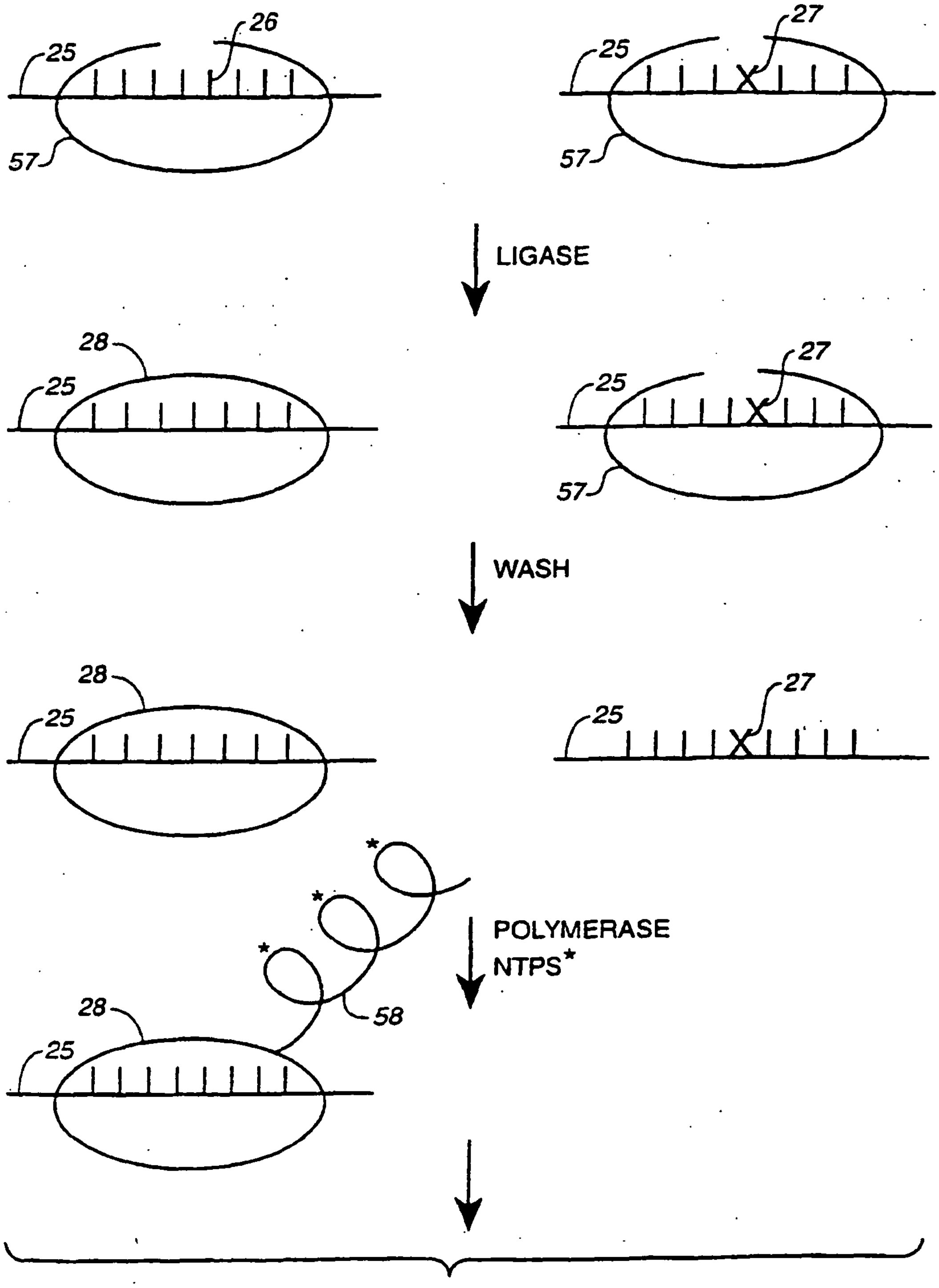
26
25
57
27
25
57
LIGASE
28
25
27
25
57
WASH
28
25
27
25
POLYMERASE
NTPS*
28
25
58

FIG._6A

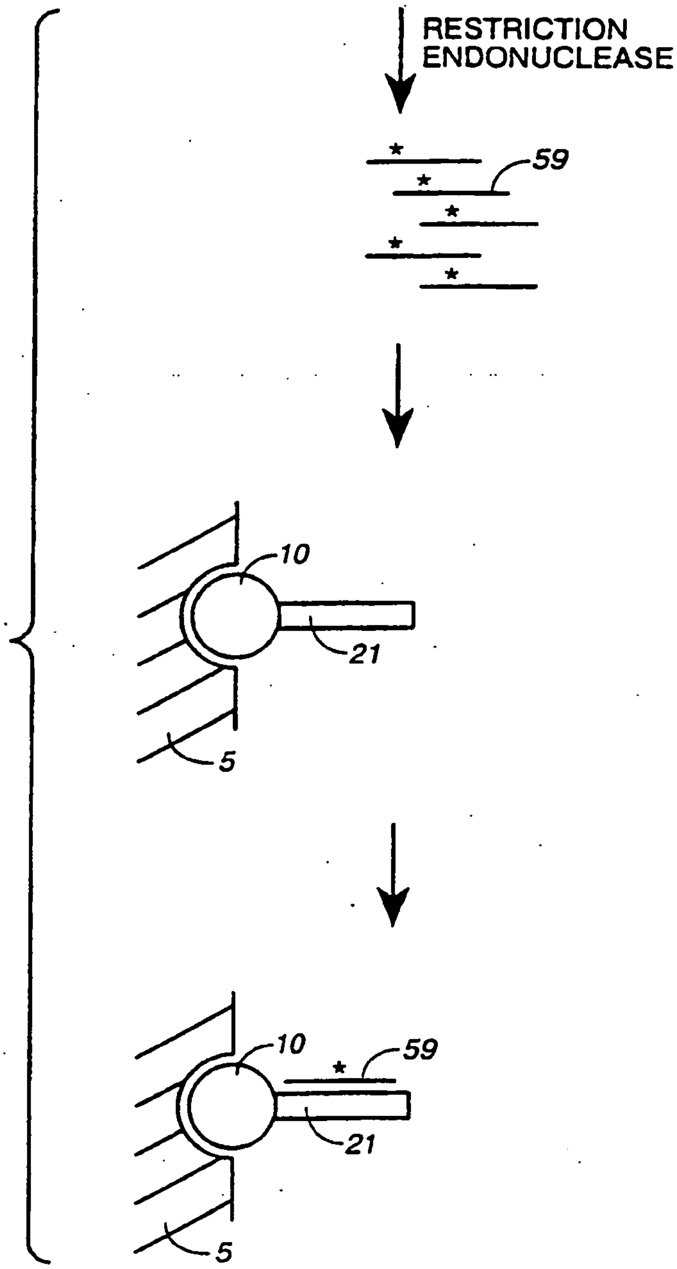


FIG._6B

FIG._7A

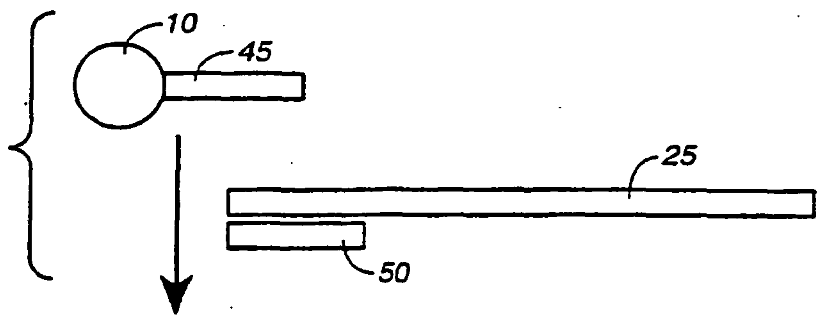


FIG._7B

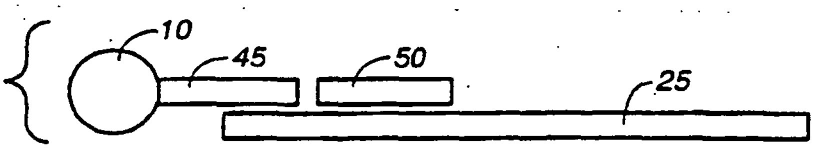


FIG._7C

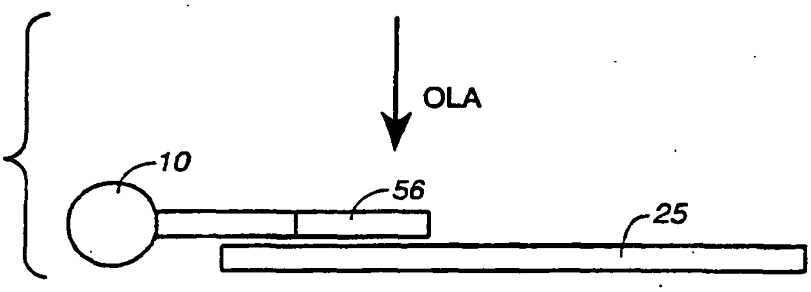


FIG._7D

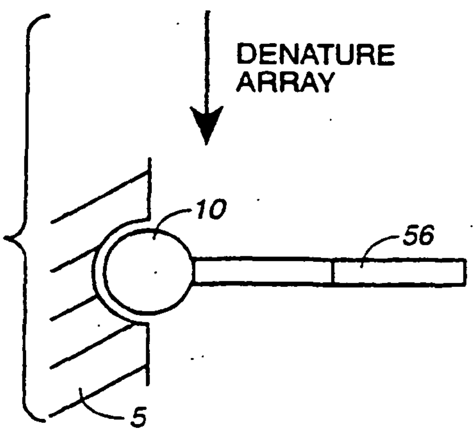

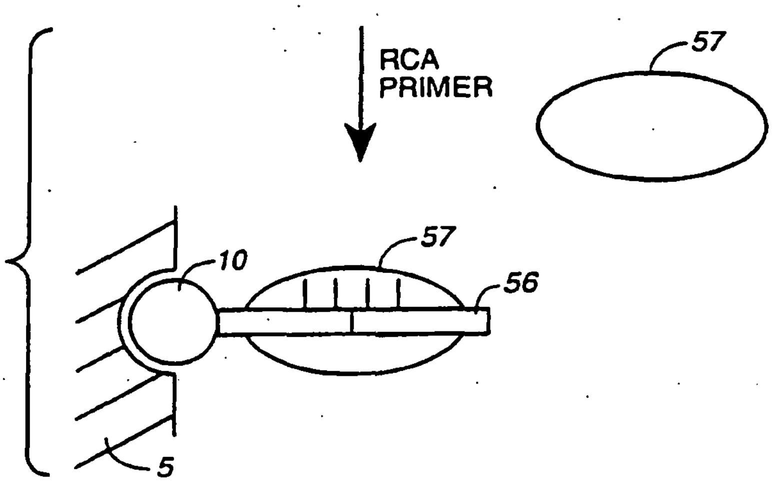


FIG._7E

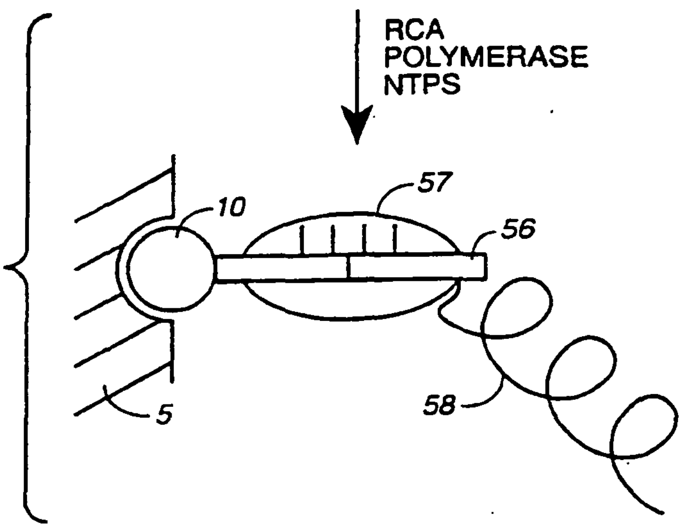


FIG._7F

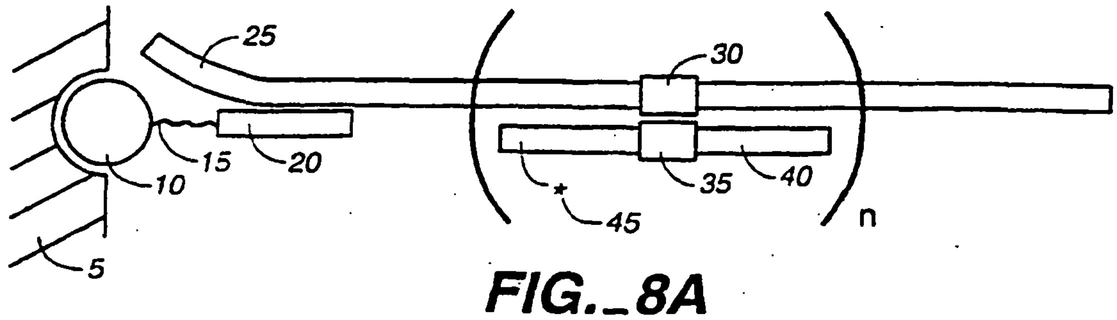


FIG._8A

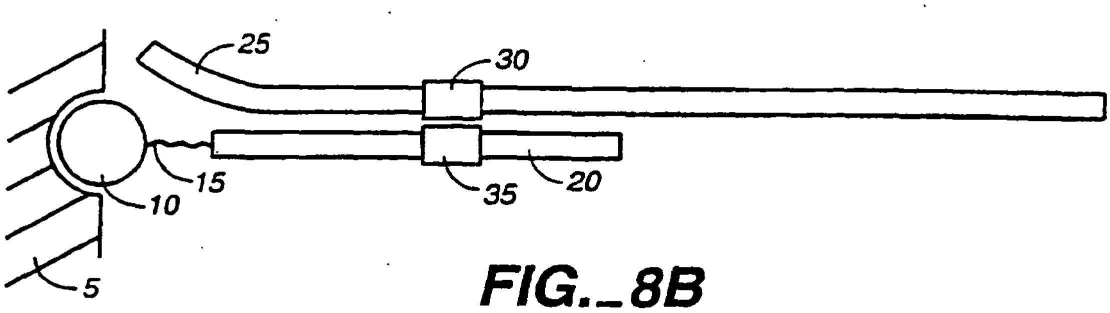


FIG._8B

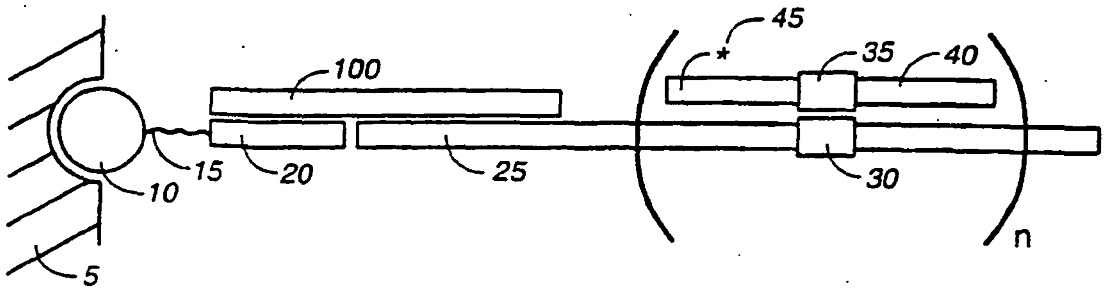


FIG._8C

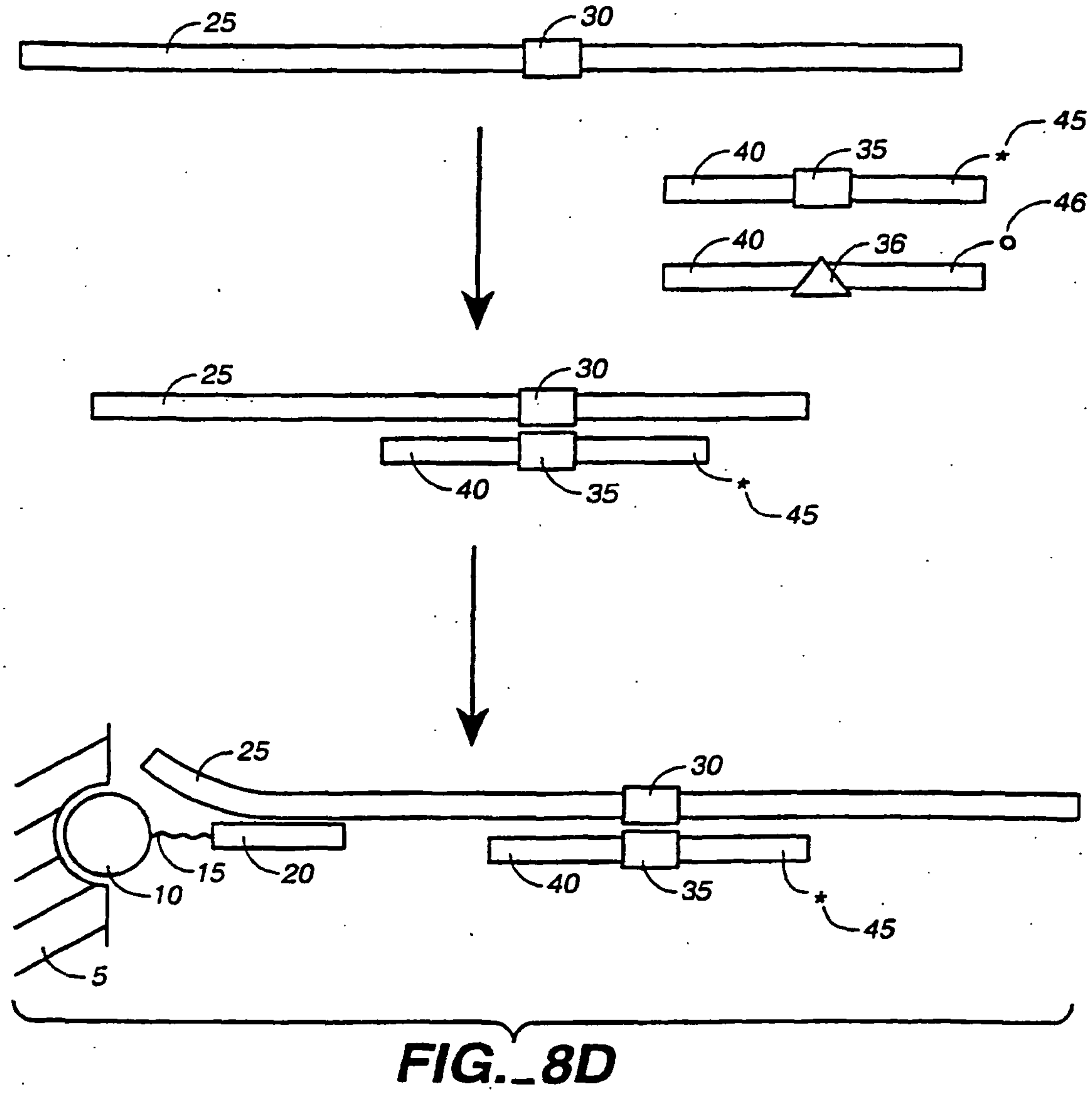
25
30
45
40
35
46
40
36
25
30
40
35
45
25
30
15
20
10
40
35
45
5

FIG._8D

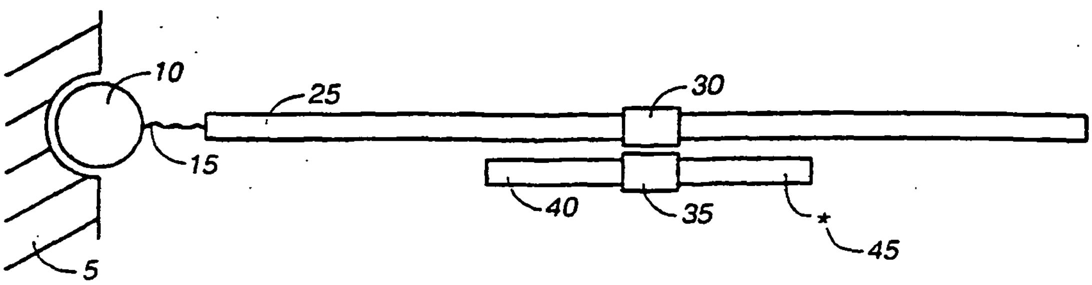
10
25
30
15
40
35
45
5

FIG._8E

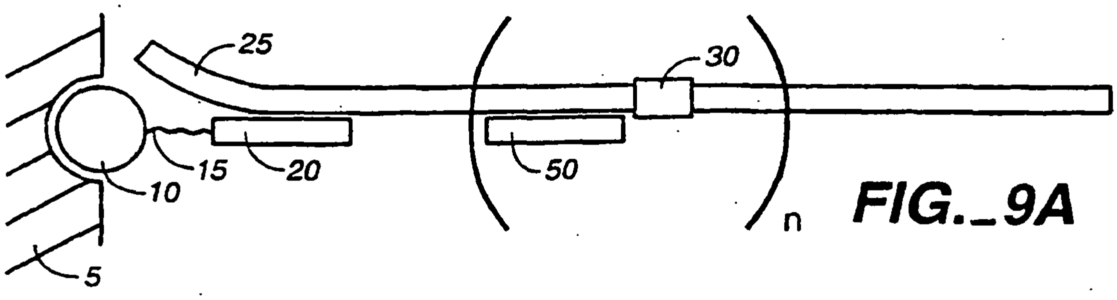


FIG._9A

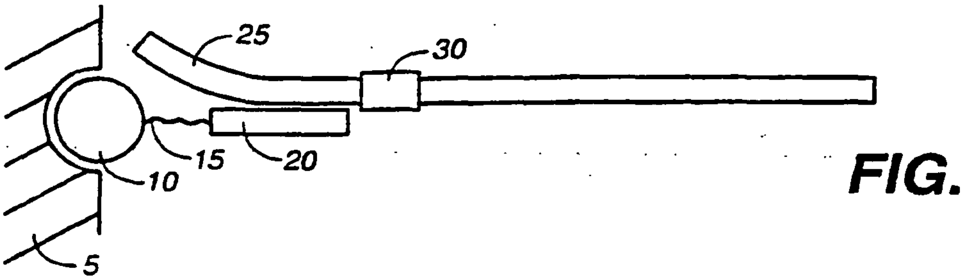


FIG._9B

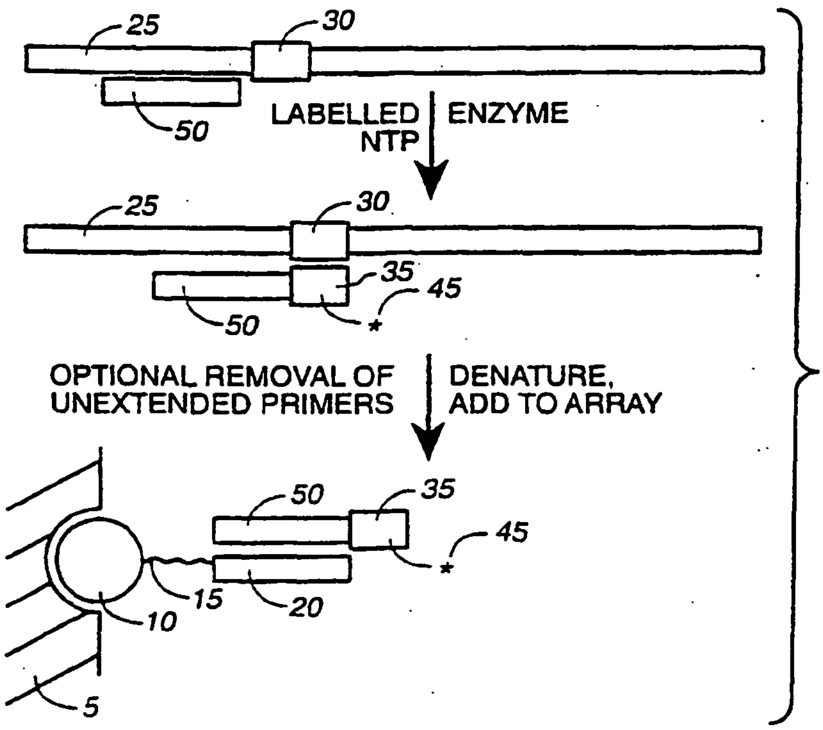


FIG._9C

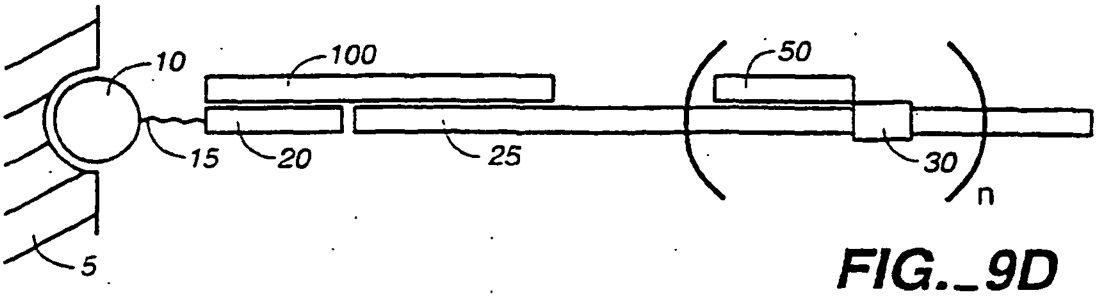


FIG._9D

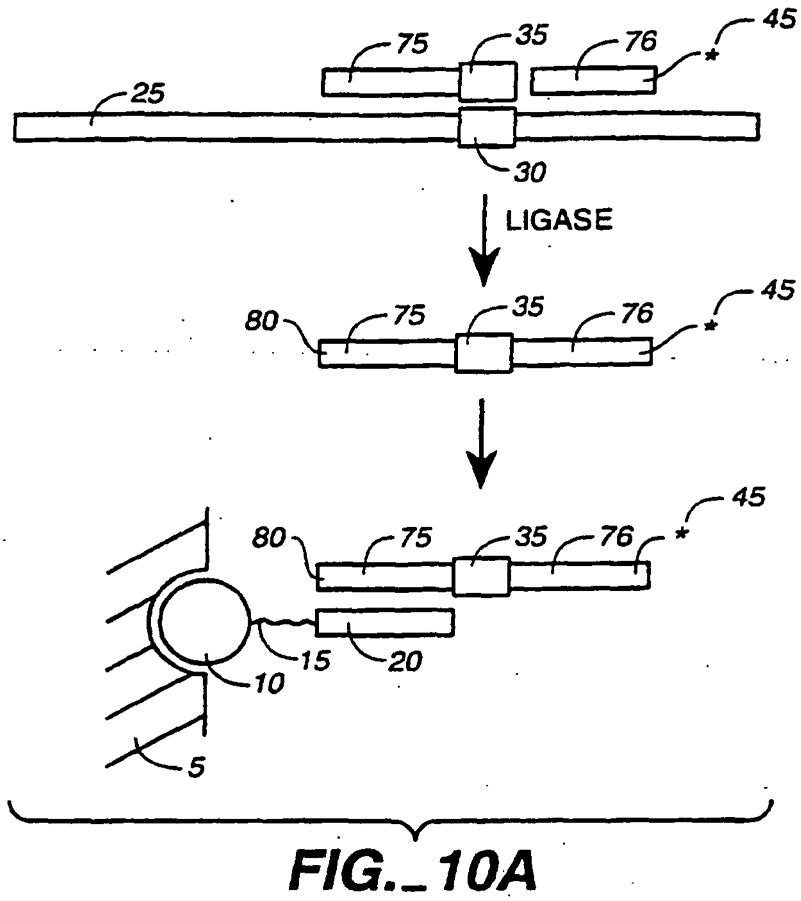


FIG._10A

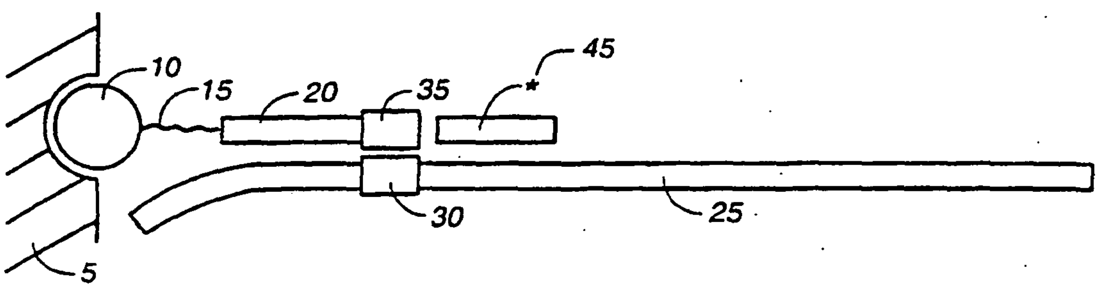


FIG._10B

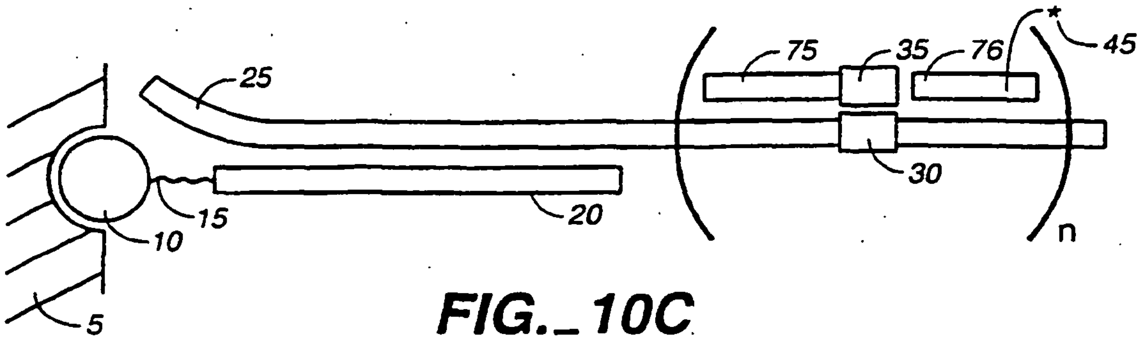


FIG._10C

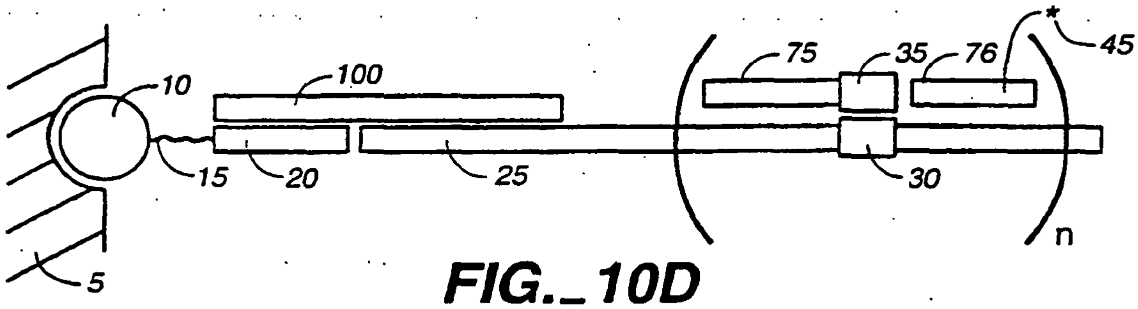


FIG._10D

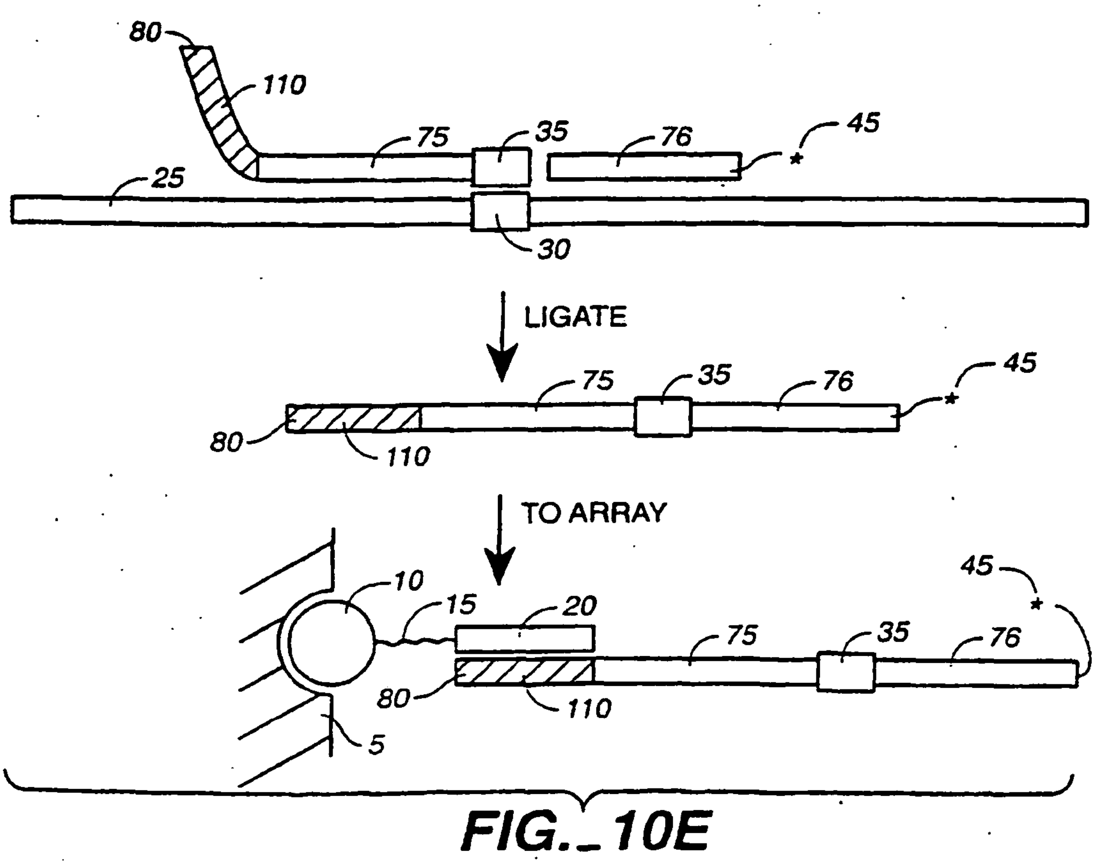


FIG._10E

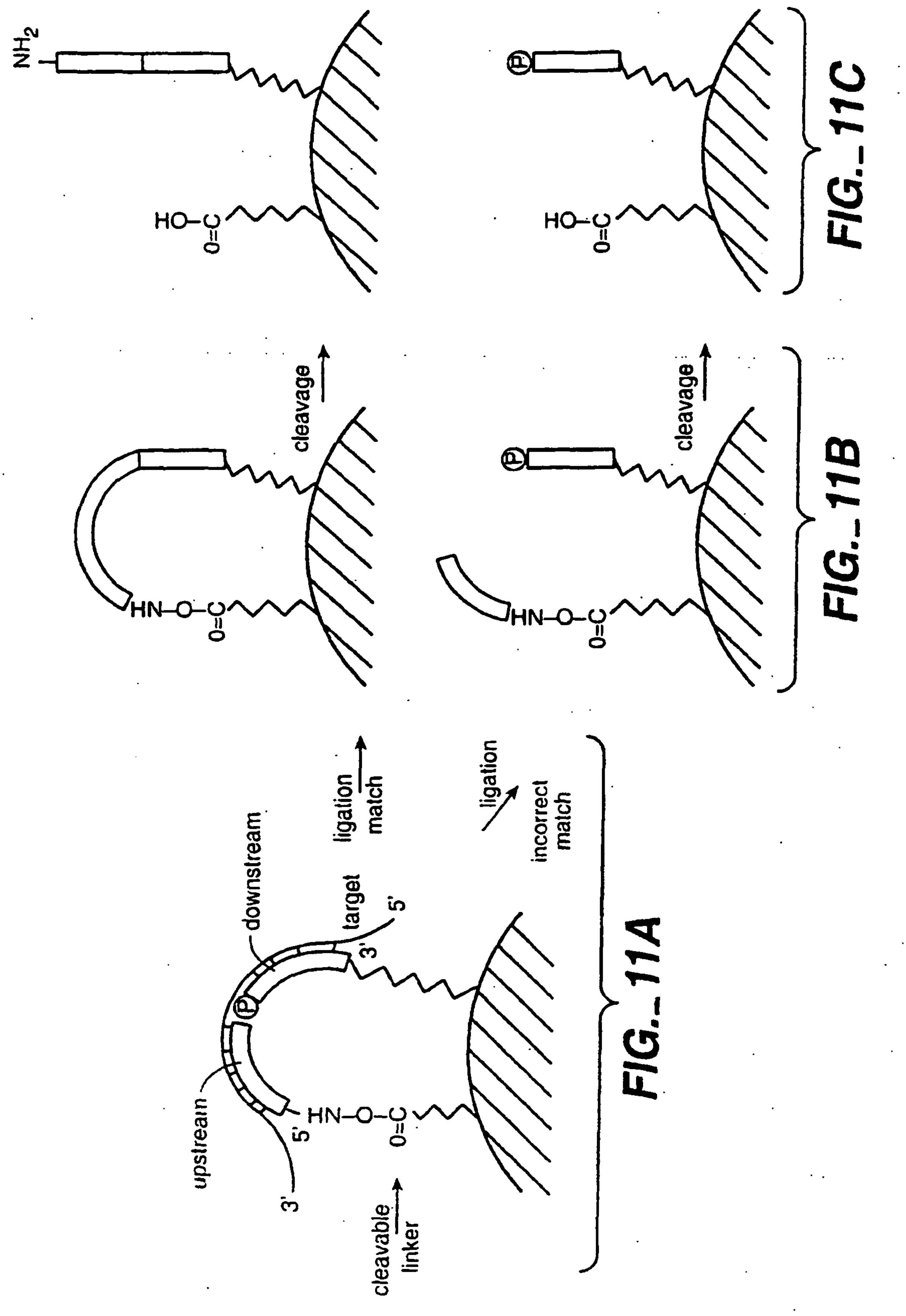


FIG._11A

FIG._11B

FIG._11C

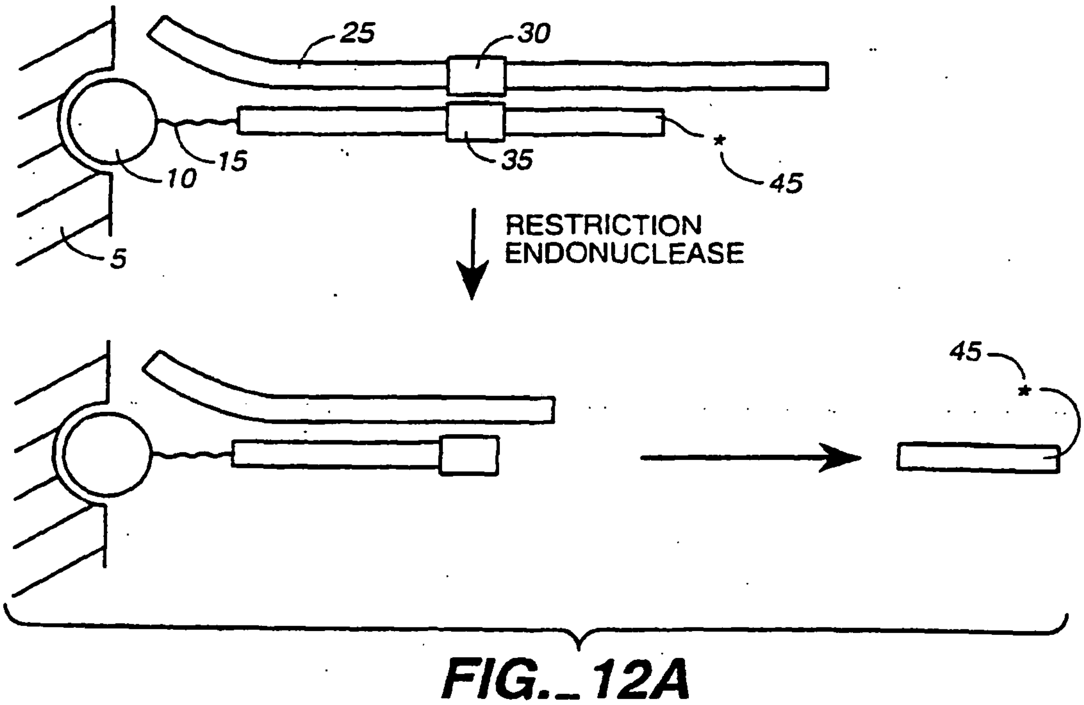


FIG._12A

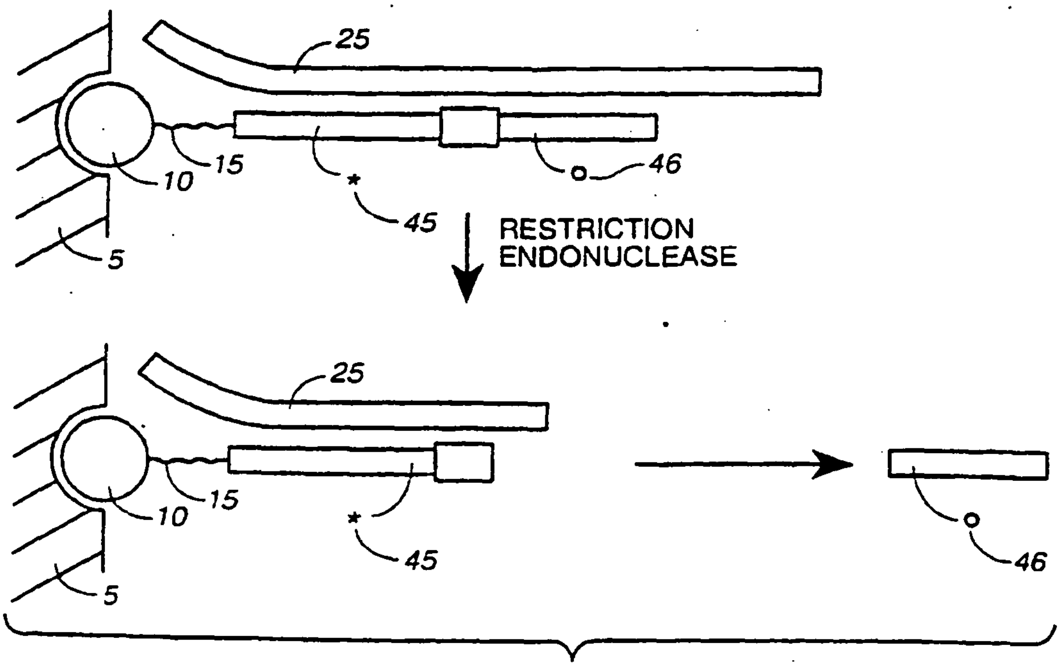


FIG._12B

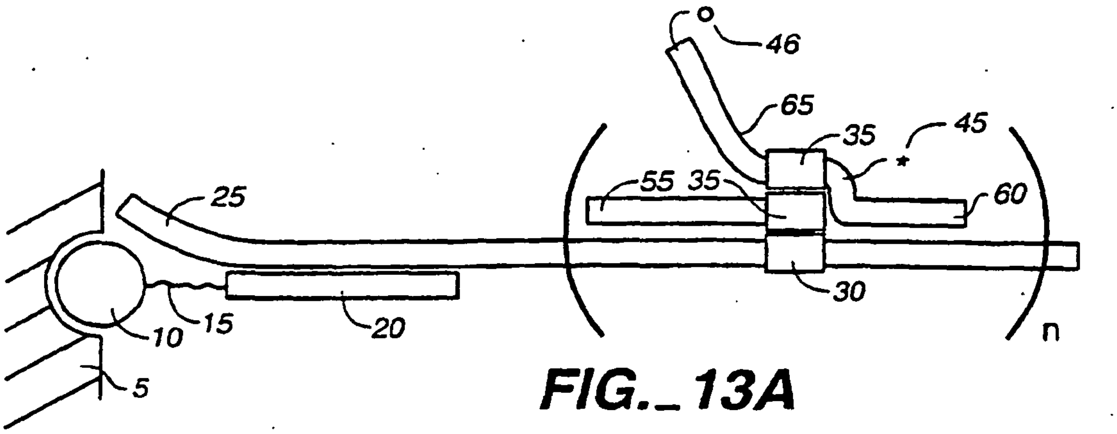


FIG._13A

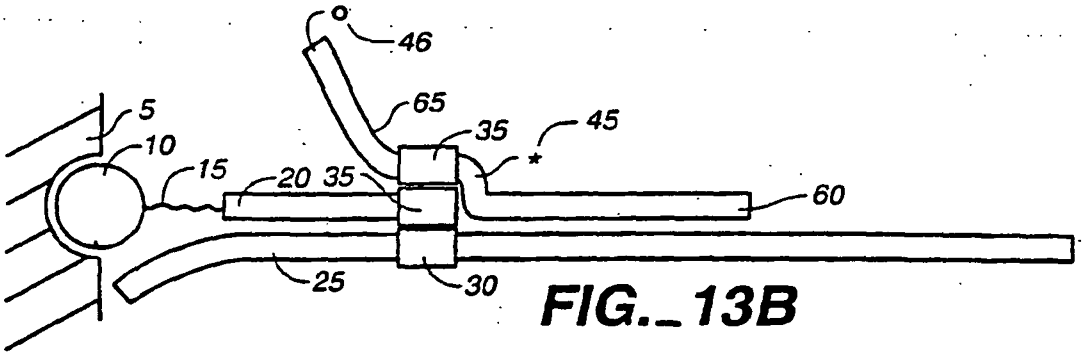


FIG._13B

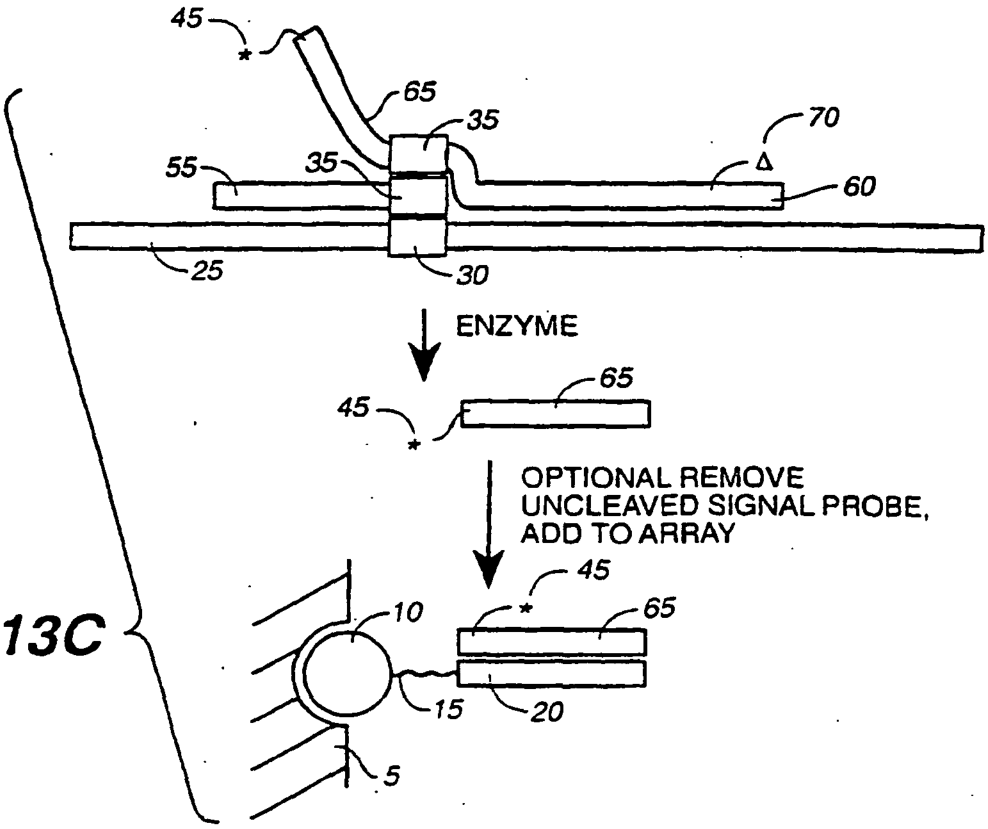


FIG._13C

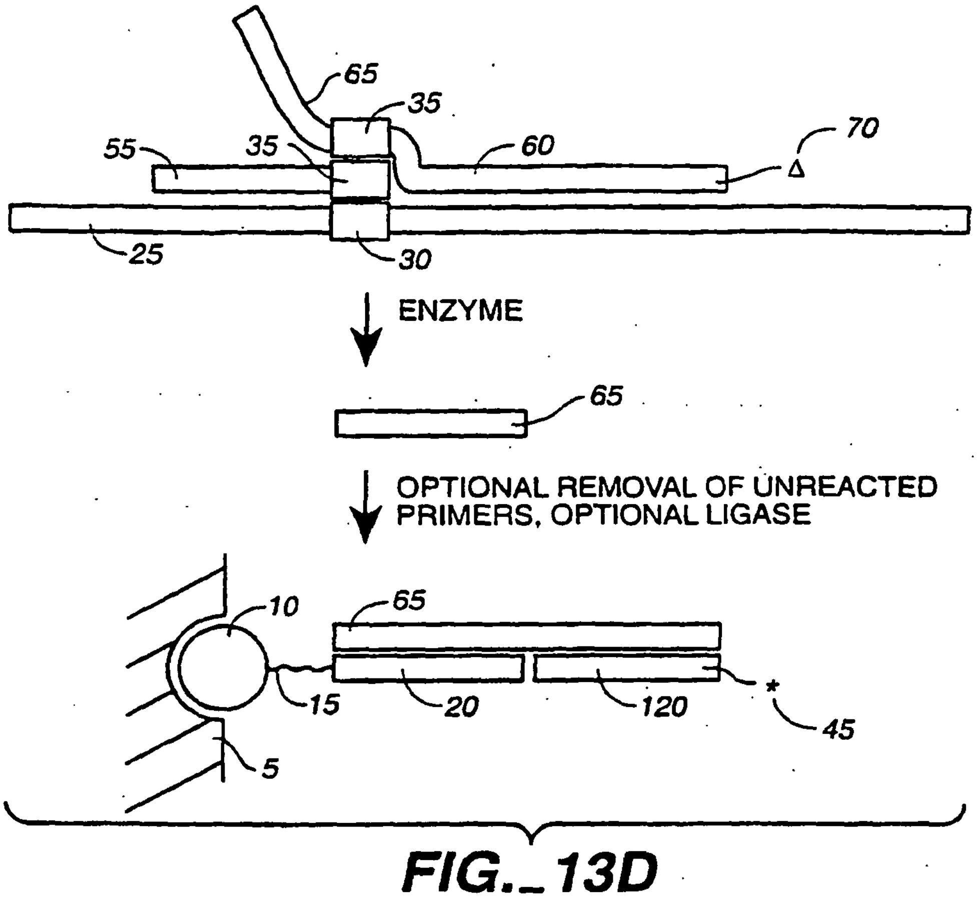


FIG._13D

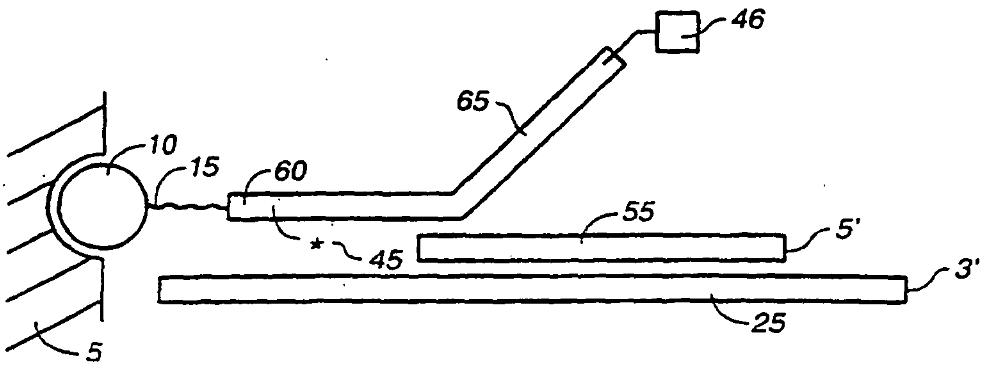


FIG._13E

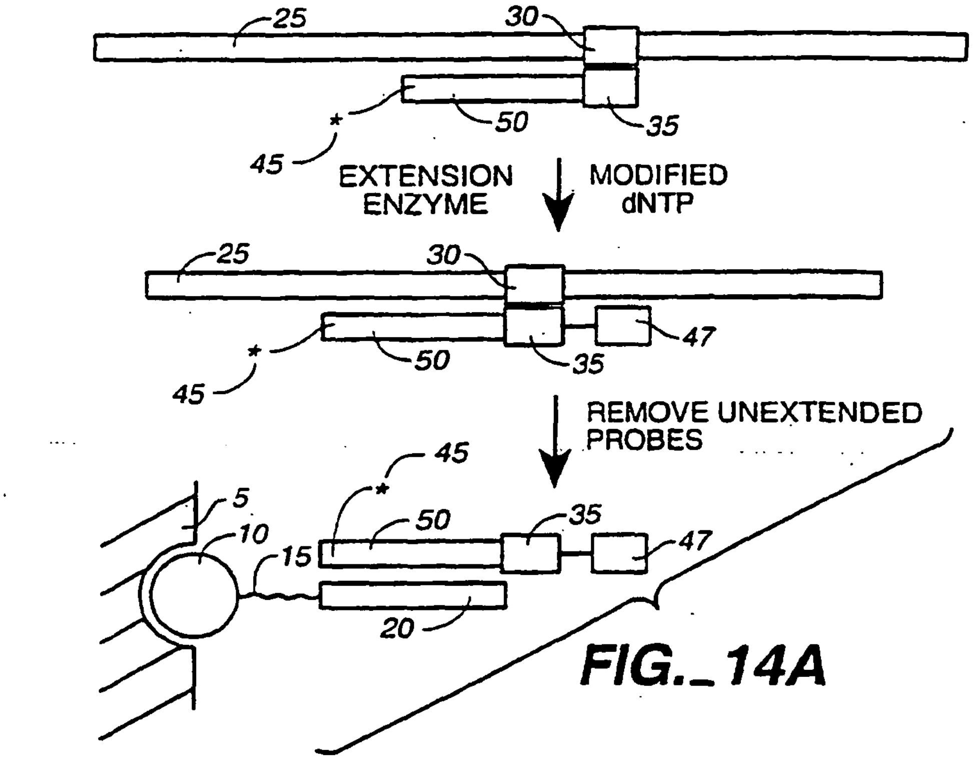


FIG._14A

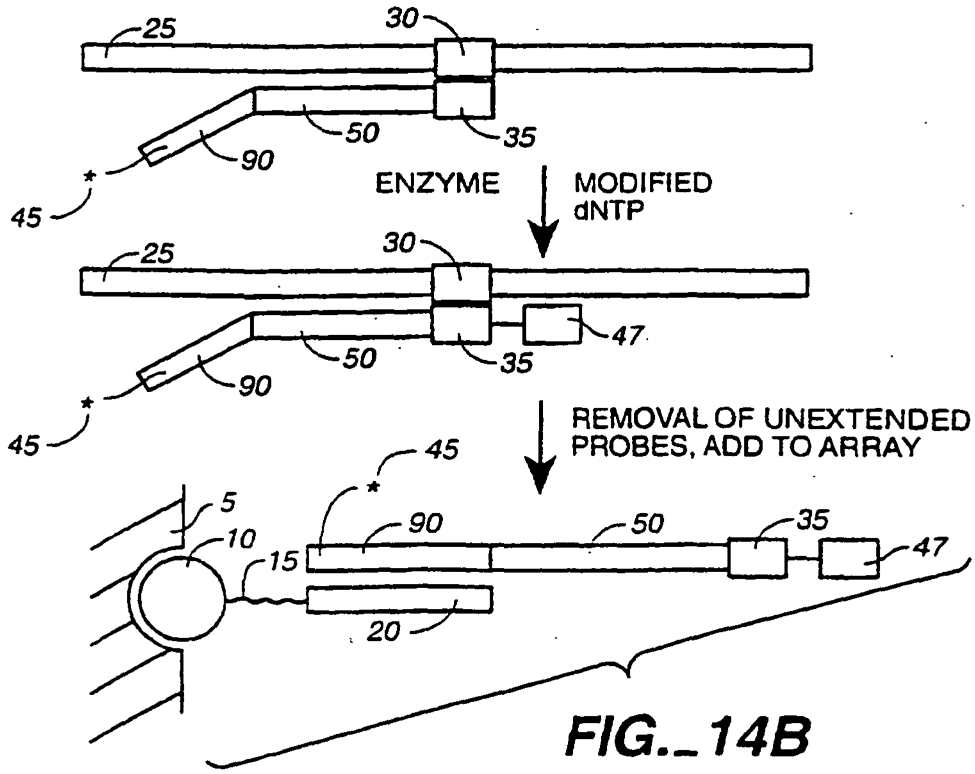


FIG._14B

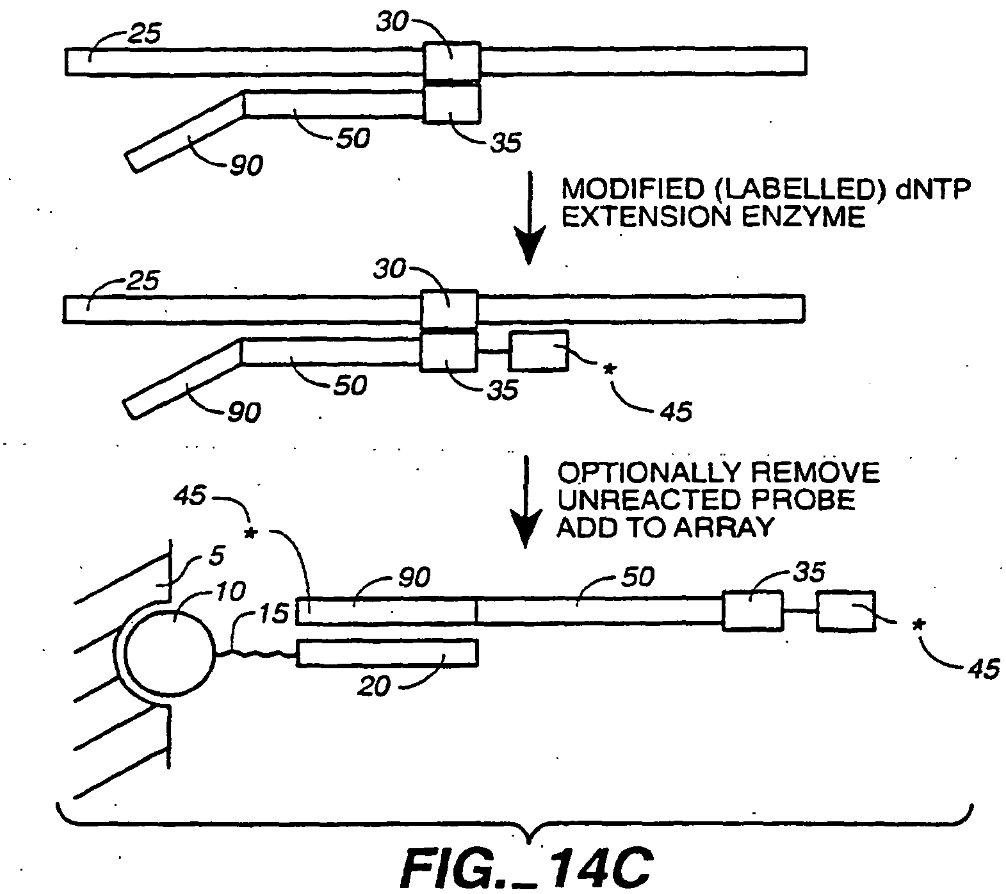


FIG._14C

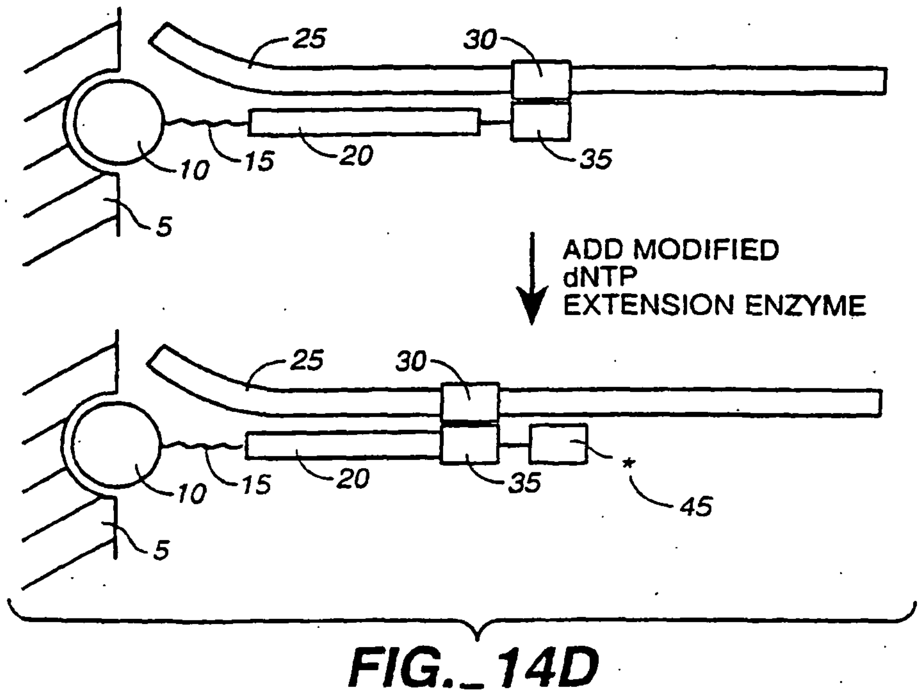


FIG._14D

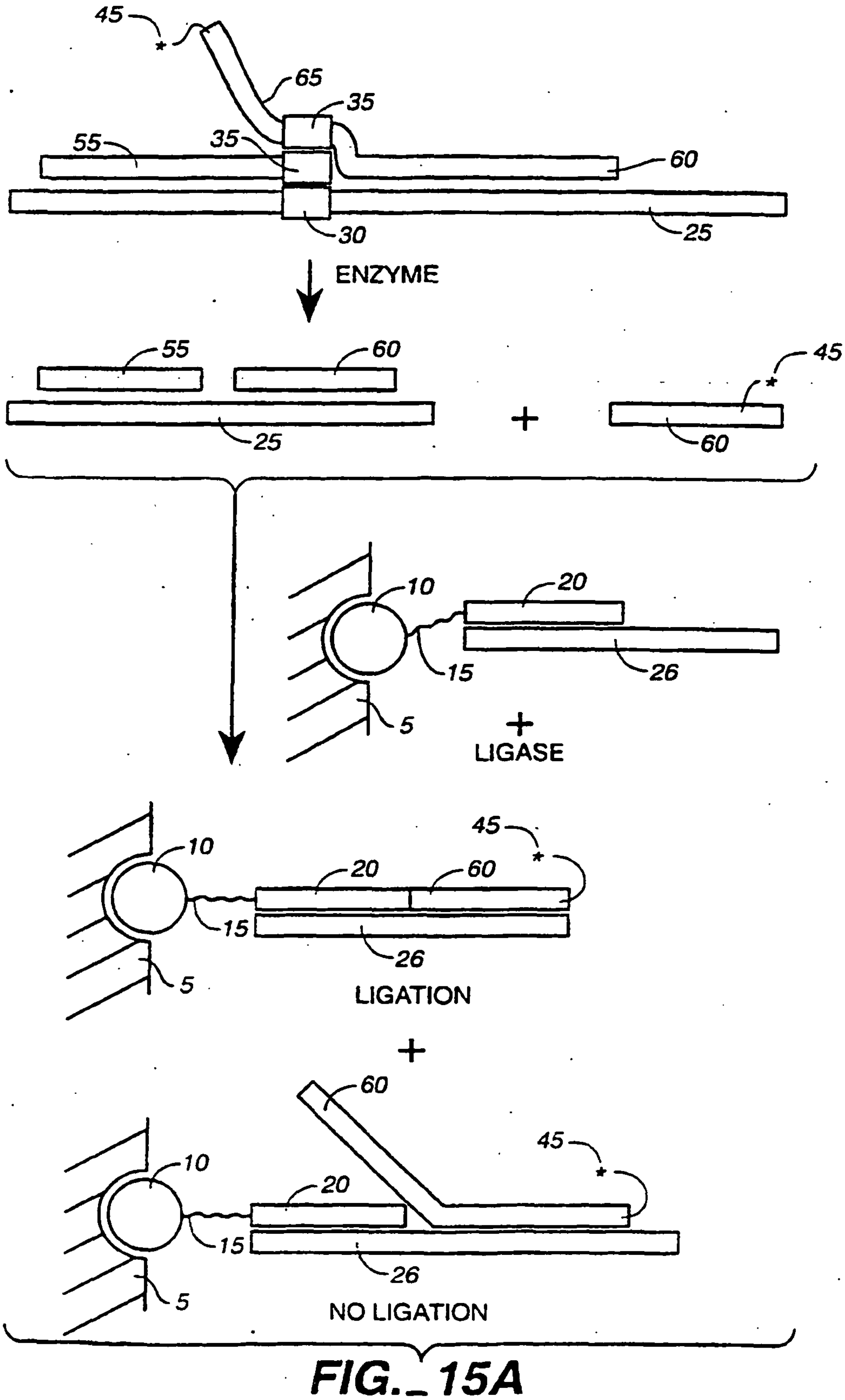
45
65
35
55
35
60
30
25
ENZYME
55
60
45
25
60
10
20
15
26
5
LIGASE
10
45
20
60
15
26
5
LIGATION
60
10
45
20
15
26
5
NO LIGATION

FIG._15A

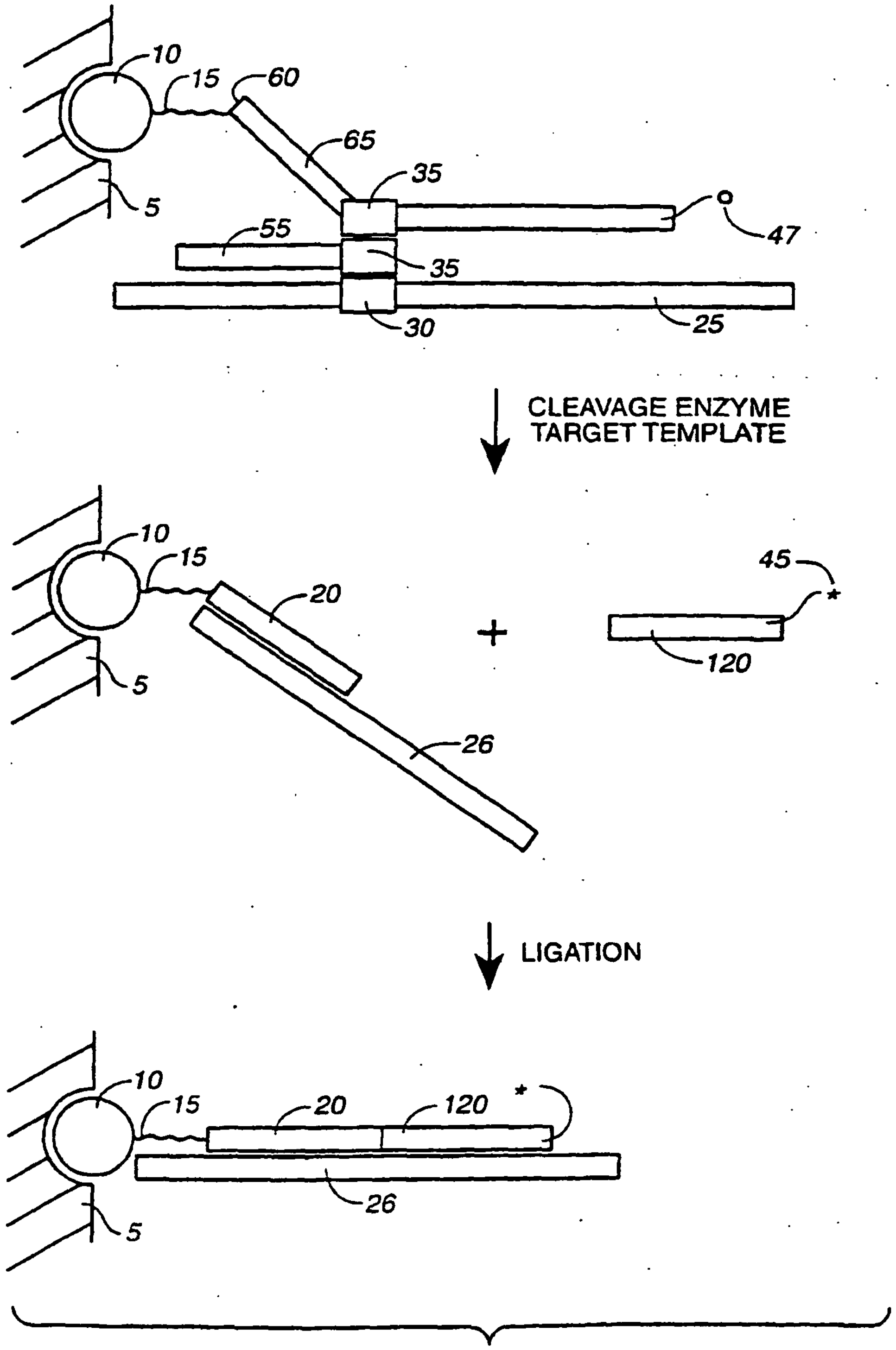
10
15
60
65
35
5
55
35
47
30
25
CLEAVAGE ENZYME
TARGET TEMPLATE
10
15
20
45
120
5
26
LIGATION
10
15
20
120
26
5

FIG._15B

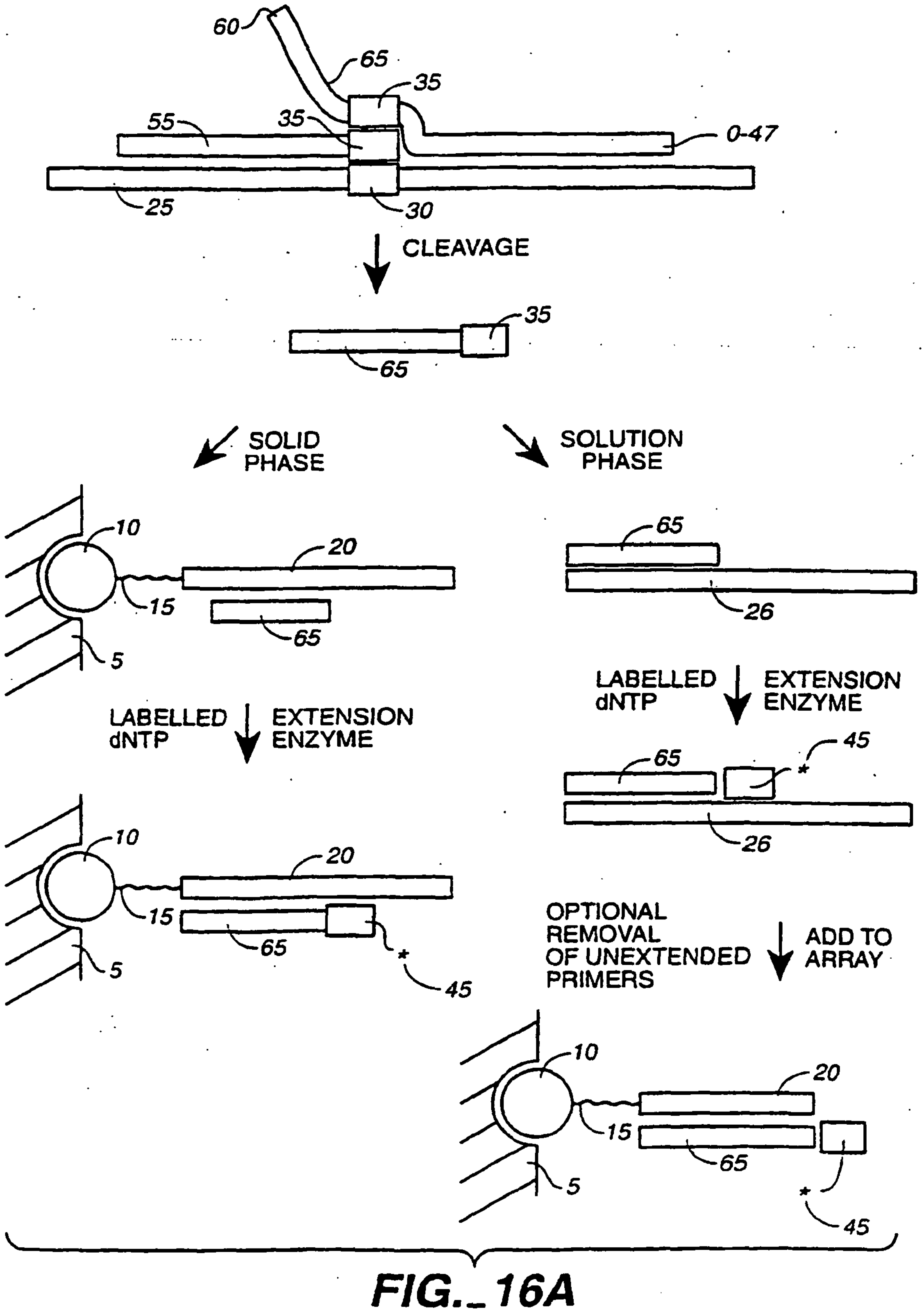
60
65
35
55
35
0-47
25
30
CLEAVAGE
35
65
SOLID PHASE
SOLUTION PHASE
10
20
15
65
5
65
26
LABELLED dNTP
EXTENSION ENZYME
LABELLED dNTP
EXTENSION ENZYME
65
45
26
10
20
15
65
5
45
OPTIONAL REMOVAL OF UNEXTENDED PRIMERS
ADD TO ARRAY
10
20
15
65
5
45

FIG._16A

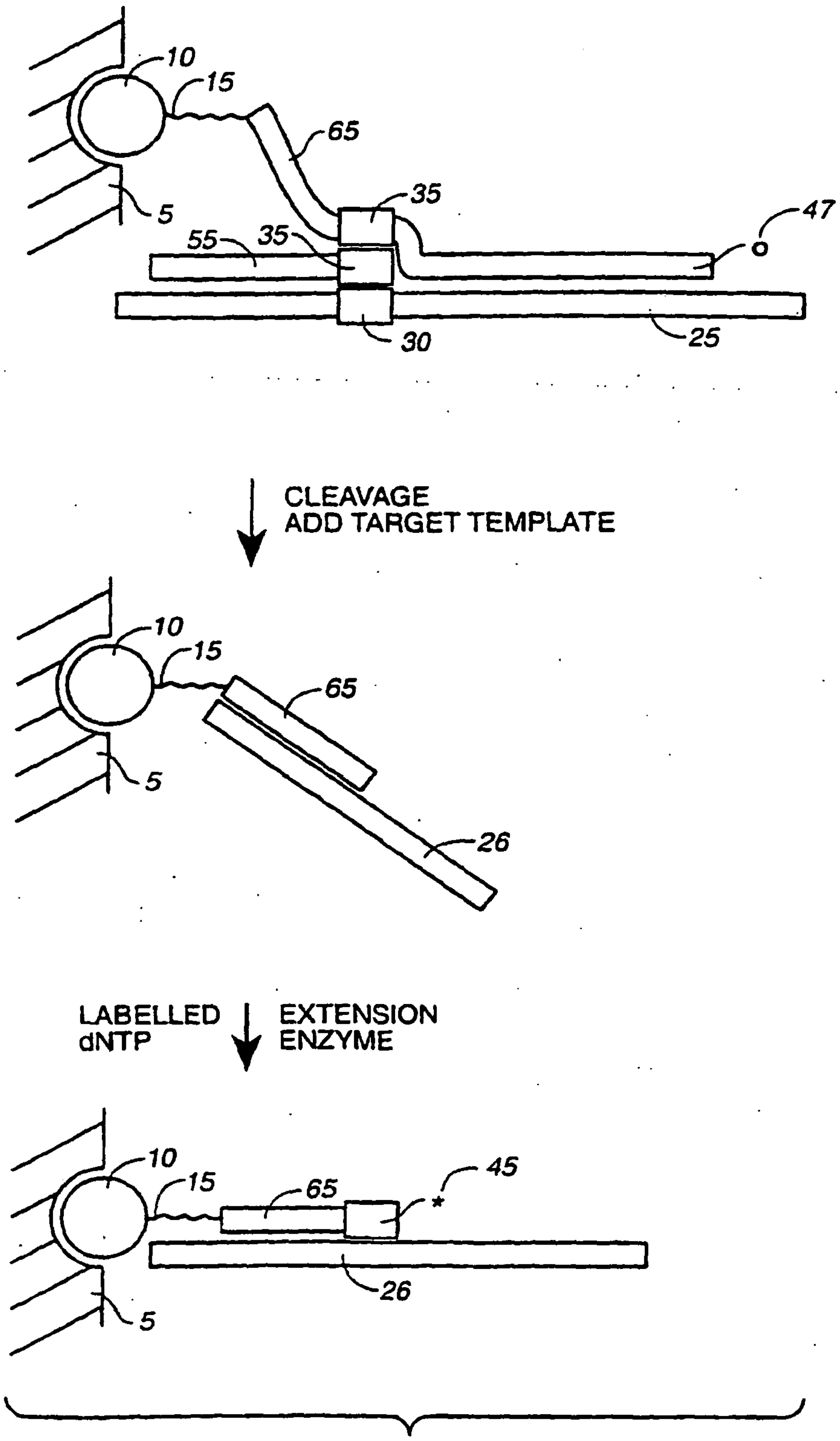
10
15
65
35
47
5
55
35
30
25
CLEAVAGE
ADD TARGET TEMPLATE
10
15
65
5
26
LABELLED
dNTP
EXTENSION
ENZYME
10
15
65
45
*
26
5

FIG._16B

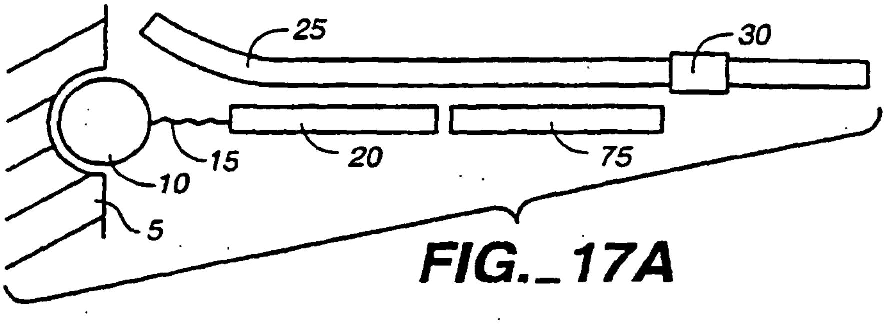


FIG._17A

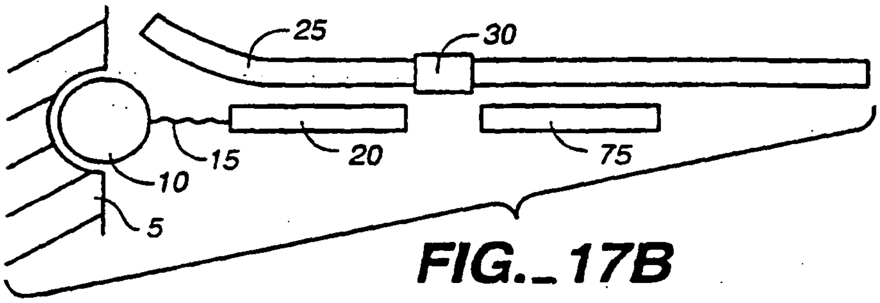


FIG._17B

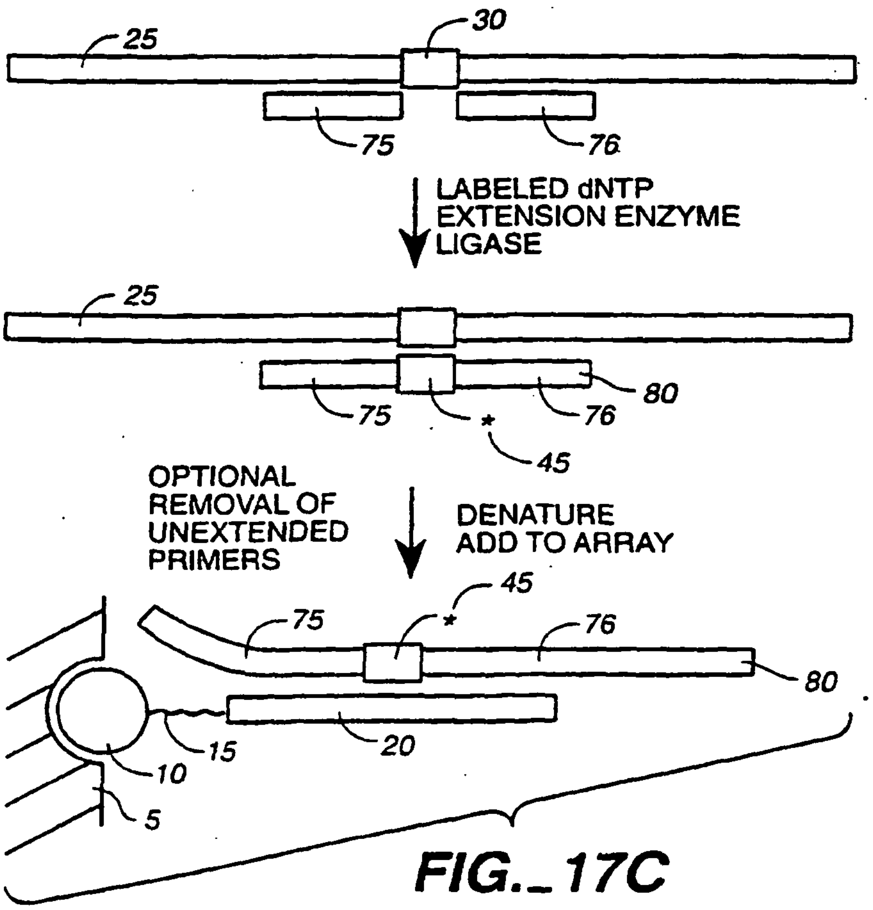


FIG._17C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4683195 A
- US 4683202 A
- US 5455166 A
- US 5130238 A
- US 5409818 A
- US 5011769 A
- US 5403719 A
- US 5660988 A
- US 4878187 A
- WO 9505480 A
- WO 951416 A
- WO 9500667 A
- US 5185243 A
- US 5679524 A
- US 5573907 A
- EP 0320308 B1
- EP 0336731 B1
- EP 0439182 B1
- WO 9001069 A
- WO 8912696 A
- WO 8909835 A
- US 5848717 A
- US 5614402 A
- US 5719028 A
- US 5541311 A
- US 5843669 A
- US 5681702 A
- US 5597909 A
- US 5545730 A
- US 5594117 A
- US 5591584 A
- US 5571670 A
- US 5580731 A
- US 5624802 A
- US 5635352 A
- US 5594118 A
- US 5359100 A
- US 5124246 A
- US 5681697 A
- US 5175270 A
- WO 9215712 A
- EP 0371437 B1
- EP 0317074 B1
- WO 9113075 A
- US 5525464 A
- US 5202231 A
- US 5695940 A
- US 4971803 A
- WO 761107 A
- US 5902723 A
- US 5547839 A
- US 5403708 A
- US 5795782 A
- WO 9323564 A
- WO 9828440 A
- WO 9813523 A
- WO 9918434 A
- WO 9967641 A
- WO 9848726 A
- WO 9850782 A
- US 20010029044 A
- US 6327410 B
- US 6424027 B
- US 20020051971 A
- US 654432 A
- US 6429027 B
- US 2002051971 A
- US 6544732 B
- EP 0799897 A1
- WO 9731256 A
- WO 9967461 A
- WO 9840726 A
- US 7348181 B
- US 5844048 A
- US 5386023 A
- US 5637684 A
- US 5602240 A
- US 5216141 A
- US 4469863 A
- US 5235033 A
- US 5034506 A
- US 7115884 B
- US 6210910 B
- US 200100290049 A
- US 2002009719 A
- WO 9945357 A
- US 5399491 A
- US 5888779 A
- US 5705365 A
- US 5710029 A
- US 5846710 A
- US 5888819 A
- US 20010029049 A
- US SN09287573 A
- US 5270163 A
- US 5475096 A

- US 5567588 A
- US 5595877 A
- US 5637459 A
- US 5683867 A
- US 5705337 A
- US 6858394 B
- US 6023540 A
- US 5881887 A
- US 5124248 A

**Non-patent literature cited in the description**


- **Nickerson.** *Current Opinion in Biotechnology,* 1993, vol. 4, 48-51
- **Abramson et al.** *Current Opinion In Biotechnology,* 1993, vol. 4, 41-47
- C.R. Newton. 1995
- **Newton et al.** *Nucl. Acid Res.,* 1989, vol. 17, 2503 9
- **Walker et al.** Molecular Methods for Virus Detection. Academic Press, Inc, 1995
- Profiting from Gene-based Diagnostics. CTB International Publishing Inc, 1996
- **Baner.** *Nuc. Acids Res.,* 1998, vol. 26, 5073-5078
- **Barany, F.** *Proc. Natl. Acad. Sci. USA,* 1991, vol. 88, 189-193
- **Lizardi et al.** *Nat. Genet.,* 1998, vol. 19, 225-232
- **Nitsen et al.** *J. Theor. Biol.,* 1997, 187273
- **Cordor et al.** *Science,* 1993, vol. 261
- **Wang et al.** *Science,* 1998, vol. 280, 1077
- **Schafer et al.** *Nature Biotechnology,* 1998, vol. 16, 33-39
- **Pastinen et al.** *Genome Res.,* 1997, vol. 7, 606-614
- **Syvänen.** *Clinica Chimica Acta,* 1994, vol. 226, 225-236
- **Sanger et al.** *Proc. Natl. Acad. Sci. USA,* 1977, vol. 74, 5463
- **Drmanac et al.** *Genomics,* 1989, vol. 4, 114
- **Koster et al.** *Nature Biotechnology,* 1996, vol. 14, 1123
- **Hyman.** *Anal. Biochem.,* vol. 174, 423
- **Metzker et al.** *Nucl. Acids Res.,* 1994, vol. 22, 4259
- **Jones.** *Biotechniques,* 1997, vol. 22, 938
- **Ronaghi et al.** *Anal. Blochem.,* 1998, vol. 242, 84
- **Nyren et al.** *Anal. Biochem.,* 1985, vol. 151, 504
- **Karamohamed ; Nyren.** *Anal. Blochem.,* 1999, vol. 271, 81
- **Canard ; Arzumanov.** *Gene,* 1994, vol. 11, 1
- **Dyatkina ; Arzumanov.** *Nucleic Acids Symp Ser,* 1987, vol. 18, 117
- **Johnson et al.** *Anal. Biochem.,* 1984, vol. 136, 192
- **Elgen ; Rigler.** *Proc. Natl Acad Sci USA,* 1994, vol. 91 (13), 5740
- **Ronaghi et al.** *Science,* 1998, vol. 281, 363
- **Chee et al.** *Nucl. Acid Res.,* 1991, vol. 19, 3301
- **Shoemaker et al.** *Nature Genetics,* 1998, vol. 14, 450
- **Beaucage et al.** *Tetrahedron,* 1993, vol. 49 (10), 1925
- **Letsinger.** *J. Org. Chem.,* 1970, vol. 35, 3800
- **Sprinzl et al.** *Eur. J. Blochem.,* 1977, vol. 81, 579
- **Letsinger et al.** *Nucl. Acids Res.,* 1988, vol. 14, 3487
- **Sawal et al.** *Chem. Lett.,* 1984, vol. 805
- **Letsinger et al.** *J. Am. Chem. Soc.,* 1988, vol. 110, 4470
- **Pauwels et al.** *Chemica Scripta,* 1900, vol. 26, 141 9
- **Mag et al.** *Nucleic Acids Res.,* 1900, vol. 19, 1437
- **Briu et al.** *J. Am. Chem. Soc.,* 1989, vol. 111, 2321
- **Egholm.** *J. Am. Chem. Soc.,* 1992, vol. 114, 1895
- **Meier et al.** *Chem. Int. Ed. Engl.,* 1992, vol. 31, 1008
- **Nielsen.** *Nature,* 1993, vol. 365, 566
- **Carlsson et al.** *Nature,* 1996, vol. 380, 207
- **Denpcy et al.** *Proc. Natl. Acad. Sci. USA,* 1995, vol. 92, 6097
- **Kledrowshi et al.** *Angew. Chem. Intl. Ed. English,* 1991, vol. 30, 423
- **Letsinger et al.** *Nucleoside & Nucleotide,* 1994, vol. 13, 1597
- Carbohydrate Modifications in Antisense Research. ASC Symposium Series 580
- **Mesmaeker et al.** *Bioorganic & Medicinal Chem. Lett.,* 1994, vol. 4, 395
- **Jeffs et al.** *J. Biomolecular NMR,* 1994, vol. 34, 17
- *Tetrahedron Lett,* 1996, vol. 37, 743
- **Jenkins et al.** *Chem. Soc. Rev.,* 1995, 169-176
- **Rawis.** *C & E News,* 02 June 1997, 35
- Molecular Cloning: A Laboratory Manual. **Manlatis et al.** Short Protocols in Molecular Biology. 1989

- **Tijssen.** Overview of principles of hybridization and the strategy of nucleic add assays. *Techniques in Biochemistry and Molecular Biology-Hybridization with Nucleic Acid Probes,* 1993
- **Richard P. Haugland.** 6th Edition of the Molecular Probes Handbook
- technical section on cross-linkers. Pierce Chemical Company catalog, 1994, 155-200
- Nucleic Add Sequence-Based Amplification. **Sooknanan et al.** Molecular Methods for Virus Detection. Academic Press, 1995, 261-285
- **Sylvanen et al.** *Genomics,* 1990, vol. 8, 684-692
- **Pastinen et al.** *Genomics Res.,* 1997, vol. T (6), 808-614
- **Reeves et al.** *Anal. Biochem.,* 1969, vol. 28, 282
- **Guillory et al.** *Anal. Biochem.,* 1971, vol. 39, 170
- **Johnson et al.** *Anal. Biochem.,* 1968, vol. 15, 273
- **Cook et al.** *Anal. Biochem.,* 1978, vol. 91, 557
- **Drake et al.** *Anal. Biochem.,* 1979, vol. 94, 117
- **Nikforov et al.** *Nucleic Acid Res.,* 1994, vol. 22, 4167
- **Barshop et al.** *Anal. Biochem.,* 1991, vol. 197 (1), 266-272
- **Justesen et al.** *Anal. Biochem.,* 1992, vol. 207 (1), 90.93
- **Lust et al.** *Clin. Chem. Acta,* 1976, vol. 66 (2), 241
- **Johnson et al.** *Anal. Biochem.,* 1968, vol. 26, 137
- **Metzker et al.** *Nucl. Add Res.,* 1994, vol. 22 (20), 4259
- **Canard.** *Gene,* 1994, vol. 148 (1), 1-6
- **Dyatkina et al.** *Nucleic Acid Symp. Ser.,* 1987, vol. 18, 117-120
- **Johnson et al.** *Anal. Biochem.,* 1984, vol. 136 (1), 192
- **Bock.** *Nature,* 1992, vol. 355, 564
- **Femulok et al.** *Current Op. Chem. Biol.,* 1998, vol. 2, 230
- **Freund ; Walpole.** Mathematical Statistics. Prentice Hall, Inc, 1980